# FASCICULE DE BREVET EUROPEEN

(11) **EP 2 699 570 B1**
(45) Date de publication et mention de la délivrance du brevet: **22.08.2018**
(21) Numéro de dépôt: 12715117.3
(22) Date de dépôt: 20.04.2012
(51) Int. Cl.: C07D 451/04, C07D 453/02, C07D 455/02, A61K 31/46, A61K 31/4375, A61K 31/439, A61P 25/00

(54) **1,2,3-TRIAZOLES 1,4-DISUBSTITUÉES, LEURS PROCÉDÉS DE PRÉPARATION ET LEURS UTILISATIONS DIAGNOSTIQUES ET THÉRAPEUTIQUES**
1,4-DISUBSTITUIERTE 1,2,3-TRIAZOLE, DEREN HERSTELLUNGSVERFAHREN UND DEREN DIAGNOSTISCHEN UND THERAPEUTISCHEN VERWENDUNGEN
1,4-DISUBSTITUTED 1,2,3-TRIAZOLES, THEIR PREPARATION PROCESSES AND THEIR DIAGNOSTIC AND THERAPEUTIC USES

(30) Priorité: 20.04.2011 FR 1153420
(43) Date de publication de la demande: 26.02.2014
(73) Titulaire: Centre National de la Recherche Scientifique (C.N.R.S.), 75016 Paris (FR); Institut National de la Santé et de la Recherche Médicale (INSERM), 75013 Paris (FR)
(72) Inventeur: ROUTIER, Sylvain, F-45510 Tigy (FR); SUZENET, Franck, F-45380 La Chapelle Saint Mesmin (FR); PIN, Frédéric, 39700 COURTEFONTAINE (FR); CHALON, Sylvie, F-37540 Saint Cyr sur Loire (FR); VERCOUILLIE, Johnny, F-37400 Amboise (FR); GUILLOTEAU, Denis, F-37540 Saint Cyr sur Loire (FR)
(74) Mandataire: Lavoix
(86) Numéro de dépôt international: PCT/EP2012/057309
(87) Numéro de publication internationale: WO 2012/143526

(56) Documents cités:
- EP-A1- 1 764 362
- EP-A1- 2 308 869
- WO-A1-96/21660
- WO-A1-2005/049612
- WO-A1-2006/065217
- WO-A1-2007/021574
- WO-A1-2009/092293
- WO-A1-2011/025690
- WO-A2-2006/065601
- JAIPRAKASH N. SANGSHETTI AND DEVANAND B. SHINDE: "One pot synthesis and SAR of some novel 3-substituted 5,6-diphenyl-1,2,4-triazines as antifungal agents", BIOORGANIC & MEDICINAL CHEMISTRY LETTERS, vol. 20, no. 2, 2010, pages 742-745, XP026812571,

## Description

La présente invention a pour objet des composés de type 1,2,3-triazole 1,4-disubstituée et leurs procédés de préparation. Elle a également pour objet les utilisations thérapeutiques et diagnostiques desdits composés notamment en tant qu'agoniste, antagoniste et/ou ligand du récepteur nicotinique alpha 7.

Les systèmes cholinergiques centraux régulent un nombre important de fonctions physiologiques et cognitives. Ces effets sont régulés par différents types de récepteurs dont les récepteurs nicotiniques, appartenant à la famille des récepteurs-canaux et comportant plusieurs sous-types qui modulent différents effets biologiques. Des composés visant sélectivement certains types de récepteurs peuvent se révéler utiles pour diagnostiquer ou traiter les pathologies qui leurs sont associées, comme par exemple des troubles cognitifs, la douleur, l'addiction au tabac etc.

Parmi les différents sous-types, les récepteurs nicotiniques alpha 7 (Rα7) sont particulièrement impliqués dans les processus cognitifs et dans un grand nombre de maladies du système nerveux central telles que l'addiction, les affections psychiatriques (schizophrénie, troubles de l'attention,...) et les affections neurodégénératives, en particulier la maladie d'Alzheimer.

L'étude de ces récepteurs, qui jouent un rôle primordial et précoce dans la physiopathologie de maladies du système nerveux central (SNC) et du système nerveux périphérique (SNP), permet de suivre l'évolution de ces maladies.

En effet, les Rα7 sont largement exprimés dans des régions cérébrales touchées au cours de la maladie d'Alzheimer et des variations de la densité de ces récepteurs sont observées et précèdent la mort neuronale dans plusieurs régions cérébrales telles que l'hippocampe, le cortex ou encore les ganglions de la base.

De plus, des agonistes et/ou antagonistes des Rα7 représentent une alternative pour le traitement de ces maladies.

A ce jour, aucun marqueur radiopharmaceutique comprenant un ¹⁸F n'est utilisé en clinique pour le diagnostic et le suivi de la maladie d'Alzheimer.

Par ailleurs, aucun ligand de type agoniste des Rα7 n'est encore prescrit pour le traitement de cette maladie.

La présente invention a pour but de fournir de nouveaux ligands des Rα7, de type agoniste ou antagoniste.

La présente invention a également pour but de fournir de nouveaux ligands des Rα7 permettant d'obtenir des marqueurs radiopharmaceutiques utilisables en imagerie moléculaire pour le diagnostic précoce et le suivi de maladies du SNC, en particulier de la maladie d'Alzheimer, et du SNP.

La présente invention a également pour but de fournir de nouveaux ligands des Rα7 pour le traitement thérapeutique de maladies du SNC, en particulier de la maladie d'Alzheimer, et du SNP.

La présente invention concerne des composés de formule (II) : 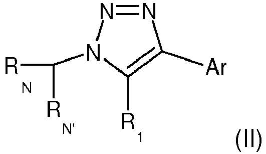 dans laquelle :
- le groupe Ar est choisi parmi les groupes phényle, pyridyle, thiophényle, furanyle, benzothiophényle, benzofuranyle et naphtalènyle, éventuellement substitués par un ou plusieurs groupes choisis parmi :
   - les atomes d'halogène,
   - le groupe -OH,
   - les groupes alkyles et alcoxyles linéaires ou ramifiés comprenant de 1 à 10 atomes de carbone, éventuellement substitués, et
   - les groupes aryles comprenant de 6 à 30 atomes de carbone et les groupes hétéroaryles comprenant de 1 à 30 atomes de carbone, éventuellement substitués ;
- les groupes R_{N} et R_{N'} forment ensemble, avec l'atome de carbone auxquels ils sont liés, un groupe choisi parmi les groupes tropane, quinuclidine et octahydroquinolizine de formules suivantes : 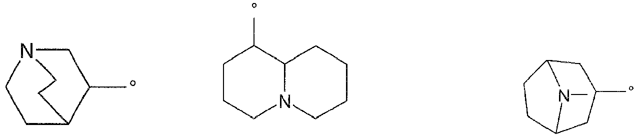 ledit groupe azacycloalcane étant éventuellement substitué par un ou plusieurs groupes choisis parmi :
   - les atomes d'halogène,
   - le groupe -OH, et
   - les groupes alkyles et alcoxyles linéaires ou ramifiés comprenant de 1 à 10 atomes de carbone, éventuellement substitués,
- R₁ est un groupe choisi parmi H ou NH₂ ;
ainsi que ses sels pharmaceutiquement acceptables, ses hydrates ou ses structures cristallines polymorphiques, ses racémates, diastéréoisomères ou énantiomères, les radicaux "alkyle", "aryle", et "hétéroaryle" éventuellement substitués susmentionnés pouvant être substitués par un ou plusieurs substituants choisis dans le groupe constitué de : CHO, amino, hydroxy, halogène, halogénoalkyle, carboxyle, alkyle, (hétéro)aryle, cycloalkyle, hétérocycloalkyle, alkaryle, alkoxy, alkylthio, alkylcarbonyle, aminocarbonyle, alkylcarboxyle, alkylamino, aryloxy, arylalkoxy, cyano, alkylsulfonyle, nitro et carboxyalkyle.

Dans la formule (II), le groupe Ar peut notamment être choisi parmi le groupe phényle ou naphtyle, éventuellement substitué par un ou plusieurs groupes choisis parmi -F, -Br, -Cl, -OCH₃, -OH, -CH₂F, -CH₂Br, -CH₂OH, -CF₃, les groupes alkyles comprenant de 1 à 10 atomes de carbone (de préférence un groupe méthyle), éventuellement substitués et un groupe aryle ou hétéroaryle, éventuellement substitués.

Le groupe Ar peut notamment être choisi parmi les groupes pyridine, thiophène, benzothiophène et benzofurane, éventuellement substitués par un ou plusieurs groupes choisis parmi -Br, -Cl, -F, -OMe, les groupes alkyles comprenant de 1 à 10 atomes de carbone (de préférence un groupe méthyle) éventuellement substitué, et les groupes aryles comprenant de 6 à 30 atomes de carbone (par exemple un groupe phényle) ou les groupes hétéroaryles comprenant de 1 à 30 atomes de carbone (par exemple un groupe thiophène, furane ou pyridine), éventuellement substitués.

Selon la présente invention, les radicaux "alkyle" représentent des radicaux hydrocarbonés saturés, en chaîne droite ou ramifiée, comprenant de 1 à 10 atomes de carbone, de préférence de 1 à 5 atomes de carbone (ils peuvent typiquement être représentés par la formule CₙH₂ₙ₊₁, n représentant le nombre d'atomes de carbone). On peut notamment citer, lorsqu'ils sont linéaires, les radicaux méthyle, éthyle, propyle, butyle, pentyle, hexyle, octyle, nonyle et décyle. On peut notamment citer, lorsqu'ils sont ramifiés ou substitués par un ou plusieurs radicaux alkyles, les radicaux isopropyle, tert-butyle, 2-éthylhexyle, 2-méthylbutyle, 2-méthylpentyle, 1-méthylpentyle et 3-méthylheptyle. Les radicaux "alkyle" substitués sont notamment des chaînes alkyles linéaires ou ramifiées en C₁-C₁₀, substituées par un ou plusieurs groupes choisis parmi :
- les atomes d'halogène, et notamment F, Cl ou Br,
- les groupes aryles comprenant de 6 à 30 atomes de carbone,
- les groupes hétéroaryles comprenant de 1 à 30 atomes de carbone, et notamment une pyridine,
- les groupes -R, -OR, -OH, -SR, R étant un groupe alkyle linéaire ou ramifié comprenant de 1 à 10 atomes de carbone,
- les groupes -NRₐR_{b}, Rₐ et R_{b} étant indépendamment choisis dans le groupe constitué de l'atome d'hydrogène et des groupes alkyles comprenant de 1 à 10 atomes de carbone.

Dans la présente demande, le symbole « ° » représente le point d'attache du groupe azacycloalcane au triazole.

Le terme "aryle" désigne un système aromatique hydrocarboné, mono ou bicyclique ou polycyclique comprenant de 6 à 30, de préférence de 6 à 10 atomes de carbone. Parmi les radicaux aryles, on peut notamment citer le radical phényle ou naphtyle.

Lorsque le radical aryle comprend au moins un hétéroatome, on parle de radical "hétéroaryle". Ainsi, le terme "hétéroaryle" désigne un système aromatique comprenant un ou plusieurs (typiquement de 1 à 6, de préférence de 1 à 4) hétéroatomes, choisis parmi l'azote, l'oxygène ou le soufre, mono ou bicyclique ou polycyclique, comprenant de 1 à 30, de préférence de 2 à 20, préférentiellement de 3 à 15 et avantageusement de 4 à 10 atomes de carbone.

Parmi les radicaux hétéroaryles, on pourra citer le pyrazinyle, le thiényle, l'oxazolyle, le furazanyle, le pyrrolyle, le 1,2,4-thiadiazolyle, le naphthyridinyle, le pyridazinyle, le quinoxalinyle, le phtalazinyle, l'imidazo[1,2-a]pyridine, l'imidazo[2,1-b]thiazolyle, le cinnolinyle, le triazinyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzothiazolyle, le furanyle, le benzofuranyle, l'imidazolyle, l'indolyle, le triazolyle, le tétrazolyle, l'indolizinyle, l'isoxazolyle, l'isoquinolinyle, l'isothiazolyle, l'oxadiazolyle, le pyrazinyle, le pyridazinyle, le pyrazolyle, le pyridyle, le pyrimidinyle, le purinyle, le quinazolinyle, le quinolinyle, l'isoquinolyle, le 1,3,4-thiadiazolyle, le thiazolyle, le triazinyle, l'isothiazolyle, le carbazolyle, le thiophényle, le benzothiophényle, ainsi que les groupes correspondants issus de leur fusion ou de la fusion avec le noyau phényle.

Les radicaux "alkyle", "aryle" et "hétéroaryle" éventuellement substitués susmentionnés peuvent être substitués par un ou plusieurs (typiquement de 1 à 5, de préférence de 1 à 3) substituants choisis parmi les groupes suivants : CHO, amino, hydroxy, halogène, et notamment F, Br ou Cl, halogénoalkyle, carboxyle, alkyle, (hétéro)aryle, cycloalkyle, hétérocycloalkyle, alkaryle, alkoxy, et notamment -OMe, alkylthio, alkylcarbonyle, aminocarbonyle, alkylcarboxyle, et notamment -COOMe, alkylamino, aryloxy, arylalkoxy, cyano, alkylsulfonyle, nitro ou carboxyalkyle.

Parmi les hétérocycloalkyles, on peut notamment citer la morpholine, la N-méthylpipérazine et la pipéridine.

Selon un mode de réalisation, les groupes "alkyle", "aryle" et "hétéroaryle" sont substitués par un ou plusieurs atomes d'halogène, de préférence F ou I.

Selon un autre mode de réalisation, le groupe « alkyle » est substitué par un ou plusieurs hétérocycloalkyles, et notamment la morpholine, la N-méthylpipérazine ou la pipéridine.

Selon un autre mode de réalisation, le groupe « alkyle » est substitué par un ou plusieurs groupes -OH.

Selon un autre mode de réalisation, le groupe « alkyle » est substitué par un ou plusieurs groupes hétéroaryles, et notamment une pyridine.

Parmi les groupes alkyles substitués, on peut notamment citer les groupes alkyles perfluorés.

Parmi les groupes aryles ou hétéroaryles, substitués ou non, on peut plus particulièrement citer les groupes suivants : 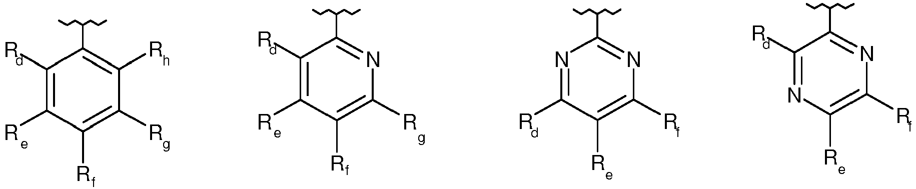 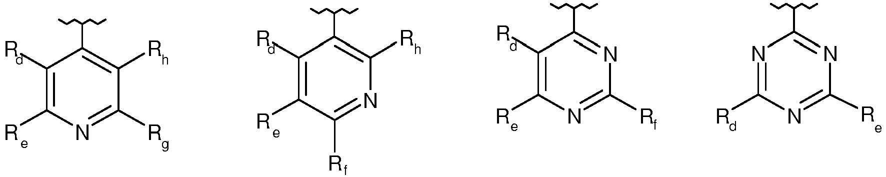 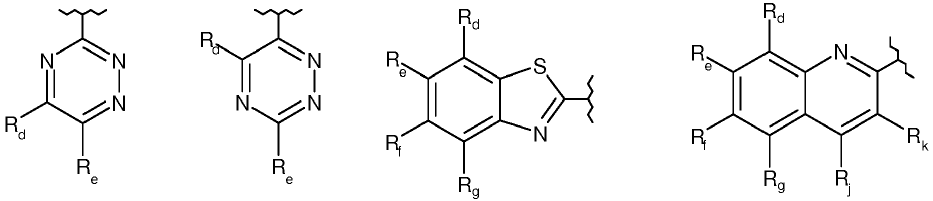 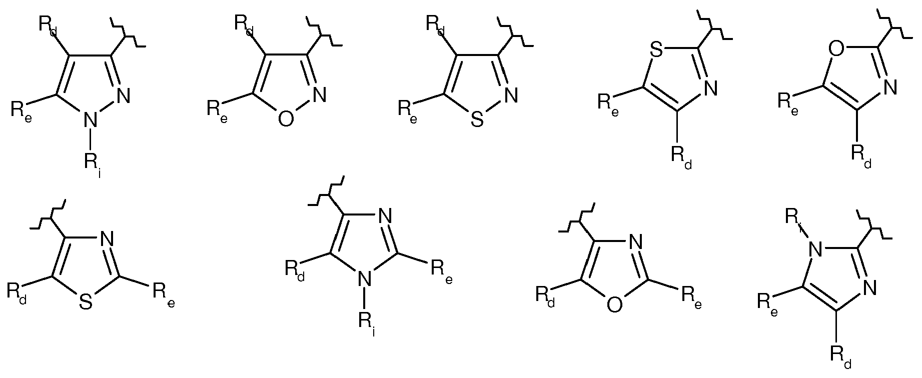 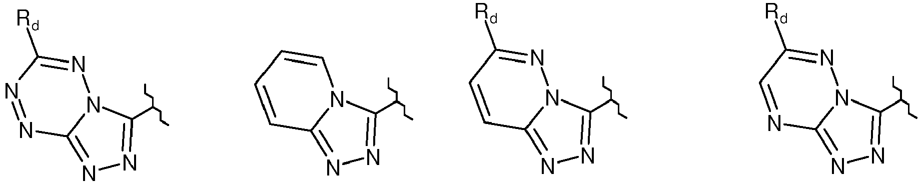 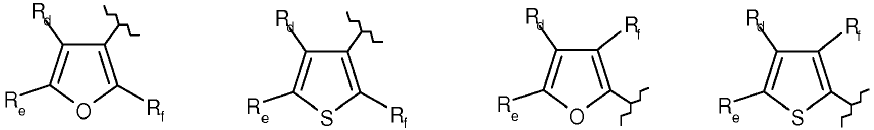 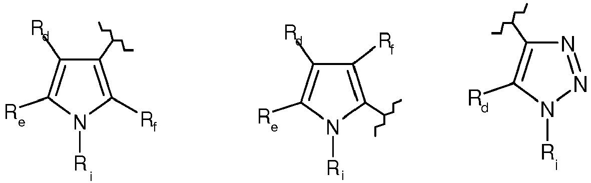 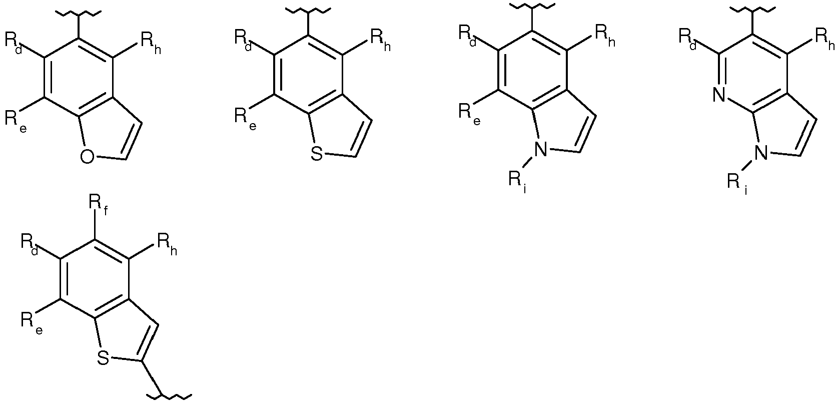 les groupes R_{d}, Rₑ, R_{f}, R_{g}, Rₕ, Rⱼ et Rₖ étant choisis, indépendamment les uns des autres, dans le groupe constitué des substituants suivants :
- un atome d'hydrogène,
- un atome d'halogène, notamment Br, Cl ou F,
- un groupe (hétéro)aryle tel que défini ci-dessus,
- un groupe alkyle comprenant de 1 à 10 atomes de carbone, et étant de préférence un groupe méthyle,
- un groupe halogénoalkyle, - un groupe -CHO,
- un groupe -CN,
- un groupe -NO₂,
- un groupe -CF₃,
- un groupe phényle,
- un groupe alkylsulfonyle,
- un groupe -CO₂R'_{α}, R'_{α} représentant un atome d'hydrogène ou un groupe alkyle comprenant de 1 à 10 atomes de carbone, notamment un groupe -CO₂H,
- un groupe -COR'_{α}, R'_{α} étant tel que défini ci-dessus, notamment un groupe - COCH₃,
- un groupe alkylthio ou un groupe OR'_{α}, R'_{α} étant tel que défini ci-dessus, notamment un groupe -OH, -OCH₃, -SCH₃, -O-CH(CH₃)₂, -O-CH₂-CH₂-CH₃,
- un groupe -NR'_{α}R_{β}, R'_{α} et R_{β} représentant indépendamment l'un de l'autre un atome d'hydrogène ou un groupe alkyle comprenant de 1 à 10 atomes de carbone, notamment un groupe NH₂,
- un groupe -CONH₂, et
- un groupe 2-pyridinyle.
le groupe Rᵢ étant un atome d'hydrogène ou un groupe alkyle comprenant de 1 à 10 atomes de carbone.

Selon un mode de réalisation, les groupes « aryles » sont substitués par un ou plusieurs groupes choisis dans le groupe constitué de : -COOMe ; -OMe ; un halogène, et notamment F, Cl ou Br ; un groupe alkyle comprenant de 1 à 10 atomes de carbone, éventuellement substitué par un halogène, tels que -CH₂F, -CH₂Cl, un groupe hétéroaryle comprenant de 1 à 30 atomes de carbone.

Selon un mode de réalisation, les groupes « hétéroaryles » sont substitués par un ou plusieurs groupes choisis dans le groupe constitué de : un halogène, et notamment F, Cl ou Br ; -NO₂ ; -C(=O)H ; un groupe aryle comprenant de 6 à 30 atomes de carbone, et notamment un phényle ; un groupe hétéroaryle comprenant de 1 à 30 atomes de carbone.

Parmi les groupes aryles, on peut citer : 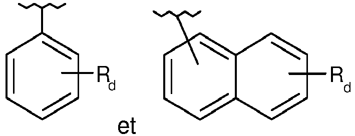 R_{d} étant tel que défini ci-dessus, et étant de préférence choisi dans le groupe constitué de : H, F, Br, OCH₃, OH, CH₂F, COOCH₃, CH₂Br, et les groupes alkyles comprenant de 1 à 10 atomes de carbone.

Parmi les groupes aryles, on peut également citer : 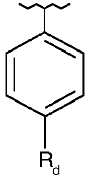 R_{d} étant tel que défini ci-dessus.

Parmi les groupes hétéroaryles, on peut citer :

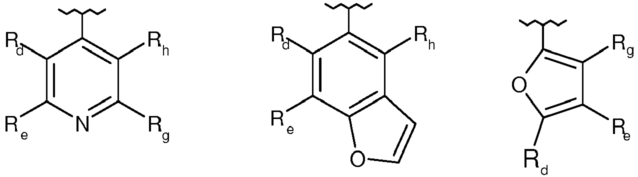

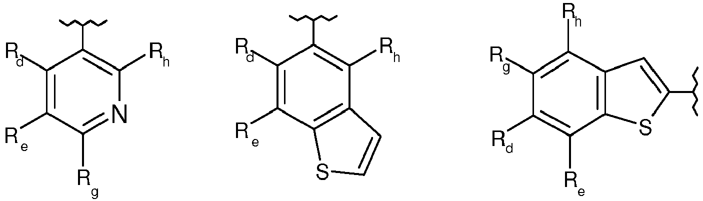

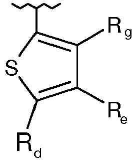

R_{d}, Rₑ, R_{g} et Rₕ étant choisis, indépendamment les uns des autres, dans le groupe constitué des substituants suivants : un atome d'hydrogène, un atome d'halogène, notamment Br, Cl ou F, et un groupe alkyle comprenant de 1 à 10 atomes de carbone, et étant de préférence un groupe méthyle, un groupe aryle comprenant de 6 à 30 atomes de carbone ou un groupe hétéroaryle comprenant de 1 à 30 atomes de carbone. En particulier, R_{d}, Rₑ, R_{g} et Rₕ sont choisis, indépendamment les uns des autres, dans le groupe constitué de :
- un atome d'hydrogène ;
- un groupe aryle comprenant de 6 à 30 atomes de carbone ;
- un groupe hétéroaryle comprenant de 1 à 30 atomes de carbone.

Les radicaux "alkoxy" selon la présente invention sont des radicaux de formule -O-alkyle, le groupe alkyle étant tel que défini précédemment.

Le terme "alkylthio" désigne un groupe -S-alkyle, le groupe alkyle étant tel que défini ci-dessus.

Le terme "alkylamino" désigne un groupe -NH-alkyle, le groupe alkyle étant tel que défini ci-dessus.

Le terme "alkylcarbonyle" désigne un groupe -CO-alkyle, le groupe alkyle étant tel que défini ci-dessus.

Le terme "alkylcarboxyle" désigne un groupe -COO-alkyle, le groupe alkyle étant tel que défini ci-dessus.

Le terme "alkylsulfonyle" désigne un groupe -SO₂-alkyle, le groupe alkyle étant tel que défini ci-dessus.

Parmi les atomes d'halogène, on cite plus particulièrement les atomes de fluor, de chlore, de brome et d'iode.

Le terme "aryloxy" désigne un groupe -O-aryle, le groupe aryle étant tel que défini ci-dessus.

Le terme "arylalkoxy" désigne un groupe aryl-alkoxy-, les groupes aryles et alkoxy étant tels que définis ci-dessus.

Le terme "carboxyalkyle" désigne un groupe HOOC-alkyle-, le groupe alkyle étant tel que défini ci-dessus. Comme exemple de groupes carboxyalkyles, on peut citer notamment le carboxyméthyle ou le carboxyéthyle.

Lorsqu'un radical alkyle est substitué par un groupe aryle, on parle de radical "arylalkyle" ou "aralkyle". Les radicaux « arylalkyles » ou « aralkyles » sont des radicaux aryl-alkyl-, les groupes aryles et alkyles étant tels que définis ci-dessus. Parmi les radicaux arylalkyles, on peut notamment citer le radical benzyle ou phénéthyle.

L'expression "sels pharmaceutiquement acceptables" fait référence aux sels d'addition acide relativement non toxiques, inorganiques et organiques, et les sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide, on trouve les sels bromhydrate, chlorhydrate, sulfate, bisulfate, phosphate, nitrate, acétate, oxalate, valérate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-b-hydroxynaphtoates, acide gentisique, iséthionates, di-p-toluoyltartrates, méthanesulfonates, éthanesulfonates, benzènesulfonates, p-toluènesulfonates, cyclohexylsulfamates et quinateslaurylsulfonate, et analogues (Voir par exemple S.M. Berge et al. « Pharmaceutical Salts » J. Pharm. Sci, 66: p.1-19 (1977)). Les sels d'addition acide peuvent également être préparés en faisant réagir séparément le composé purifié sous sa forme acide avec une base organique ou inorganique et en isolant le sel ainsi formé. Les sels d'addition acide comprennent les sels aminés et métalliques. Les sels métalliques adaptés comprennent les sels de sodium, potassium, calcium, baryum, zinc, magnésium et aluminium. Les sels de sodium et de potassium sont préférés. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les amines qui sont souvent utilisées en chimie médicinale en raison de leur faible toxicité et de leur acceptabilité pour l'usage médical : ammoniaque, éthylènediamine, N-méthyl-glucamine, lysine, arginine, ornithine, choline, N,N'-dibenzyléthylènediamine, chloroprocaïne, diéthanolamine, procaïne, N-benzyl-phénéthylamine, diéthylamine, pipérazine, tris(hydroxyméthyl)-aminométhane, hydroxyde de tétraméthyl-ammonium, triéthylamine, dibenzylamine, éphénamine, dehydroabiétylamine, N-éthylpipéridine, benzylamine, tétra-méthylammonium, tétraéthylammonium, méthylamine, diméthylamine, triméthyl-amine, éthylamine, acides aminés de base, par exemple lysine et arginine, et dicyclohexylamine, et analogues.

L'invention se rapporte également aux formes tautomères, aux énantiomères, diastéréoisomères, épimères et aux sels organiques ou minéraux des composés de formule générale (II).

En particulier, les groupes alkyles substitués dans les composés de formule (II) sont substitués par au moins un groupe -OH ou un halogène, et notamment un chlore, un brome ou un fluor. De préférence, lesdits groupes alkyles substitués sont choisis parmi CH₂OH, CH₂Cl, CH₂F.

En particulier, les groupes aryles substitués dans les composés de formule (II) sont substitués par au moins un substituant choisi dans le groupe constitué de : halogène, et notamment un fluor ou un chlore ; COOCH₃ ; CH₂F ; CH₂Cl.

En particulier, les groupes hétéroaryles dans les composés de formule (II) sont non substitués.

En particulier, les groupes hétéroaryles substitués dans les composés de formule (II) sont substitués par au moins un substituant choisi dans le groupe constitué de : -C(=O)H, NO₂, halogène, tel que F ou Cl.

Selon un mode de réalisation, dans les composés de formule (II), R₁ représente un atome d'hydrogène.

Parmi les composés de l'invention on peut notamment citer les composés de formule (II-1) suivante : 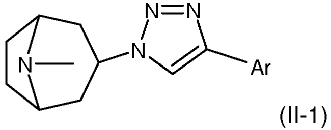 dans laquelle Ar est tel que défini ci-dessus.

Les composés de formule (II-1) sont des composés de formule (II) telle que définie ci-dessus, dans laquelle les groupes R_{N} et R_{N'} forment ensemble, avec l'atome de carbone auxquels ils sont liés, un groupe tropane.

Selon un mode de réalisation, pour les composés de formule (II-1), le groupe Ar représente un groupement phényle, éventuellement substitué par un ou plusieurs groupes choisis parmi :
- les atomes d'halogène,
- le groupe -OH,
- les groupes alkyles et alcoxyles linéaires ou ramifiés comprenant de 1 à 10 atomes de carbone, éventuellement substitués, et
- les groupes aryles comprenant de 6 à 30 atomes de carbone et les groupes hétéroaryles comprenant de 1 à 30 atomes de carbone, éventuellement substitués.

Parmi ces composés de formule (II-1), on peut donc citer les composés de formule (II-1-1) suivante : 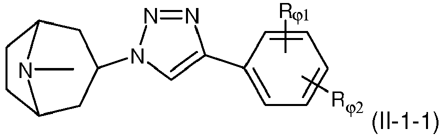 dans laquelle R_{ϕ1} et R_{ϕ2} sont choisis parmi H, les atomes d'halogène, les groupes alkyles et alkoxy linéaires ou ramifiés comportant de 1 à 10 atomes de carbone, éventuellement substitués.

En particulier, lorsque R_{ϕ1} et/ou R_{ϕ2} représente(nt) un groupe alkyle, ledit groupe alkyle est éventuellement substitué par au moins un substituant choisi dans le groupe constitué de : -OH et un atome d'halogène, tel qu'un fluor.

Selon un mode de réalisation, la présente invention concerne également des composés de formule (II-1-1-1) suivante : 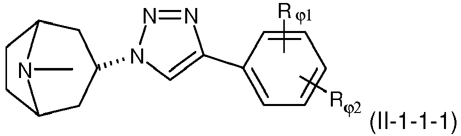 dans laquelle R_{ϕ1} et R_{ϕ2} sont tels que définis ci-dessus dans la formule (II-1-1).

Ainsi, la présente invention concerne les composés suivants :

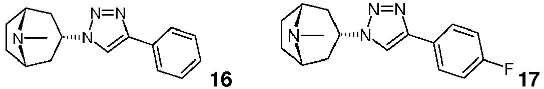

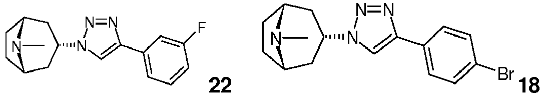

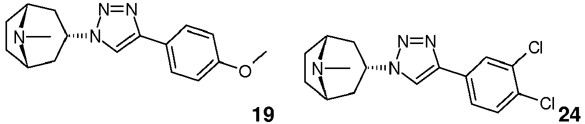

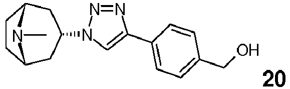

Selon un autre mode de réalisation, la présente invention concerne aussi les composés suivants :

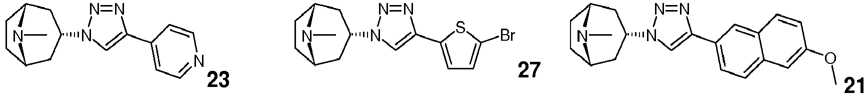

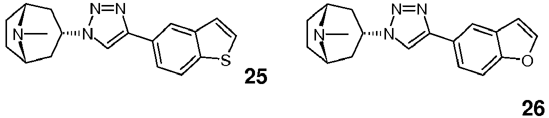

Parmi les composés de l'invention on peut notamment citer les composés de formule (II-2) suivante : 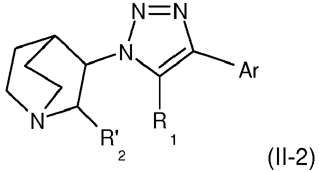 dans laquelle :
- les groupes R₁ et Ar sont tels que définis ci-dessus, et
- le groupe R'₂ représente un groupe alkyle linéaire ou ramifié comprenant de 1 à 10 atomes de carbone, éventuellement substitué par un groupe aryle comprenant de 6 à 30 atomes de carbone et un groupe hétéroaryle comprenant de 1 à 30 atomes de carbone.

Les composés de formule (II-2) sont des composés de formule (II) telle que définie ci-dessus, dans laquelle les groupes R_{N} et R_{N'} forment ensemble, avec l'atome de carbone auxquels ils sont liés, un groupe quinuclidine, éventuellement substitué.

Selon un mode de réalisation, pour les composés de formule (II-2), le groupe Ar représente un groupement phényle, éventuellement substitué par un ou plusieurs groupes choisis parmi :
- les atomes d'halogène, notamment F, Cl ou Br,
- le groupe -OH,
- les groupes alkyles et alcoxyles linéaires ou ramifiés comprenant de 1 à 10 atomes de carbone, éventuellement substitués, et
- les groupes aryles comprenant de 6 à 30 atomes de carbone ou les groupes hétéroaryles comprenant de 1 à 30 atomes de carbone, éventuellement substitués.

En particulier, dans la formule (II-2), lesdits groupes alkyles substitués sont substitués par au moins un groupe -OH ou un atome d'halogène, tel que F ou Cl.

En particulier, dans la formule (II-2), lesdits groupes aryles sont non substitués.

En particulier, dans la formule (II-2), lesdits groupes aryles substitués sont substitués par au moins un halogène, tel que F, Cl ou Br.

En particulier, dans la formule (II-2), lesdits groupes hétéroaryles sont non substitués.

En particulier, dans la formule (II-2), lesdits groupes hétéroaryles substitués sont substitués par au moins un substituant choisi dans le groupe constitué de : -C(=O)H, NO₂, et un halogène, tel que F.

Parmi les composés de formule (II-2), on peut citer les composés de formule (II-2-a) ou (II-2-b) suivantes : 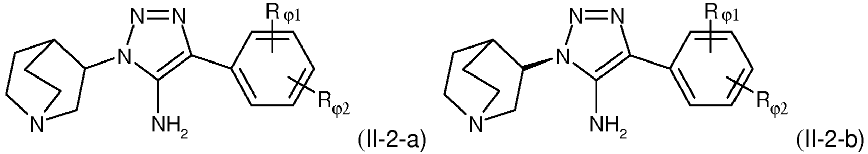 dans laquelle R_{ϕ1} et R_{ϕ2} sont choisis parmi H, les atomes d'halogène, les groupes alkyles et alkoxy linéaires ou ramifiés comportant de 1 à 10 atomes de carbone, éventuellement substitués.

Parmi ces composés, on peut par exemple citer le composé suivant :

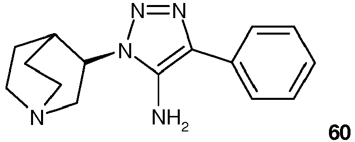

Pour les composés de formule (II-2), R₁ peut représenter H.

Pour les composés de formule (II-2), R'₂ peut représenter H.

Parmi ces composés de formule (II-2), on peut donc citer les composés de formule (II-2-1) suivante : 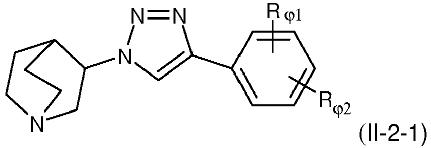 dans laquelle R_{ϕ1} et R_{ϕ2} sont choisis parmi H, les atomes d'halogène, les groupes alkyles et alkoxy linéaires ou ramifiés comportant de 1 à 10 atomes de carbone, éventuellement substitués.

Selon un mode de réalisation, dans la formule (II-2-1), au moins l'un de R_{ϕ1} et R_{ϕ2} représente un groupe alkyle substitué par un groupe -OH. En particulier, au moins l'un de R_{ϕ1} et R_{ϕ2} représente CH₂OH.

Selon un mode de réalisation, dans la formule (II-2-1) au moins l'un de R_{ϕ1} et R_{ϕ2} représente un groupe alkyle substitué par un halogène. En particulier, au moins l'un de R_{ϕ1} et R_{ϕ2} représente CH₂F ou CH₂Cl.

Selon un mode de réalisation, dans la formule (II-2-1), au moins l'un de R_{ϕ1} et R_{ϕ2} représente un halogène, et notamment F, Cl ou Br. En particulier, au moins l'un de R_{ϕ1} et R_{ϕ2} représente F, Cl ou Br.

Selon un mode de réalisation, dans la formule (II-2-1), au moins l'un de R_{ϕ1} et R_{ϕ2} représente un groupe alcoxy, et notamment un méthoxy.

Selon un mode de réalisation, la présente invention concerne des composés de formule (II-2-1-1) suivante : 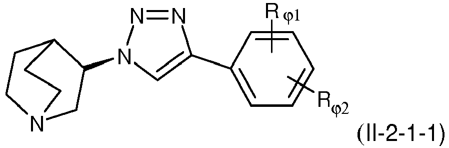 dans laquelle R_{ϕ1} et R_{ϕ2} sont tels que définis ci-dessus dans la formule (II-2-1).

Selon un mode de réalisation, la présente invention concerne des composés de formule (II-2-1-2) suivante : 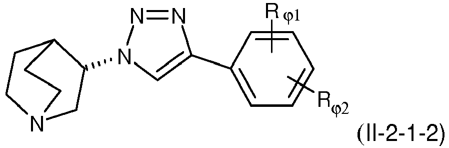 dans laquelle R_{ϕ1} et R_{ϕ2} sont tels que définis ci-dessus dans la formule (II-2-1).

Ainsi, la présente invention concerne les composés suivants :

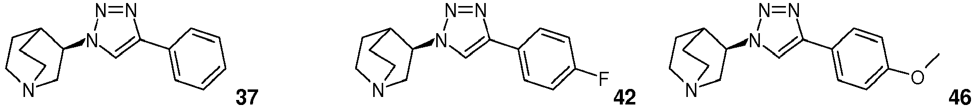

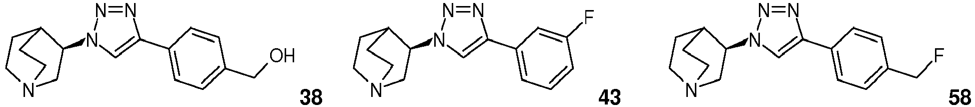

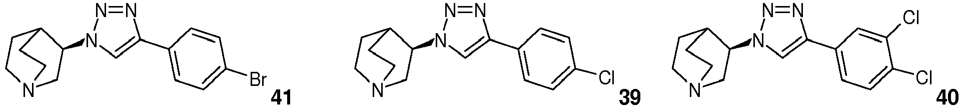

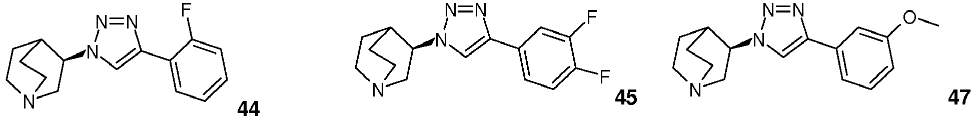

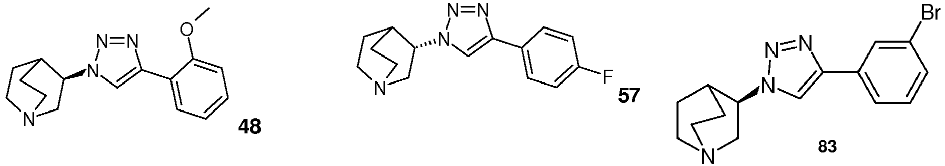

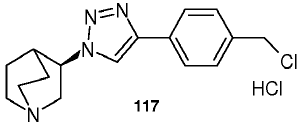

Selon un mode de réalisation, dans la formule (II-2), le groupe Ar représentant un groupe hétéroaryle mono- ou bicyclique est substitué par au moins un halogène, et notamment F ou Br.

Selon un autre mode de réalisation, dans la formule (II-2), le groupe Ar, représentant un groupe hétéroaryle mono- ou bicyclique, est substitué par au moins un groupe alcoxy non substitué, tel qu'un groupe méthoxy.

Selon un mode de réalisation, dans la formule (II-2), le groupe Ar représentant un groupe hétéroaryle mono- ou bicyclique n'est pas substitué.

Selon un mode de réalisation, dans la formule (II-2), le groupe Ar représentant un groupe hétéroaryle mono- ou bicyclique est substitué par au moins un groupe aryle comportant de 1 à 30 atomes de carbone, et notamment un groupe phényle, ledit groupe aryle étant substitué par au moins un substituant choisi dans le groupe constitué de : F, Cl, COOMe, CH₂F et CH₂Cl.

Selon un mode de réalisation, dans la formule (II-2), le groupe Ar représentant un groupe hétéroaryle mono- ou bicyclique est substitué par au moins un groupe hétéroaryle comportant de 1 à 30 atomes de carbone, éventuellement substitué par au moins un substituant choisi dans le groupe constitué de : NO₂ ou un halogène, et notamment F.

Ainsi, la présente demande décrit les composés suivants :

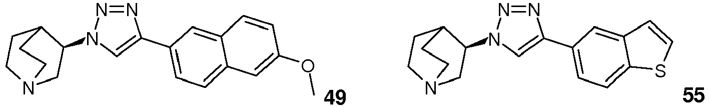

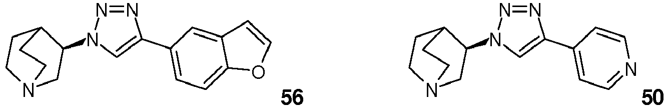

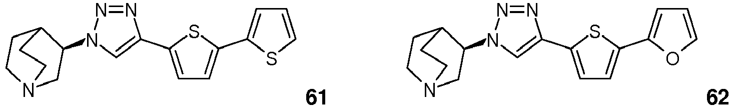

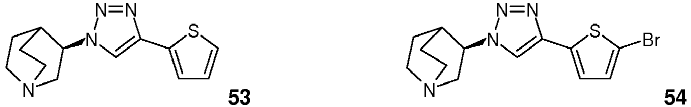

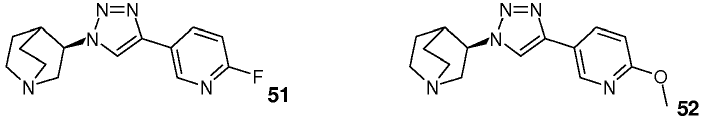

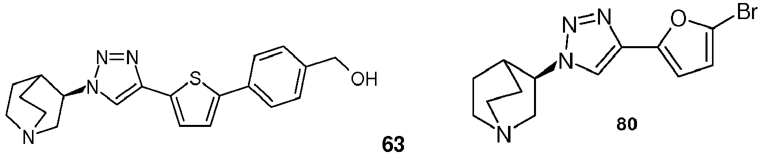

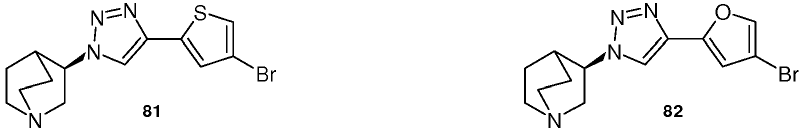

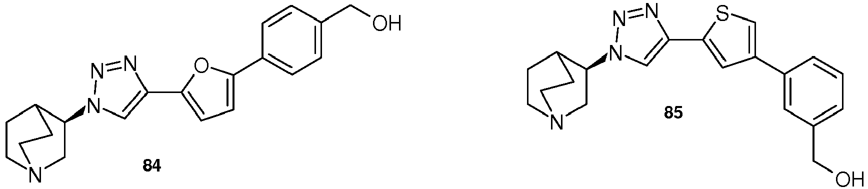

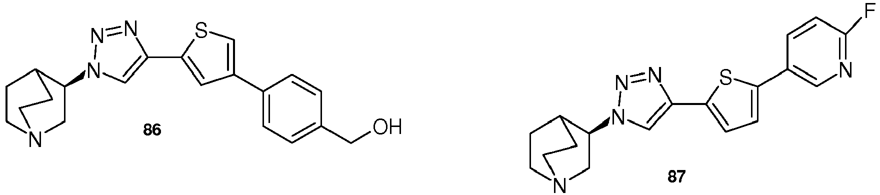

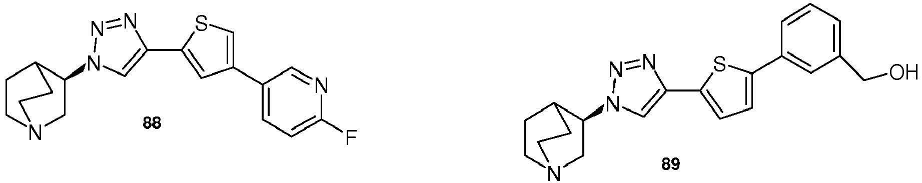

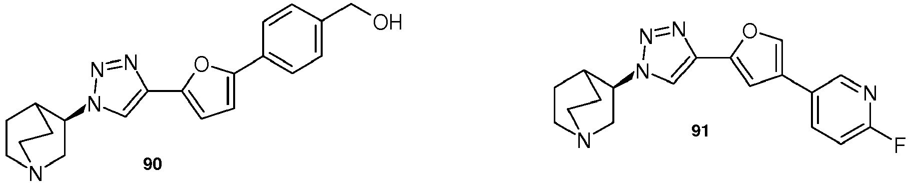

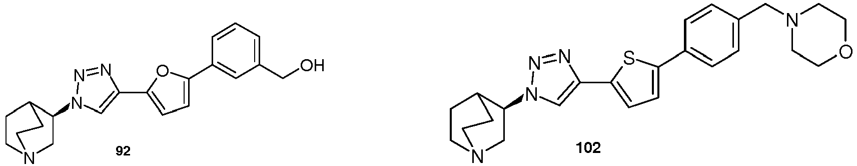

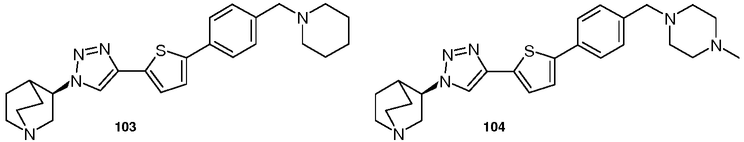

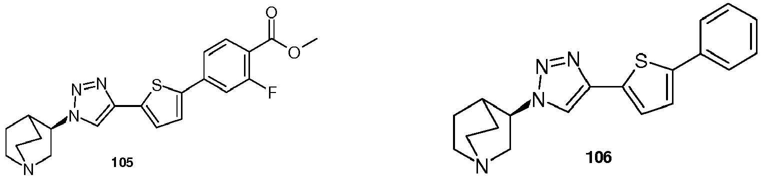

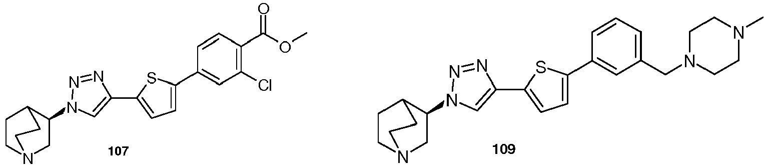

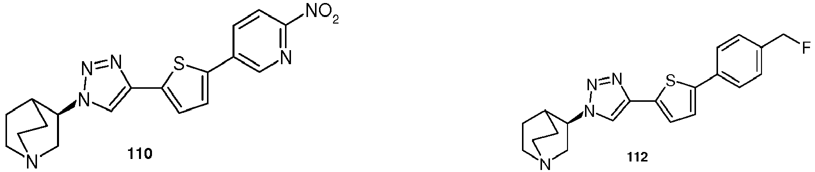

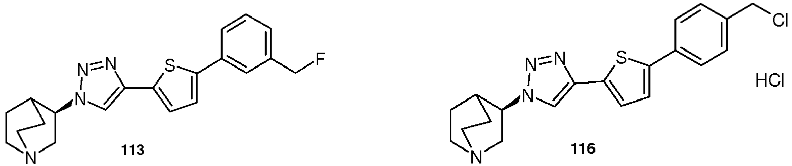

Parmi ces composés de formule (II-2), on peut également citer les composés de formule (II-2-3) suivante : 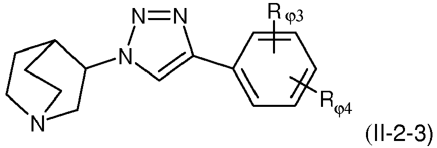 dans laquelle R_{ϕ3} et R_{ϕ4} sont choisis parmi les groupes aryles comprenant de 6 à 30 atomes de carbone ou les groupes hétéroaryles comprenant de 1 à 30 atomes de carbone.

Selon un mode de réalisation, dans la formule (II-2-3), au moins l'un de R_{ϕ3} et R_{ϕ4} représente un groupe hétéroaryle non substitué.

Selon un mode de réalisation, dans la formule (II-2-3), au moins l'un de R_{ϕ3} et R_{ϕ4} représente un groupe hétéroaryle substitué par un substituant choisi dans le groupe constitué de : -C(=O)H, NO₂ et un halogène, notamment F.

Selon un mode de réalisation, dans la formule (II-2-3), au moins l'un de R_{ϕ3} et R_{ϕ4} représente un groupe aryle substitué par au moins un groupe alkyle comprenant de 1 à 10 atomes de carbone, ledit groupe alkyle étant substitué par un halogène. En particulier, au moins l'un de R_{ϕ3} et R_{ϕ4} représente un phényle substitué par CH₂F ou CH₂Cl.

Ainsi, la présente demande décrit les composés suivants : 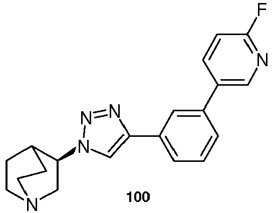 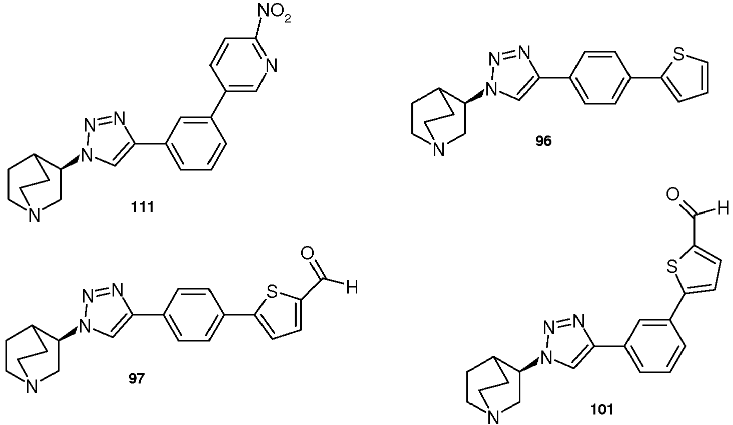 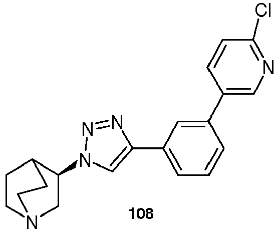 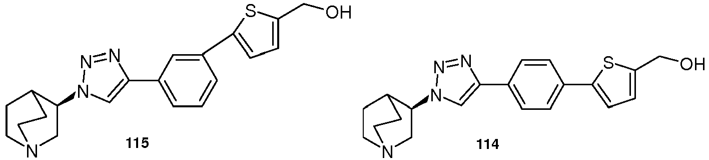 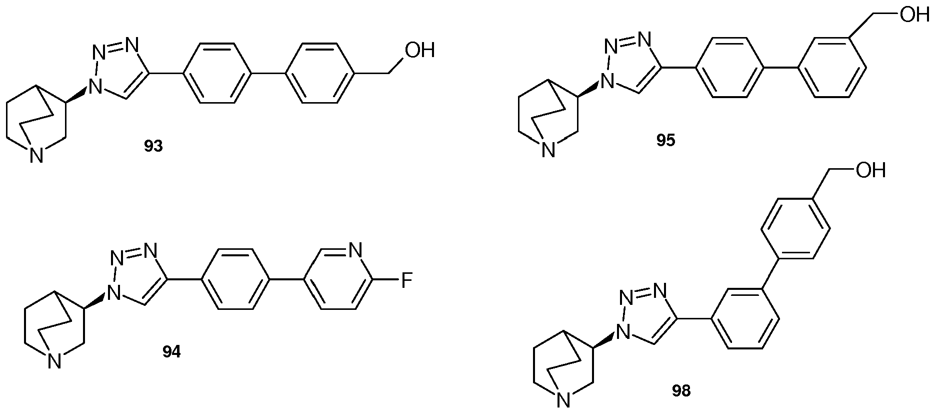 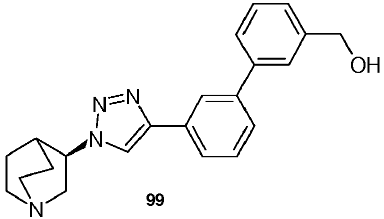 Parmi ces composés de formule (II-2), on peut citer les composés de formule (II-2-2) suivante : 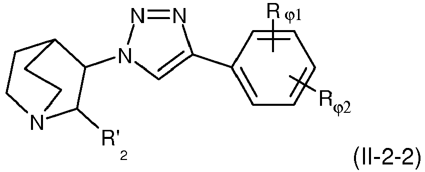 dans laquelle :
- les groupes R_{ϕ1} et R_{ϕ2} sont choisis parmi H, les atomes d'halogène, les groupes alkyles et alkoxy linéaires ou ramifiés comportant de 1 à 10 atomes de carbone, éventuellement substitués par un groupe hydroxy ou un halogène, et
- le groupe R'₂ représente H ou un groupe alkyle comportant de 1 à 10 atomes de carbone, éventuellement substitué par un groupe aryle comportant de 6 à 30 atomes de carbone ou par un groupe aryle hétéroaryle comportant de 1 à 30 atomes de carbone, par exemple un groupe pyridine.

Selon un mode de réalisation, la présente invention concerne des composés de formule (II-2-2-1) suivante :

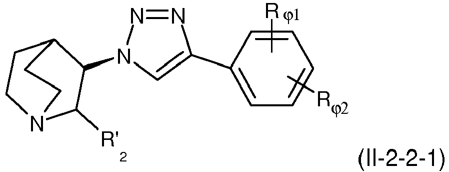

De préférence, pour les composés de formule (II-2-2) ou (II-2-2-1), R'₂ représente un groupe -CH₂pyridine.

Ainsi, la présente invention concerne le composé suivant :

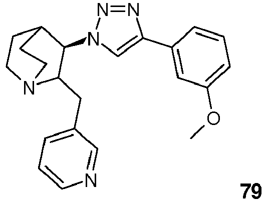

Parmi les composés de l'invention on peut notamment citer les composés de formule (II-3) suivante : 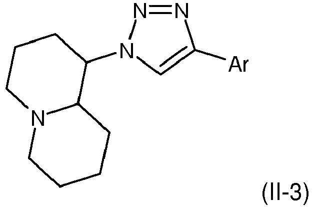 dans laquelle Ar est tel que défini ci-dessus.

Parmi les composés de l'invention on peut notamment citer les composés de formule (II-3-1) suivante : 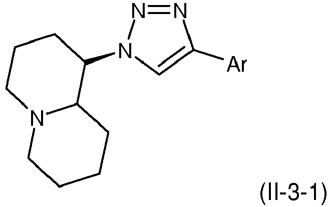 dans laquelle Ar est tel que défini ci-dessus.

Les composés de formule (II-3) sont des composés de formule (II) telle que définie ci-dessus, dans laquelle les groupes R_{N} et R_{N'} forment ensemble, avec l'atome de carbone auxquels ils sont liés, un groupe octahydroquinolizine, éventuellement substitué.

Ainsi, la présente invention concerne les composés suivants :

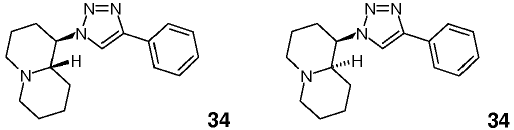

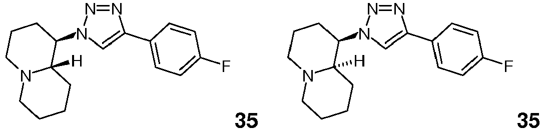

Selon un mode de réalisation particulier, les composés de l'invention comportent au moins un isotope radioactif choisi dans le groupe constitué de D, ¹⁸F, ¹¹C ou d ¹²³I.

Selon un mode de réalisation, les composés de l'invention comportent au moins un isotope radioactif choisi dans le groupe constitué de ¹⁸F ou ¹²³I.

Selon un mode de réalisation, le groupe R₂ des composés de l'invention est choisi dans le groupe constitué de ¹⁸F, ¹¹C ou ¹²³I.

Selon un mode de réalisation, le groupe R₂ des composés de l'invention est choisi dans le groupe constitué de ¹⁸F ou ¹²³I.

La présente invention concerne également les composés de formule (II) dans laquelle le groupe Ar représente un groupe phényle, thiophène, pyridine, furanne, substitué par au moins un groupe alkyle comprenant de 1 à 10 atomes de carbone substitué par au moins un isotope radioactif choisi dans le groupe constitué de ¹⁸F, ¹¹C et ¹²³I, de préférence ¹⁸F et ¹²³I.

Ainsi, la présente invention concerne en particulier le composé suivant :

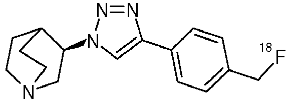

La présente invention concerne également les composés de formule (II) dans laquelle le groupe Ar représente un groupe phényle, thiophène, pyridine, furanne, substitué par un groupe aryle ou hétéroaryle comprenant de 1 à 30 atomes de carbone, par exemple un groupe phényle, thiophène, pyridine, furanne, substitué par au moins un groupe alkyle comprenant de 1 à 10 atomes de carbone substitué par au moins un isotope radioactif choisi dans le groupe constitué de ¹⁸F, ¹¹C et ¹²³I, de préférence ¹⁸F et ¹²³I.

La présente invention concerne également une composition pharmaceutique comprenant un composé de formule (II) telle que définie ci-dessus, ou tout composé tel que mentionné ci-dessus, en association avec un véhicule pharmaceutiquement acceptable.

La présente invention concerne donc un composé tel que défini ci-dessus de formule (II) pour son utilisation comme médicament.

La présente invention concerne aussi un médicament comprenant un composé de formule (II), éventuellement sous forme de sel pharmaceutiquement acceptable, d'hydrate ou de structure cristalline polymorphique, de racémate, diastéréoisomère ou d'énantiomère.

La présente invention concerne aussi une composition pharmaceutique comprenant un composé de formule (II), éventuellement sous forme de sel pharmaceutiquement acceptable, d'hydrate ou de structure cristalline polymorphique, de racémate, diastéréoisomère ou d'énantiomère, et un excipient pharmaceutiquement acceptable.

Les compositions pharmaceutiques selon l'invention peuvent être présentées sous des formes destinées à l'administration par voie parentérale, orale, rectale, permuqueuse ou percutanée.

Les compositions pharmaceutiques incluant ces composés de formule générale (II) seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalinoterreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0,5 mg à 1 000 mg) en fonction de l'indication thérapeutique et de la voie d'administration, ainsi que de l'âge et du poids du sujet.

La présente invention concerne également un composé tel que défini ci-dessus de formule (II), ou tout composé tel que mentionné ci-dessus, pour son utilisation en tant qu'agoniste du récepteur nicotinique alpha 7 (Rα7).

La présente invention concerne aussi un composé tel que défini ci-dessus de formule (II), ou tout composé tel que mentionné ci-dessus, pour son utilisation en tant qu'antagoniste du récepteur nicotinique alpha 7 (Rα7).

La présente invention concerne également un composé tel que défini ci-dessus de formule (II), ou tout composé tel que mentionné ci-dessus, pour son utilisation dans le cadre du traitement ou de la prévention de maladies liées au dérèglement des systèmes cholinergiques et impliquant le récepteur nicotinique alpha 7 (Rα7).

Plus particulièrement, lesdites maladies sont choisies dans le groupe constitué des maladies du système nerveux central, telles que l'addiction, les affections psychiatriques par exemple la schizophrénie et les troubles de l'attention, les affections neurodégénératives par exemple la maladie d'Alzheimer.

Lesdites maladies sont également choisies dans le groupe constitué des désordres cholinergiques du système nerveux central (SNC) ou du système nerveux périphérique (SNP), les maladies ou désordres liés à la contraction des muscles lisses, les maladies endocrines, les maladies ou désordres de maladies neurodégénératives, les maladies ou désordres relatifs à l'inflammation, la douleur et les syndromes liés au sevrage lors de la suppression de l'abus de substances chimiques.

Les maladies du SNC concernées sont nombreuses et variées. Concernant les maladies neurodégénératives, on peut citer les maladies d'Alzheimer et de Parkinson qui sont associées à des désordres cognitifs liés à un déficit des fonctions cholinergiques. Les troubles psychiatriques associés à une altération des récepteurs nicotiniques centraux comprennent la schizophrénie, mais aussi la dépression et l'anxiété. On peut noter que plus récemment un effet bénéfique d'antagonistes cholinergiques a été proposé comme nouvelle approche thérapeutique dans l'autisme. L'une des applications thérapeutiques les plus prometteuses pour les agonistes cholinergiques concerne la douleur. Par ailleurs la nicotine ou des agonistes partiels peuvent aider au sevrage addictif (en particulier tabagique). On peut enfin citer des applications potentielles pour le traitement de l'épilepsie ou du syndrome de Gilles de la Tourette.

Dans les désordres associés au SNP, les applications potentielles des ligands des Rα7 sont également très variées. On peut citer des maladies liées à la contraction des muscles lisses ou du myocarde, des maladies endocrines, des maladies liées à l'inflammation.

Les composés de l'invention peuvent être utilisés comme agent de diagnostic, prévention ou curatif ou être éléments de méthodes pour certaines pathologies ou désordres, parmi lesquelles on peut citer l'anxiété, les désordres cognitifs, les déficits ou dysfonctions d'apprentissage et de mémoire, les déficits d'attention, l'hyperactivité, la maladie d'Alzheimer, la maladie de Parkinson, la maladie d'Huntington, la sclérose latérale amyothrophique, le syndrome de Gilles de la Tourette, la dépression, les manies, la maniacodépression, la psychose, la schizophrénie, les désordres compulsifs obsessionnels, les désordres paniques, les désordres alimentaires incluant l'anorexie nécrotique, la boulimie et l'obésité, la narcolepsie, la nociception, l'AIDS-démence, la démence sénile, les déficits cognitifs, les déficiences légères de la cognition, la déficience de la mémoire liée à l'âge, la maladie de Pick, la démence associée au corps de Lewi, le syndrome de Down, la sclérose amyothrophique latérale, la maladie de Huntington, la diminution de la fonction cérébrale liée au traumatisme crânien, la neuropathie périphérique, l'autisme, la dyslexie, la dyskénie tardive, l'hyper kinésie, l'épilepsie, les syndromes post traumatiques, les phobies sociales, les troubles du sommeil, les pseudodémences, le syndrome de Ganser, le syndrome pré-menstruel, le désordre dystrophique de la phase tardive lutéale (LLPDD), la fatigue chronique, le mutisme, la tricotillomanie, le jet-lag, l'hypertension, l'arythmie cardiaque, les désordres de contraction des muscles lisses incluant les désordres convulsifs, l'angine de poitrine, les convulsions, diarrhées, asthme, épilepsie, dyskénie tardive, hyperkinésie, éjaculation précoce et difficultés érectiles, les désordres du système endocrinien incluant la thyrotoxicité et phéochromocytose, les désordres neurodégénératifs incluant l'anoxie transitoire et la neurodégénérescence, la douleur moyenne, sévère, modérée, aigue, chronique ou récurrente, la douleur neuropathique, la douleur migraineuse, la douleur postopératoire, la douleur fantomatique, les maux de tête, la douleur centrale, la douleur associée aux neuropathies diabétiques, les complications liées au diabète, et autres activités systémiques et neuroimmunomodulatrices, les neuralgies post-thérapeutiques ou des blessures nerveuses périphériques, les désordres inflammatoires incluant l'inflammation cutanée, acné, et rosacae, la maladie de Crohn, les inflammations intestinales, les colites ulcéreuses et diarrhées, le sevrage tabagique, l'ischémie, la sepsie, la cicatrisation de plaies, les syndromes associés au retrait ou à la terminaison de prises de substances chimiques addictives incluant la nicotine, héroïne, cocaïne, morphine, benzodiazépine, analogues de benzodiazépine et alcool.

Les composés de l'invention peuvent être utilisés comme outils de diagnostic, agent de monitoring dans des méthodes de diagnostic et en particulier pour l'imagerie de récepteurs in vivo (neuro-imagerie) sous leur forme marquée ou non.

Les composés de l'invention peuvent être utilisés pour le traitement de maladies addictives liées à l'arrêt de l'utilisation de substances addictives. Ces substances incluent les composés contenant de la nicotine comme le tabac, les opiacés comme l'héroïne, la cocaïne, morphine, cannabis, benzodiazépines et dérivés de benzodiazépines, et l'alcool. Le sevrage de ces substances addictives est en général une expérience traumatique caractérisée par une anxiété, une frustration, des difficultés de concentration, de la colère, une agitation, une décroissance du rythme cardiaque, une augmentation de l'appétit et un gain de poids. Dans ce contexte « traitement » couvre le traitement, la prévention, la prophylaxie et soulagement de ces effets de sevrage et donc l'abstinence aussi bien que du traitement résulte une consommation réduite de la substance addictive.

La présente invention concerne également l'utilisation des composés de l'invention comportant au moins un isotope radioactif choisi parmi D, ¹⁸F, ¹¹C ou ¹²³I tels que définis ci-dessus, pour son utilisation comme marqueur radiopharmaceutique.

La présente invention concerne également l'utilisation des composés de l'invention comportant au moins un isotope radioactif choisi parmi D, ¹⁸F, ¹¹C ou ¹²³I tels que définis ci-dessus, pour son utilisation comme marqueur radiopharmaceutique dans le diagnostic précoce et le suivi de maladies du système nerveux central, en particulier de la maladie d'Alzheimer.

La présente invention concerne également l'utilisation des composés de l'invention comportant au moins un isotope radioactif choisi parmi D, ¹⁸F, ¹¹C ou ¹²³I tels que définis ci-dessus, pour son utilisation comme marqueur radiopharmaceutique du Rα7.

De tels composés radiopharmaceutiques sont particulièrement intéressants en tant que ligand radiomarqué, aussi appelé radiotraceur ou encore radiomarqueur, pour le suivi de maladies impliquant le Rα7, telles que des maladies du système nerveux central, en particulier de la maladie d'Alzheimer.

De tels composés, en particulier ceux comportant un isotope ¹⁸F ou ¹¹C, sont adaptés à l'utilisation en imagerie TEP (tomographie par émission de positons) pour le diagnostic précoce de la maladie d'Alzheimer.

De tels composés, en particulier ceux comportant un isotope ¹²³I, sont adaptés à l'utilisation en imagerie TEMP (tomographie d'émission monophotonique) pour le diagnostic précoce de la maladie d'Alzheimer.

La TEP est basée sur l'utilisation de composés radiopharmaceutiques et permet l'exploration de fonctions biologiques *in vivo* de manière atraumatique ainsi que la mise en évidence de lésions cérébrales apparaissant au cours de maladies neurodégénératives comme la maladie d'Alzheimer. LA TEP est un technique particulièrement adaptée au diagnostic précoce et le suivi de l'efficacité de nouveaux traitements.

La TEP permet l'exploration le suivi *in vivo* de manière atraumatique de changements physiologiques à l'échelle moléculaire. Cette technique d'imagerie utilise des médicaments radiopharmaceutiques spécifiques d'une cible moléculaire, tels que des ligands de récepteurs, marqués par un radio-isotope émetteur de positons tel que le ¹⁸F.

Le diagnostic de la maladie d'Alzheimer repose actuellement sur des critères cliniques n'offrant qu'un diagnostic de probabilité.

La visualisation des lésions *in vivo,* causées par la maladie d'Alzheimer, par des techniques d'imagerie moléculaire constitue un outil de diagnostic sensible et spécifique de cette maladie.

L'imagerie moléculaire (TEP ou TEMP) permet d'apporter des informations sur l'état d'une fonction à l'échelle moléculaire même en l'absence de modifications anatomiques.

L'utilisation de composés de l'invention marqués par un isotope radioactif en imagerie moléculaire permet donc de réaliser un diagnostic précoce et différentiel de maladies telles que la maladie d'Alzheimer et de suivre l'efficacité des thérapies.

La présente invention concerne également un procédé de préparation d'un composé de formule (II) telle que définie ci-dessus,
ledit procédé comprenant une étape de formation d'un cycle 1,2,3-triazole par mise en présence d'un composé de formule (II-a) suivante et d'un composé de formule (II-b) suivante :

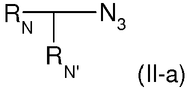

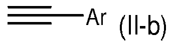

Cette étape de formation d'un cycle 1,2,3-triazole est effectuée de préférence en présence de sels de cuivre (II), par exemple de CuSO₄.5H₂O. Elle est également de préférence effectuée en présence d'ascorbate de sodium. Elle est de préférence effectuée à température ambiante et pendant environ 20 heures.

De préférence, dans les composés de formule (II), R₁ = H.
Dans le cas où R₁ est différent de H, cette étape est éventuellement suivie d'une étape de fonctionnalisation de la position 5 du noyau 1,2,3-triazole, ce par quoi on obtient le groupement R₁ des composés (II).

La présente invention concerne également un autre procédé de préparation d'un composé intermédiaire de synthèse de formule (II-a) telle que définie ci-dessus, ledit procédé comprenant une étape d'azidation in situ, notamment en présence d'un réactif d'azidation tel que l'azoture de sulfo-imidazole par exemple en présence de sulfate de cuivre hydraté, d'ascorbate de sodium et de carbonate de sodium dans le méthanol, d'un composé (II-a-1) de formule suivante : 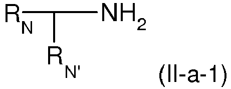 sous forme libre ou sous forme de sel, par exemple de sel de chlorhydrate.

L'étape d'azidation susmentionnée est effectuée de préférence à température ambiante pendant environ 20 heures, et peut être effectuée in situ.

La présente invention concerne également un autre procédé de préparation d'un composé intermédiaire de synthèse de formule (II-a) telle que définie ci-dessus,
ledit procédé comprenant une étape d'azidation, notamment en présence d'un réactif d'azidation tel que l'azoture de sodium dans la DMF à 140°C, d'un composé (II-a-2) de formule suivante : 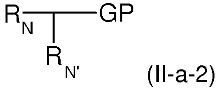 dans laquelle le groupe -GP représente un groupe partant, comme par exemple un groupe -Oms ou -OTs.

L'étape d'azidation susmentionnée est effectuée de préférence à 75°C pendant environ 18 heures.

La présente invention concerne également un autre procédé de préparation d'un composé de formule (II) telle que définie ci-dessus
dans laquelle
R₁ représente un groupe -NH₂,
ledit procédé comprenant une étape de formation d'un cycle 1,2,3-triazole par mise en présence d'un composé de formule (II-a) telle que définie ci-dessus et d'un composé de formule (II-c) suivante :

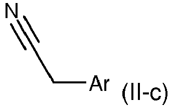

Cette étape de formation d'un cycle 1,2,3-triazole est effectuée de préférence en présence de sels de cuivre (II), par exemple de CuSO₄.5H₂O. Elle est également de préférence effectuée en présence d'ascorbate de sodium. Elle est de préférence effectuée à température ambiante et pendant environ 20 heures.

La présente demande décrit également un procédé de préparation d'un composé de formule (I-F-1) suivante : 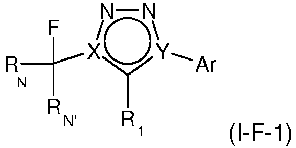 dans laquelle les groupes X, Y, Ar, R₁, R_{N} et R_{N'} sont tels que définis ci-dessus,
ledit procédé comprenant une étape de fluoration, notamment en présence d'un réactif de fluoration, d'un composé de formule (I-OH-1) suivante :

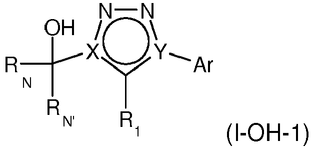

Cette étape de fluoration est effectuée de préférence en présence d'un réactif de fluoration choisi parmi le tetrafluorure de soufre SF₄, le bis(2-méthoxyéthyl)aminotrifluorosulfure (deoxo-fluor) et le diéthylaminotrifluosulfure (DAST, NEt₂SF₃), de préférence à 0°C pendant environ une heure.
De préférence, cette étape de fluoration est effectuée en présence d'un réactif de fluoration comportant des radioéléments ¹⁸F.

De préférence, dans les composés de formule (I-F-1) et (I-OH-1) :
- R₁ représente H, et/ou
- les groupes R_{N} et R_{N'} forment ensemble, avec l'atome de carbone auxquels ils sont liés, un groupe quinuclidine, et/ou
- le groupe Ar représente un groupe phényle, éventuellement substitué par un brome ou un méthoxy, et/ou
- X représente C et Y représente N.

La présente demande décrit également un procédé de préparation d'un composé de formule (I-F-2) suivante : 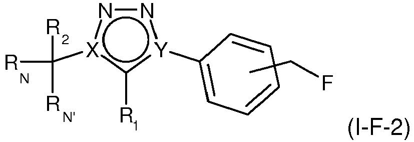 dans laquelle les groupes X, Y, R₁, R₂, R_{N} et R_{N'} sont tels que définis ci-dessus,
ledit procédé comprenant une étape de fluoration, notamment en présence d'un réactif de fluoration, d'un composé de formule (I-OH-2) suivante :

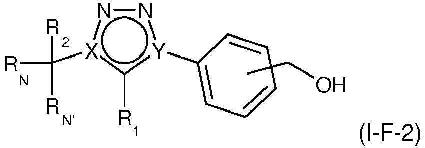

Cette étape de fluoration est effectuée de préférence en présence d'un réactif de fluoration choisi parmi le tétrafluorure de soufre SF₄, le bis(2-méthoxyéthyl)aminotrifluorosulfure (deoxo-fluor) et le diéthylaminotrifluosulfure (DAST, NEt₂SF₃), de préférence à 0°C pendant environ une heure.

De préférence, cette étape de fluoration est effectuée de manière directe ou indirecte en présence d'un réactif de fluoration comportant des radioéléments ¹⁸F.

De préférence, dans les composés de formule (I-F-2) et (I-OH-2) :
- R₁ représente H, et/ou
- R₂ représente H, et/ou
- les groupes R_{N} et R_{N'} forment ensemble, avec l'atome de carbone auxquels ils sont liés, un groupe quinuclidine, et/ou
- X représente N et Y représente C.

### EXEMPLES

### PRÉPARATION DES COMPOSÉS DE L'INVENTION

### Préparation de précurseurs de composés de type tropane (formule (II-1))

### Préparation de la 3-endo-tropanamine (2) :

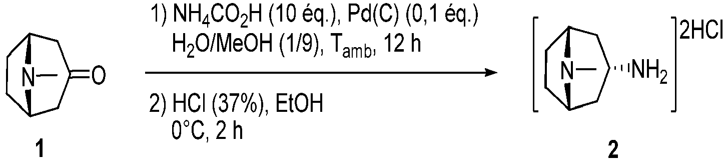

**3-*Endo*-tropanamine (2).** Une solution de formate d'ammonium (25,0 g, 0,40 mol) dans 12,5 mL d'eau est ajoutée à une solution de tropanone **1** (6,00 g, 43,0 mmol) dans 110 mL de méthanol. Après solubilisation totale du milieu réactionnel, du Pd/C (4,60 g, 4,30 mmol) est additionné puis la réaction est agitée à température ambiante pendant 12 heures en prenant soin de bien laisser échapper le dihydrogène formé. Ensuite, le milieu réactionnel est filtré sur célite puis le filtrat est évaporé sous pression réduite. L'huile obtenue est diluée dans 100 mL d'éthanol puis à 0°C, 7,5 mL d'une solution de HCl concentrée (37%) est ajoutée goutte à goutte. Enfin, la solution est agitée pendant 2 heures jusqu'à précipitation totale de la 3-*endo*-tropanamine **2.** Le produit est récupéré par filtration sous vide sous la forme d'un solide blanc avec un rendement de 98%. Mp : >250°C ; IR (ATR, Diamond) : *v*(cm⁻¹) : 1032, 1068, 1104, 1175, 1230, 1356, 1385, 1448, 1517, 1532, 1606, 2047, 2551, 2587, 2627, 2649, 2764, 2876 ; RMN ¹H (250 MHz, DMSO-*d₆*) : *δ*(ppm) 2,05 - 2,40 (m, 6H), 2,62 - 2,80 (m, 5H), 3,40 - 3,60 (m, 1H), 3,70 - 3,92 (m, 2H), 8,32 - 8,75 (m, 3H), 10,98 - 11,34 (m, 1H) ; RMN ¹³C (100 MHz, DMSO-*d₆*) : *δ*(ppm) 23,2 (2CH₂), 31,9 (2CH₂), 37,9 (CH₃), 39,2 (CH), 60,2 (2CH) ; MS (IS) : m/z = 141,0 [MH]⁺.

### Préparation d'alcynes vrais (7-9) à partir d'aldéhydes (3-5)

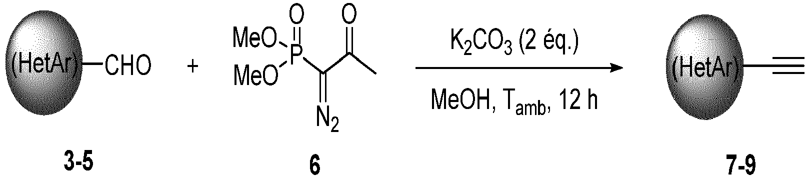

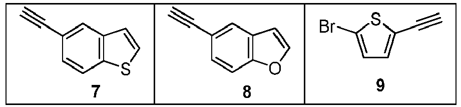

**Procédure générale A**: A une solution de dimethyl 1-diazo-2-oxopropylphosphonate **6** (1,15 g, 6,00 mmol) dans 60 mL de méthanol anhydre sont ajoutés successivement l'aldéhyde **3-5** (5,00 mmol) et du K₂CO₃ (1,38 g, 10,0 mmol). Le milieu réactionnel est agité à température ambiante pendant 12 h. Après évaporation douce du solvant sous pression réduite, les alcynes **7-9** sont purifiés par chromatographie sur colonne de gel de silice avec comme éluant un mélange éther de pétrole/acétate d'éthyle permettant une bonne séparation.

**5-Ethynylbenzo[*b*]thiophène (7).** Le produit est isolé sous la forme d'un solide blanc avec un rendement de 84% en suivant la procédure générale A. R*_{f}*: 0,40 (EP/AcOEt : 99/1) ; Mp: 55°C; IR (ATR, Diamond): *v*(cm⁻¹) : 1050, 1088, 1222, 1258, 1324, 1411, 1431, 3082, 3101, 3277 ; RMN ¹H (250 MHz, CDCl₃) : *δ*(ppm) 3,11 (s, 1H), 7,31 (d, 1H, *J* = 5,5 Hz), 7,43 - 7,51 (m, 2H), 7,83 (d, 1H, *J* = 8,4 Hz), 7,99 (s, 1H) ; RMN ¹³C (100 MHz, CDCl₃) : *δ*(ppm) 76,9 (CH), 84,1 (C_{q}), 118,1 (C_{q}), 122,6 (CHₐᵣₒ), 123,8 (CHₐᵣₒ), 127,6 (CHₐᵣₒ), 127,7 (2CHₐᵣₒ), 139,6 (C_{q}), 140,3 (C_{q}) ; MS (IS) : m/z = 159,1 [MH]⁺.

**5-Ethynylbenzo[*b*]furane (8).** Le produit est isolé sous la forme d'une huile jaune avec un rendement de 90% en suivant la procédure générale A. R*_{f}*: 0,52 (EP/AcOEt : 95/5) ; IR (ATR, Diamond) : *v*(cm⁻¹) : 882, 1029, 1107, 1118, 1195, 1265, 1329, 1436, 1460, 3290 ; RMN ¹H (250 MHz, CDCl₃) : *δ*(ppm) 3,03 (s, 1H), 6,75 (d, 1H, *J* = 2,2 Hz), 7,43 - 7,46 (m, 2H), 7,64 (d, 1H, *J* = 2,2 Hz), 7,77 (s, 1H) ; RMN ¹³C (100 MHz, CDCl₃) : *δ*(ppm) 76,0 (CH), 84,2 (C_{q}), 106,7 (CHₐᵣₒ), 111,7 (CHₐᵣₒ), 116,8 (C_{q}), 125,6 (CHₐᵣₒ), 127,7 (C_{q}), 128,6 (CHₐᵣₒ), 146,1 (CHₐᵣₒ), 155,0 (C_{q}) ; MS (IS) : m/z = 143,1 [MH]⁺.

**2-Bromo-5-éthynylthiophène (9).** Le produit est isolé sous la forme d'une huile jaune avec un rendement de 92% en suivant la procédure générale A. R*_{f}*: 0,50 (EP/AcOEt: 99/1) ; IR (ATR, Diamond) : *v*(cm⁻¹) : 966, 1048, 1137, 1209, 1419, 1519, 1669, 2102, 3294 ; RMN ¹H (250 MHz, CDCl₃) : *δ*(ppm) 3,37 (s, 1H), 6,92 (d, 1H, *J* = 3,9 Hz), 7,02 (d, 1H, *J* = 3,9 Hz) ; RMN ¹³C (100 MHz, CDCl₃) : *δ*(ppm) 76,2 (CH), 82,5 (C_{q}), 113,7 (C_{q}), 124,0 (C_{q}), 130,1 (CHₐᵣₒ), 133,6 (CHₐᵣₒ) ; MS (IS) : m/z = 188,1 [MH]⁺.

### Préparation d'alcynes (13-14) par la réaction de couplage de Sonoaashira

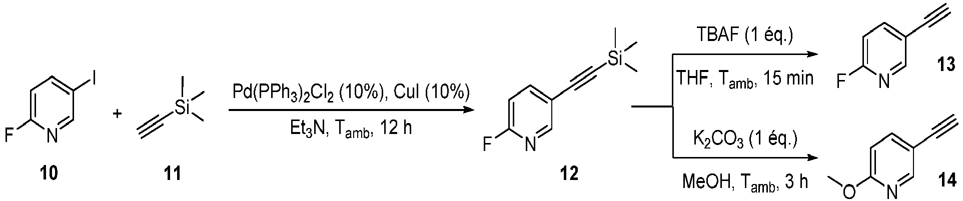

A une solution de la 2-fluoro-5-iodopyridine **10** (4,00 g, 17,9 mmol) dans 30 mL de triéthylamine préalablement dégazée sont ajoutés successivement l'éthynyl(triméthyl)silane **11** (2,78 mL, 19,7 mmol), du iodure de cuivre (340 mg, 1,79 mmol) et du Pd(PPh₃)₂Cl₂ (1,25 g, 1,79 mmol). La réaction est agitée à température ambiante pendant 12 heures. A l'issu de la réaction, le milieu réactionnel est dilué dans 100 mL d'eau puis extrait avec de l'éther éthylique. La phase organique est séchée sur MgSO₄ anhydre, filtrée puis concentrée sous pression réduite. Le produit de couplage **12** est engagé dans les réactions suivantes sans aucune autre purification.

**5-Ethynyl-2-fluoropyridine (13).** Le dérivé silylé **12** (8,97 mmol) est agité dans 50 mL de THF pendant 15 minutes en présence de 9,00 mL d'une solution de fluorure de tetra-*n-*butylammonium (1 M dans le THF) ajoutée goutte à goutte. A l'issu de la réaction, le solvant est évaporé et l'alcyne **13** est purifié par chromatographie sur colonne de gel de silice en utilisant comme éluant un mélange éther de pétrole/acétate d'éthyle (95/5). Le produit est isolé sous la forme d'un liquide rouge avec un rendement de 82%. R*_{f}*: 0,49 (EP/AcOEt : 95/5) ; IR (ATR, Diamond) : *v* (cm⁻¹) : 927, 1020, 1130, 1240, 1365, 1477, 1575, 3074 ; RMN ¹H (250 MHz, CDCl₃) : *δ*(ppm) 3,19 (s, 1H), 6,90 (dd, 1H, *J* = 8,5 Hz et 3,0 Hz), 7,85 (ddd, 1H, *J* = 8,5 Hz, 7,6 Hz et 2,3 Hz), 8,34 (d, 1H, *J* = 2,3 Hz) ; RMN ¹³C (100 MHz, CDCl₃) : *δ*(ppm) 79,2 (CH), 80,7 (d, C_{q}, *J* = 1 Hz), 109,6 (d, CHₐᵣₒ, *J* = 38 Hz), 117,3 (d, C_{q}, *J* = 5 Hz), 144,4 (d, CHₐᵣₒ, *J* = 8 Hz), 151,4 (d, CHₐᵣₒ, *J* = 15 Hz), 163,1 (d, C_{q}, *J* = 243 Hz) ; MS (IS) : m/z = 122,2 [MH]⁺.

**5-Ethynyl-2-méthoxypyridine (14).** Le dérivé silylé **12** (17,9 mmol) est agité dans 60 mL de méthanol pendant 3 heures en présence de K₂CO₃ (2,47 g, 17,9 mmol). Le milieu réactionnel est évaporé sous pression réduite et l'alcyne **14** est purifié par chromatographie sur colonne de gel de silice en utilisant comme éluant un mélange éther de pétrole/acétate d'éthyle (95/5). Le produit est isolé sous la forme d'un liquide jaune avec un rendement de 75%. R*_{f}*: 0,72 (EP/AcOEt : 96/4) ; IR (ATR, Diamond) : *v*(cm⁻¹) : 1021, 1126, 1251, 1284, 1305, 1367, 1488, 1559, 1600, 3290 ; RMN ¹H (250 MHz, CDCl₃) : *δ*(ppm) 3,10 (s, 1H), 3,94 (s, 3H), 6,69 (d, 1H, *J* = 8,6 Hz), 7,63 (dd, 1H, *J* = 8,6 Hz et 2,3 Hz), 8,30 (d, 1H, *J* = 2,3 Hz) ; RMN ¹³C (100 MHz, CDCl₃) : *δ*(ppm) 53,9 (CH₃), 78,8 (CH), 80,9 (C_{q}), 110,9 (CHₐᵣₒ), 112,1 (C_{q}), 141,8 (CHₐᵣₒ), 151,0 (CHₐᵣₒ), 163,9 (C_{q}) ; MS (IS) : m/z = 134,0 [MH]⁺.

### Préparation des composés 16-27 de type tropane (formule (II-1))

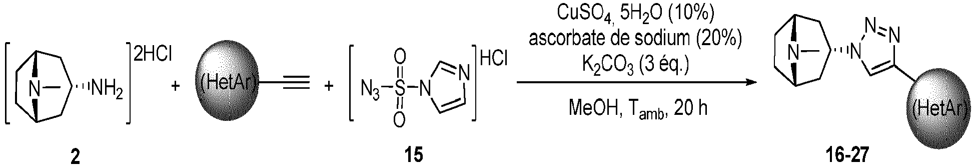

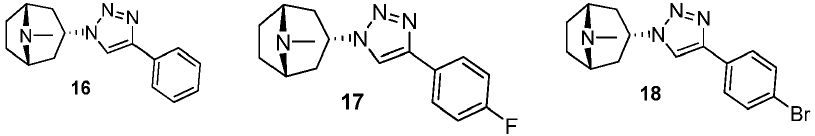

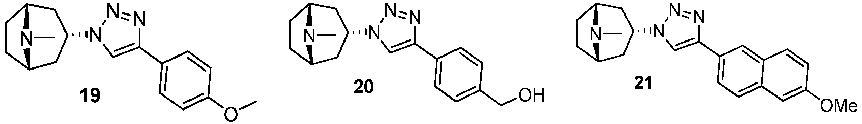

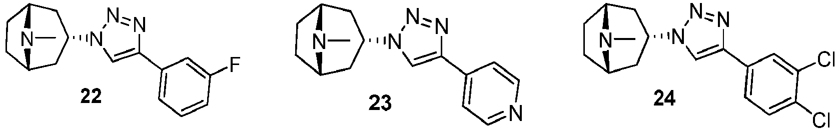

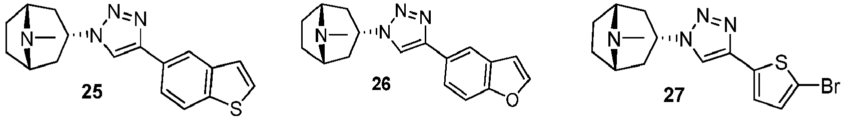

**Procédure générale B** : Sous argon, la 3-*endo*-tropanamine **2** (212 mg, 1,00 mmol) et l'azoture du 1*H*-imidazole-1-sulfonyle **15** (232 mg, 1,10 mmol) sont solubilisés dans 6 mL de méthanol puis sont ajoutés successivement du K₂CO₃ (415 mg, 3,00 mmol) et une quantité catalytique de CuSO₄,5H₂O (25 mg, 0,100 mmol). Le milieu réactionnel est agité à température ambiante pendant 2 h puis concentré sous pression réduite. Le solide obtenu est alors repris dans 10 mL d'éther éthylique, filtré sous vide puis lavé deux fois avec 10 mL d'éther éthylique. Enfin, le filtrat est évaporé sous pression réduite et l'azoture ainsi obtenu est engagé dans l'étape suivant sans aucune autre purification.

Ce dernier est solubilisé dans 6 mL de méthanol puis sont ajoutés successivement l'alcyne désiré (1,00 mmol), du CuSO₄,5H₂O (25 mg, 0,100 mmol) et de l'ascorbate de sodium (40 mg, 0,200 mmol). Le milieu réactionnel est agité à température ambiante pendant 12 h. A l'issu du temps de réaction, le méthanol est évaporé sous pression réduite puis le résidu est chromatographié sur colonne de gel de silice en utilisant comme éluant un mélange CH₂Cl₂/MeOH/NH₄OH permettant une bonne séparation. Afin d'éliminer les traces d'imidazole souvent présent dans les produits triazoles chromatographiés, le mélange est solubilisé dans de l'acétate d'éthyle, lavé deux fois à l'eau, séché sur MgSO₄ anhydre et évaporé à sec.

***Endo*-3-(4-phényl-1*H*-1,2,3-triazol-1-yl)tropane (16).** Le produit est isolé sous la forme d'un solide blanc avec un rendement de 40% en suivant la procédure générale B. R*_{f}*: 0,30 (CH₂Cl₂/MeOH/NH₄OH : 90/10/0,1) ; Mp: 135°C ; IR (ATR, Diamond): *v*(cm⁻¹): 1015, 1078, 1116, 1142, 1217, 1335, 1411, 1451, 1481, 2441, 2934 ; RMN ¹H (250 MHz, CDCl₃) : *δ*(ppm) 1,72 - 1,80 (m, 2H), 1,99 - 2,10 (m, 2H), 2,46 (s, 3H), 2,66 (d, 2H, *J* = 15,0 Hz), 2,80 - 2,89 (m, 2H), 3,40 - 3,46 (m, 2H), 4,70 (dt, 1H, *J* = 6,8 Hz et 3,6 Hz), 7,31 - 7,36 (m, 1H), 7,40 - 7,46 (m, 2H), 7,84 (d, 2H, *J* = 8,1 Hz), 7,87 (s, 1H) ; RMN ¹³C (100 MHz, CDCl₃): *δ*(ppm) 25,2 (2CH₂), 35,0 (2CH₂), 40,1 (CH₃), 51,5 (CH), 60,4 (2CH), 119,1 (CHₐᵣₒ), 125,9 (2CHₐᵣₒ), 128,5 (CHₐᵣₒ), 129,1 (2CHₐᵣₒ), 130,7 (C_{q}), 148,2 (C_{q}) ; HRMS (EI-MS) : calculée pour C₁₆H₂₁N₄ m/z = 269,1766, trouvée m/z = 269,1757.

***Endo*-3-(4-(4-fluorophényl)-1*H*-1,2,3-triazol-1-yl)tropane (17).** Le produit est isolé sous la forme d'un solide blanc avec un rendement de 31% en suivant la procédure générale B. R*_{f}*: 0,28 (CH₂Cl₂/MeOH/NH₄OH : 90/10/0,1) ; Mp: 254°C ; IR (ATR, Diamond) : *v*(cm⁻¹) : 1014, 1081, 1141, 1161, 1224, 1402, 1429, 1452, 1496, 2446, 2470 ; RMN ¹H (250 MHz, CDCl₃) : *δ*(ppm) 1,89 - 2,19 (m, 4H), 2,63 (s, 3H), 2,75 (d, 2H, *J* = 15,8 Hz), 3,17 - 3,30 (m, 2H), 3,62 - 3,70 (m, 2H), 4,79 (t, 1H, *J* = 7,2 Hz), 7,12 (t, 2H, *J* = 8,6 Hz), 7,81 (dd, 2H, *J* = 8,6 Hz et 5,3 Hz), 7,88 (s, 1H) ; RMN ¹³C (100 MHz, CDCl₃) : *δ*(ppm) 24,5 (2CH₂), 34,1 (2CH₂), 39,5 (CH₃), 50,6 (CH), 61,3 (2CH), 116,2 (d, 2CHₐᵣₒ, *J* = 22 Hz), 119,1 (CHₐᵣₒ), 126,7 (d, C_{q}, *J* = 3 Hz), 127,6 (d, 2CHₐᵣₒ, *J* = 8 Hz), 147,8 (C_{q}), 163,0 (d, C_{q}, *J* = 248 Hz) ; HRMS (EI-MS) : calculée pour C₆H₂₆FN₄ m/z = 287,1672, trouvée m/z = 287,1669.

***Endo*-3-(4-(4-bromophényl)-1*H-*1,2,3-triazol-1-yl)tropane (18).** Le produit est isolé sous la forme d'un solide blanc avec un rendement de 42% en suivant la procédure générale B. R*_{f}*: 0,22 (CH₂Cl₂/MeOH/NH₄OH : 90/10/0,1) ; Mp : 226°C ; IR (ATR, Diamond) : *v*(cm⁻¹) : 973, 1009, 1068, 1116, 1148, 1214, 1232, 1334, 1396, 1422, 1450, 1478, 2932 ; RMN ¹H (250 MHz, CDCl₃) : *δ*(ppm) 1,48 - 1,57 (m, 2H), 1,90 - 2,00 (m, 2H), 2,28 (s, 3H), 2,51 - 2,57 (m, 4H), 3,19 - 3,27 (m, 2H), 4,58 - 4,66 (m, 1H), 7,54 (d, 2H, *J* = 8,6 Hz), 7,72 (d, 2H, *J* = 8,6 Hz), 7,85 (s, 1H) ; RMN ¹³C (100 MHz, CDCl₃) : *δ*(ppm) 25,9 (2CH₂), 35,9 (2CH₂), 40,6 (CH₃), 52,4 (CH), 59,5 (2CH), 119,1 (CHₐᵣₒ), 122,1 (C_{q}), 127,4 (2CHₐᵣₒ), 130,0 (C_{q}), 132,2 (2CHₐᵣₒ), 146,7 (C_{q}) ; HRMS (EI-MS) : calculée pour C₁₆H₂₀BrN₄ m/z = 347,0871, trouvée m/z = 347,0879.

***Endo*-3-(4-(4-méthoxyphényl)-1*H*-1,2,3-triazol-1-yl)tropane (19).** Le produit est isolé sous la forme d'un solide blanc avec un rendement de 21% en suivant la procédure générale B. R*_{f}*: 0,27 (CH₂Cl₂/MeOH/NH₄OH: 90/10/0,1); Mp: 118°C; IR (ATR, Diamond) : *v*(cm⁻¹) : 972, 1018, 1036, 1078, 1177, 1219, 1245, 1333, 1397, 1497, 1616, 2943 ; RMN ¹H (250 MHz, CDCl₃) : *δ*(ppm) 1,65 (d, 2H, *J* = 8,2 Hz), 1,93 - 2,03 (m, 2H), 2,36 (s, 3H), 2,55 - 2,75 (m, 4H), 3,29 - 3,36 (m, 2H), 3,84 (s, 3H), 4,64 (dt, 1H, *J* = 6,2 Hz et 3,3 Hz), 6,96 (d, 2H, *J* = 8,8 Hz), 7,76 (d, 2H, *J* = 8,8 Hz), 7,77 (s, 1H) ; RMN ¹³C (100 MHz, CDCl₃) : *δ*(ppm) 25,5 (2CH₂), 35,4 (2CH₂), 40,3 (CH₃), 51,8 (CH), 55,6 (CH₃), 59,9 (2CH), 114,5 (2CHₐᵣₒ), 118,3 (CH), 123,6 (C_{q}), 127,2 (2CHₐᵣₒ), 147,8 (C_{q}), 159,8 (C_{q}) ; HRMS (EI-MS) : calculée pour C₁₇H₂₃N₄O m/z = 299,1872, trouvée m/z = 299,1881.

**(4-(1-(*Endo*-tropan-3-yl)-1*H*-1,2,3-triazol-4-yl)phényl)méthanol (20).** Le produit est isolé sous la forme d'un solide blanc avec un rendement de 45% en suivant la procédure générale B. R*_{f}*: 0,19 (CH₂Cl₂/MeOH/NH₄OH : 90/10/0,1) ; Mp: >250°C ; IR (ATR, Diamond) : *v*(cm⁻¹) : 1013, 1060, 1082, 1401, 1431, 1452, 1493, 1650, 2506, 2556, 2587, 3352 ; RMN ¹H (250 MHz, DMSO-*d₆*) : *δ*(ppm) 1,47 (d, 2H, *J* = 8,2 Hz), 1,88 - 2,07 (m, 2H), 2,44 (s, 3H), 2,60 - 2,82 (m, 4H), 3,50 - 3,58 (m, 2H), 4,56 (s, 2H), 4,64 - 4,75 (m, 1H), 5,28 (s large, 1H, OH), 7,43 (d, 2H, *J* = 8,1 Hz), 7,87 (d, 2H, *J* = 8,1 Hz), 8,88 (s, 1H) ; RMN ¹³C (100 MHz, DMSO-*d₆*) : *δ*(ppm) 24,4 (2CH₂), 32,9 (2CH₂), 39,8 (CH₃), 50,7 (CH), 59,4 (2CH), 62,6 (CH₂), 120,8 (CHₐᵣₒ), 124,8 (2CHₐᵣₒ), 126,9 (2CHₐᵣₒ), 129,2 (C_{q}), 142,2 (C_{q}), 146,4 (C_{q}) ; HRMS (EI-MS) : calculée pour C₁₇H₂₃N₄O m/z = 299,1872, trouvée m/z = 299,1859.

***Endo*-3-(4-(6-méthoxynaphthalèn-2-yl)-1*H*-1,2,3-triazol-1-yl)tropane (21).** Le produit est isolé sous la forme d'un solide blanc avec un rendement de 33% en suivant la procédure générale B. R*_{f}*: 0,24 (CH₂Cl₂/MeOH/NH₄OH : 90/10/0,1) ; Mp: >250°C ; IR (ATR, Diamond) : *v*(cm⁻¹) : 908, 1021, 1123, 1163, 1212, 1262, 1345, 1393, 1453, 1612, 2932 ; RMN ¹H (250 MHz, CDCl₃) : *δ*(ppm) 1,76 (d, 2H, *J* = 8,0 Hz), 1,97 - 2,07 (m, 2H), 2,43 (s, 3H), 2,61 - 2,72 (m, 2H), 2,76 - 2,90 (m, 2H), 3,35 - 3,45 (m, 2H), 3,93 (s, 3H), 4,65 - 4,75 (m, 1H), 7,13 - 7,20 (m, 2H), 7,75 - 7,82 (m, 2H), 7,87 - 7,94 (m, 1H), 7,94 (s, 1H), 8,26 (s, 1H) ; RMN ¹³C (100 MHz, CDCl₃) : *δ*(ppm) 25,4 (2CH₂), 35,3 (2CH₂), 40,3 (CH₃), 51,8 (CH), 55,6 (CH₃), 60,2 (2CH), 106,0 (CHₐᵣₒ), 119,0 (CHₐᵣₒ), 119,5 (CHₐᵣₒ), 124,5 (2CHₐᵣₒ), 126,0 (C_{q}), 217,6 (CHₐᵣₒ), 129,2 (C_{q}), 129,9 (CHₐᵣₒ), 134,6 (C_{q}), 148,2 (C_{q}), 158,2 (C_{q}) ; HRMS (EI-MS) : calculée pour C₂₁H₂₅N₄O m/z = 349,2028, trouvée m/z = 349,2029.

***Endo-*3-(4-(3-fluorophényl)-1*H*-1,2,3-triazol-1-yl)tropane (22).** Le produit est isolé sous la forme d'un solide blanc avec un rendement de 34% en suivant la procédure générale B. R*_{f}*: 0,23 (CH₂Cl₂/MeOH/NH₄OH : 90/10/0,1) ; Mp : 124°C ; IR (ATR, Diamond) : *v*(cm⁻¹) : 1017, 1087, 1156, 1198, 1227, 1336, 1416, 1450, 1477, 1584, 1620, 2937, 2963, 3094 ; RMN ¹H (250 MHz, CDCl₃) : *δ*(ppm) 1,47 - 1,57 (m, 2H), 1,92 - 2,00 (m, 2H), 2,28 (s, 3H), 2,51 - 2,57 (m, 4H), 3,18 - 3,26 (m, 2H), 4,62 (quint, 1H, *J* = 5,2 Hz), 6,94 - 7,09 (m, 1H), 7,32 - 7,42 (m, 1H), 7,52 - 7,65 (m, 1H), 7,58 (s, 1H), 7,86 (s, 1H) ; RMN ¹³C (100 MHz, CDCl₃) : *δ*(ppm) 25,9 (2CH₂), 35,9 (2CH₂), 40,6 (CH₃), 52,5 (CH), 59,5 (2CH), 112,8 (d, CHₐᵣₒ, *J* = 22 Hz), 115,1 (d, CHₐᵣₒ, *J =* 22 Hz), 119,4 (CHₐᵣₒ), 121,4 (d, CHₐᵣₒ, *J* = 3 Hz), 130,6 (d, CHₐᵣₒ, *J* = 8 Hz), 133,2 (d, C_{q}, *J* = 8 Hz), 146,6 (d, C_{q}, *J* = 3 Hz), 163,4 (d, C_{q}, *J* = 246 Hz) ; HRMS (EI-MS) : calculée pour C₁₆H₂₀FN₄ m/z = 287,1672, trouvée m/z = 287,1671.

***Endo*-3-(4-(pyridin-4-yl)-1*H*-1,2,3-triazol-1-yl)tropane (23).** Le produit est isolé sous la forme d'un solide brun avec un rendement de 43% en suivant la procédure générale B. R*_{f}*: 0,12 (CH₂Cl₂/MeOH/NH₄OH : 90/10/0,1) ; Mp : 146°C ; IR (ATR, Diamond) : *v*(cm⁻¹) : 817, 1016, 1060, 1082, 1217, 1340, 1426, 1610, 2936 ; RMN ¹H (400 MHz, CDCl₃) : *δ*(ppm) 1,48 - 1,57 (m, 2H), 1,89 - 2,03 (m, 2H), 2,30 (s, 3H), 2,48 - 2,66 (m, 4H), 3,21 - 3,30 (m, 2H), 4,63 (dt, 1H, *J* = 6,8 Hz et 3,3 Hz), 7,72 (d, 2H, *J* = 6,0 Hz), 7,99 (s, 1H), 8,64 (d, 2H, *J* = 6,0 Hz) ; RMN ¹³C (100 MHz, CDCl₃) : *δ*(ppm) 25,9 (2CH₂), 35,9 (2CH₂), 40,6 (CH₃), 52,6 (CH), 59,5 (2CH), 120,1 (2CHₐᵣₒ), 120,6 (CHₐᵣₒ), 138,3 (C_{q}), 145,2 (C_{q}), 150,6 (2CHₐᵣₒ) ; HRMS (EI-MS) : calculée pour C₁₅H₂₀N₅ m/z = 270,1719, trouvée m/z = 270,1711.

***Endo*-3-(4-(3,4-dichlorophényl)-1*H*-1,2,3-triazol-1-yl)tropane (24).** Le produit est isolé sous la forme d'un solide blanc avec un rendement de 22% en suivant la procédure générale B. R*_{f}*: 0,32 (CH₂Cl₂/MeOH/NH₄OH : 90/10/0,1) ; Mp: 136°C ; IR (ATR, Diamond) : *v*(cm⁻¹) : 825, 1021, 1082, 1117, 1131, 1231, 1336, 1423, 1459, 1473, 2934 ; RMN ¹H (400 MHz, CDCl₃) : *δ*(ppm) 1,49 - 1,54 (m, 2H), 1,89 - 2,03 (m, 2H), 2,29 (s, 3H), 2,48 - 2,62 (m, 4H), 3,21 - 3,27 (m, 2H), 4,63 (dt, 1H, *J* = 6,6 Hz et 3,3 Hz), 7,48 (d, 1H, *J* = 8,3 Hz), 7,68 (dd, 1H, *J* = 8,3 Hz et 1,9 Hz), 7,86 (s, 1H), 7,92 (d, 1H, *J* = 1,9 Hz) ; RMN ¹³C (100 MHz, CDCl₃) : *δ*(ppm) 25,9 (2CH₂), 35,9 (2CH₂), 40,6 (CH₃), 52,6 (CH), 59,5 (2CH), 119,2 (CHₐᵣₒ), 125,0 (CHₐᵣₒ), 127,6 (CHₐᵣₒ), 131,0 (CHₐᵣₒ et C_{q}), 132,0 (C_{q}), 133,2 (C_{q}), 145,6 (C_{q}) ; HRMS (EI-MS) : calculée pour C₁₆H₁₉N₄Cl₂ m/z = 337,0987, trouvée m/z = 337,0980.

***Endo*-3-(4-(1-benzothiophèn-5-yl)-1*H*-1,2,3-triazol-1-yl)tropane (25).** Le produit est isolé sous la forme d'un solide blanc avec un rendement de 34% en suivant la procédure générale B. R*_{f}*: 0,25 (CH₂Cl₂/MeOH/NH₄OH : 90/10/0,1) ; Mp: 163°C ; IR (ATR, Diamond) : *v*(cm⁻¹) : 818, 896, 1018, 1079, 1114, 1218, 1338, 1394, 1439, 1552, 2100, 2929, 3036 ; RMN ¹H (400 MHz, CDCl₃) : *δ*(ppm) 1,54 - 1,62 (m, 2H), 1,93 - 2,02 (m, 2H), 2,30 (s, 3H), 2,54 - 2,61 (m, 4H), 3,20 - 3,28 (m, 2H), 4,64 (quint, 1H, *J* = 5,0 Hz), 7,37 (d, 1H, *J* = 5,4 Hz), 7,47 (d, 1H, *J* = 5,4 Hz), 7,81 (dd, 1H, *J* = 8,4 Hz et 1,2 Hz), 7,91 (s, 1H), 7,92 (d, 1H, *J* = 8,4 Hz), 8,33 (d, 1H, *J* = 1,2 Hz) ; RMN ¹³C (100 MHz, CDCl₃) : *δ*(ppm) 25,8 (2CH₂), 35,8 (2CH₂), 40,5 (CH₃), 52,3 (CH), 59,6 (2CH), 119,0 (CHₐᵣₒ), 120,7 (CHₐᵣₒ), 122,3 (CHₐᵣₒ), 123,0 (CHₐᵣₒ), 124,2 (CHₐᵣₒ), 127,3 (C_{q}), 127,4 (CHₐᵣₒ), 139,5 (C_{q}), 140,2 (C_{q}), 147,9 (C_{q}) ; HRMS (EI-MS) : calculée pour C₁₈H₂₁N₄S m/z = 325,1487, trouvée m/z = 325,1488.

***Endo*-3-(4-(1-benzofuran-5-yl)-1*H*-1,2,3-triazol-1-yl)tropane (26).** Le produit est isolé sous la forme d'un solide blanc avec un rendement de 35% en suivant la procédure générale B. R*_{f}*: 0,23 (CH₂Cl₂/MeOH/NH₄OH : 90/10/0,1) ; Mp: 104°C ; IR (ATR, Diamond) : *v*(cm⁻¹) : 988, 1030, 1067, 1108, 1224, 1338, 1443, 1457, 1496, 1518, 2361, 2873, 2929, 3115, 3351 ; RMN ¹H (400 MHz, CDCl₃) : *δ*(ppm) 1,55 - 1,60 (m, 2H), 1,94-1,99 (m, 2H), 2,30 (s, 3H), 2,55 - 2,60 (m, 4H), 3,22 - 3,27 (m, 2H), 4,64 (quint, 1H, *J* = 5,0 Hz), 6,77 - 6,83 (m, 1H), 7,54 (d, 1H, *J* = 8,6 Hz), 7,64 (d, 1H, *J* = 2,1 Hz), 7,76 (dd, 1H, *J* = 8,6 Hz et 1,5 Hz), 7,85 (s, 1H), 8,10 (d, 1H, *J* = 1,5 Hz) ; RMN ¹³C (100 MHz, CDCl₃) : *δ*(ppm) 25,8 (2CH₂), 35,8 (2CH₂), 40,6 (CH₃), 52,2 (CH), 59,6 (2CH), 107,0 (CHₐᵣₒ), 111,9 (CHₐᵣₒ), 118,6 (CHₐᵣₒ), 118,7 (CHₐᵣₒ), 122,6 (CHₐᵣₒ), 126,0 (C_{q}), 128,1 (C_{q}), 145,8 (CHₐᵣₒ), 148,1 (C_{q}), 155,0 (C_{q}) ; HRMS (EI-MS) : calculée pour C₁₈H₂₁N₄O m/z = 309,1715, trouvée m/z = 309,1709.

***Endo*-3-(4-(5-bromothiophèn-2-yl)-1*H*-1-1,2,3-triazol-1-yl)tropane (27).** Le produit est isolé sous la forme d'un solide blanc avec un rendement de 29% en suivant la procédure générale B. R*_{f}*: 0,32 (CH₂Cl₂/MeOH/NH₄OH : 90/10/0,1) ; Mp: 138°C ; IR (ATR, Diamond) : *v* (cm⁻¹) : 969, 1016, 1066, 1104, 1132, 1228, 1331, 1407, 1435, 2913, 2940, 2966, 3274 ; RMN ¹H (400 MHz, CDCl₃) : *δ* (ppm) 1,56 - 1,66 (m, 2H), 1,96 - 2,01 (m, 2H), 2,35 (s, 3H), 2,50 - 2,57 (m, 2H), 2,63 - 2,73 (m, 2H), 3,28 - 3,33 (m, 2H), 4,58 - 4,67 (m, 1H), 7,01 (d, 1H, *J* = 3,8 Hz), 7,10 (d, 1H, *J* = 3,8 Hz), 7,73 (s, 1H) ; RMN ¹³C (100 MHz, CDCl₃) : *δ* (ppm) 25,8 (2CH₂), 35,4 (2CH₂), 40,3 (CH₃), 52,2 (CH), 59,8 (2CH), 112,2 (C_{q}), 118,5 (CHₐᵣₒ), 124,3 (CHₐᵣₒ), 130,6 (CHₐᵣₒ), 134,8 (C_{q}), 142,3 (C_{q}) ; HRMS (EI-MS) : calculée pour C₁₄H₁₈N₄SBr m/z = 353,0436, trouvée m/z = 353,0447.

### Préparation d'un précurseur des composés de type octahydroquinolizine (formule (II-3))

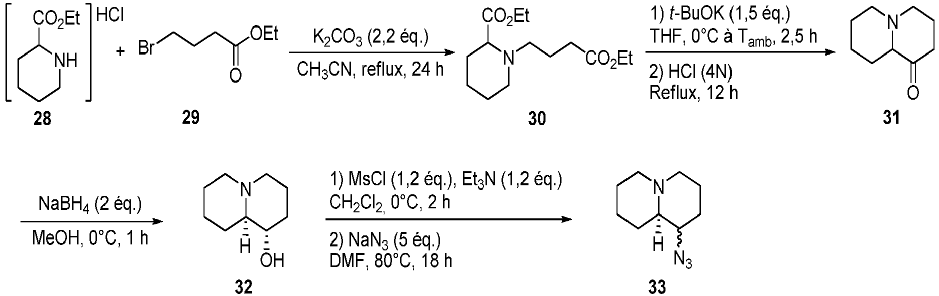

**2-(4-Ethoxy-4-oxobutyl)pipéridine-1-carboxylate d'éthyle (30).** A une solution du chlorhydrate du pipecolinate d'éthyle **28** (12,4 g, 64,0 mmol) dans 300 mL d'acétonitrile sont ajoutés le 4-bromobutanoate d'éthyle **29** (11,0 mL, 76,8 mmol) et du K₂CO₃ (19,5 g, 140,8 mmol). Le milieu réactionnel est ensuite chauffé à reflux pendant 24 heures. A l'issu du temps de réaction, le solvant est évaporé sous pression réduite puis le résidu est repris dans de l'acétate d'éthyle. La phase organique est lavée avec de l'eau, séchée sous MgSO₄ anhydre et concentrée sous vide. Le brut est purifié par chromatographie sur colonne de gel de silice avec comme éluant un mélange éther de pétrole/acétate d'éthyle (4/1). Le diester **30** est isolé sous la forme d'un liquide incolore avec un rendement de 80%. R*_{f}* : 0,29 (EP/AcOEt : 3/1) ; IR (ATR, Diamond) : *v* (cm⁻¹) : 1027, 1123, 1158, 1372, 1446, 1730, 2936 ; RMN ¹H (400 MHz, CDCl₃) : *δ* (ppm) 1,22 (t, 3H, *J* = 7,1 Hz), 1,24 (t, 3H, *J* = 7,1 Hz), 1,31 - 1,40 (m, 1H), 1,55 - 1,61 (m, 3H), 1,73 - 1,80 (m, 4H), 2,14 - 2,21 (m, 1H), 2,24 - 2,40 (m, 3H), 2,50 - 2,57 (m, 1H), 3,00 - 3,09 (m, 2H), 4,11 (q, 2H, *J* = 7,1 Hz), 4,17 (q, 2H, *J* = 7,1 Hz) ; RMN ¹³C (100 MHz, CDCl₃) : *δ* (ppm) 14,5 (2CH₃), 22,3 (CH₂), 22,8 (CH₂), 25,5 (CH₂), 29,8 (CH₂), 32,4 (CH₂), 50,5 (CH₂), 55,8 (CH₂), 60,4 (CH₂), 60,5 (CH₂), 65,3 (CH), 173,8 (C=O), 174,0 (C=O) ; MS (IS) : m/z = 272,3 [MH]⁺.

**Hexahydro-2*H*-quinolizin-1(6*H*)-one (31).** Une solution de 2-(4-éthoxy-4-oxobutyl)pipéridine-1-carboxylate d'éthyle **30** (5,00 g, 18,4 mmol) dans 100 mL de THF anhydre est refroidie à 0°C puis du *t*-BuOK (3,10 g, 27,6 mmol) est ajouté par portion. Le milieu réactionnel est agité à 0°C pendant 30 minutes puis 2 heures à température ambiante. Ensuite, le THF est évaporé sous pression réduite et le résidu est dilué dans de l'acétate d'éthyle. La phase organique est lavée à l'eau, séchée sur MgSO₄ anhydre et concentré sous vide. Le résidu est repris dans 60 mL d'une solution diluée d'acide chlorhydrique (4N) puis chauffé à reflux pendant 12 heures. A l'issu de la réaction, le milieu réactionnel est neutralisé par l'ajout de NaHCO₃ solide puis la phase aqueuse est extraire par du CH₂Cl₂. La phase organique est alors séchée sur MgSO₄ anhydre et concentrée sous pression réduite. La cétone **31** est isolée sous la forme d'un liquide rouge avec un rendement de 74%. R*_{f}* : 0,20 (AcOEt) ; IR (ATR, Diamond) : *v* (cm⁻¹) : 1076, 1145, 1174, 1281, 1319, 1344, 1443, 1718, 2933 ; RMN ¹H (250 MHz, CDCl₃) : *δ* (ppm) 1,10 - 1,65 (m, 4H), 1,75 - 1,87 (m, 1H), 1,90 - 2,05 (m, 3H), 2,08 -2,32 (m, 2H), 2,33 - 2,55 (m, 3H), 2,87 - 3,00 (m, 2H) ; RMN ¹³C (100 MHz, CDCl₃) : *δ* (ppm) 24,0 (CH₂), 24,4 (CH₂), 25,6 (CH₂), 25,8 (CH₂), 39,4 (CH₂), 55,0 (CH₂), 57,1 (CH₂), 71,2 (CH), 207,5 (C=O) ; MS (IS) : m/z = 154,1 [MH]⁺.

**Octahydro-2*H*-quinolizin-1-ol (32).** A une solution de l'hexahydro-2*H*-quinolizin-1(6*H*)-one **31** (1,70 g, 11,1 mmol) dans 100 mL de méthanol à 0°C est ajouté par portion du NaBH₄ (840 mg, 22,2 mmol) et le milieu réactionnel est agité à cette température pendant 1 heure. Après évaporation du solvant, le résidu est repris dans du dichlorométhane puis la phase organique est lavée à l'eau, séchée sur MgSO₄ anhydre et concentrée sous pression réduite. L'alcool **32** est purifié par chromatographie sur colonne de gel de silice en utilisant comme éluant un mélange CH₂Cl₂/MeOH/NH₄OH (90/10/0,1). Il est isolé sous la forme d'un solide blanc avec un rendement de 78%. R*_{f}* : 0,32 (CH₂Cl₂/MeOH : 4/1) ; Mp : 70°C ; IR (ATR, Diamond) : *v* (cm⁻¹) : 968, 1020, 1046, 1085, 1107, 1179, 1276, 1346, 1442, 1469, 2761, 2804, 2856, 2925, 2943, 3130 ; RMN ¹H (250 MHz, CDCl₃) : *δ* (ppm) 1,06 - 1,34 (m, 3H), 1,51 - 1,72 (m, 5H), 1,75 - 1,85 (m, 2H), 1,93 - 2,08 (m, 3H), 2,09 - 2,18 (m, 1H), 2,67 - 2,76 (m, 1H), 2,79 - 2,88 (m, 1H), 3,28 (ddd, 1H, *J* = 11,1 Hz, 8,8 Hz et 4,6 Hz) ; RMN ¹³C (100 MHz, CDCl₃) : *δ* (ppm) 23,5 (CH₂), 24,3 (CH₂), 25,9 (CH₂), 28,9 (CH₂), 34,2 (CH₂), 56,1 (CH₂), 53,3 (CH₂), 69,0 (CH), 72,8 (CH) ; MS (IS) : m/z = 156,1 [MH]⁺.

**1-Azidooctahydro-2*H*-quinolizine (33).** A une solution de l'octahydro-2*H*-quinolizin-1-ol **32** (700 mg, 4,51 mmol) dans 40 mL de CH₂Cl₂ à 0°C sont ajoutés de la triéthylamine (760 µL, 5,41 mmol) et du chlorure de mésyle (420 µL, 5,41 mmol) pendant une période de 30 minutes. Le milieu réactionnel est agité à 0°C pendant 2 heures. La réaction est hydrolysée par l'ajout d'une solution saturée en NaHCO₃. La phase organique est séparée, séchée sur MgSO₄ anhydre et évaporée sous pression réduite. Le dérivé mésylé préparé (1,05 g, 4,51 mmol) est chauffé à 80°C dans 30 mL de DMF pendant 18 heures en présence de l'azoture de sodium (1,46 g, 22,6 mmol). A l'issu de la réaction, le solvant est évaporé puis le résidu est repris dans du CH₂Cl₂. La phase organique est lavée deux fois à l'eau, séchée sur MgSO₄ anhydre et concentrée sous pression réduite. L'azoture **33** est purifié par chromatographie sur colonne de gel de silice en utilisant l'acétate d'éthyle comme éluant. Il est isolé sous la forme d'une huile jaune avec un rendement de 71% sous la forme de deux diastéréoisomères non séparables dans des proportions (3/2). R*_{f}* : 0,15 (AcOEt) ; IR (ATR, Diamond) : *v* (cm⁻¹) : 980, 1024, 1113, 1133, 1155, 1254, 1444, 2090, 2799, 2930 ; RMN ¹H (250 MHz, CDCl₃) : *δ* (ppm) **Diastéréoisomère majoritaire** *d* 1,11 - 1,30 (m, 2H), 1,56 - 1,82 (m, 6H), 1,95 - 2,17 (m, 3H), 2,30 - 2,46 (m, 2H), 2,70 - 2,79 (m, 1H), 2,94 - 3,07(m, 2H). **Diastéréoisomère minoritaire** *d* 1,30 - 1,50 (m, 2H), 1,56 - 1,82 (m, 6H), 1,95 - 2,17 (m, 3H), 2,30 - 2,46 (m, 2H), 2,79 - 2,88 (m, 1H), 2,98 - 3,07(m, 1H), 3,17 - 3,27 (m, 1H) ; RMN ¹³C (100 MHz, CDCl₃) : *δ* (ppm) **Diastéréoisomère majoritaire** *d* 21,8 (CH₂), 23,5 (CH₂), 29,7 (CH₂), 31,8 (CH₂), 33,0 (CH₂), 55,8 (CH₂), 58,1 (CH₂), 68,1 (CH), 68,4 (CH). **Diastéréoisomère minoritaire** *d* 23,8 (CH₂), 24,3 (CH₂), 25,8 (CH₂), 29,8 (CH₂), 30,3 (CH₂), 55,8 (CH₂), 56,4 (CH₂), 63,8 (CH), 66,5 (CH) ; MS (IS) : m/z = 181,4 [MH]⁺.

### Préparation des composés 34-35 de type octahydroquinolizine (formule (II-3))

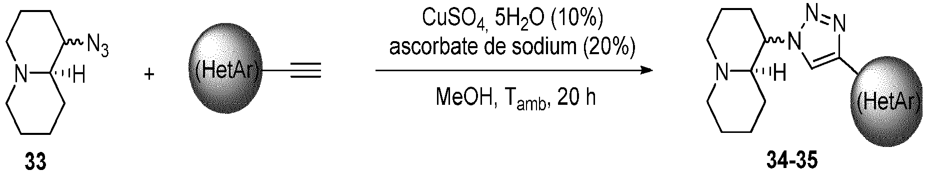

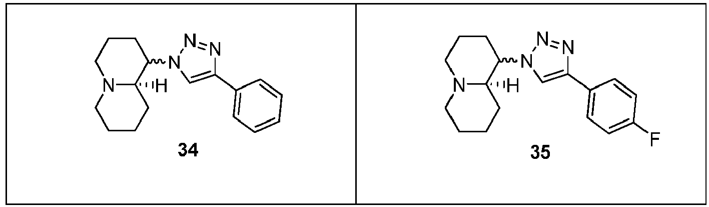

**Procédure générale C :** Sous argon, l'azoture **33** (180 mg, 1,00 mmol) est solubilisé dans 6 mL de méthanol puis sont ajoutés successivement l'alcyne (1,00 mmol), du CuSO₄,5H₂O (25 mg, 0,100 mmol) et de l'ascorbate de sodium (40 mg, 0,200 mmol). Le milieu réactionnel est agité à température ambiante pendant 12 heures. A l'issu du temps de réaction, le méthanol est évaporé sous pression réduite puis le résidu est repris dans le dichlorométhane. La phase organique est lavée avec une solution saturée en NaHCO₃, séchée sur MgSO₄ anhydre et concentrée sous pression réduite. Les produits **34** et **35** sont chromatographiés sur colonne de gel de silice en utilisant comme éluant un mélange CH₂Cl₂/MeOH/NH₄OH (99/1/0,1).

**1-(4-Phényl-1*H*-1,2,3-triazol-1-yl)octahydro-2*H*-quinolizine (34).** En suivant la procédure générale C, le produit est isolé sous la forme d'un solide blanc avec un rendement de 94% sous la forme de deux diastéréoisomères séparables dans des proportions 3/2.
**Diastéréoisomère minoritaire** : R*_{f}* : 0,46 (CH₂Cl₂/MeOH/NH₄OH : 99/1/0,1) ; Mp : 150°C ; IR (ATR, Diamond) : *v* (cm⁻¹) : 977, 1023, 1113, 1219, 1294, 1349, 1372, 1434, 1460, 1482, 2754, 2804, 2852, 2925, 3081 ; RMN ¹H (250 MHz, CDCl₃) : *δ* (ppm) 1,04 - 1,28 (m, 3H), 1,50 - 1,74 (m, 3H), 1,81 - 1,91 (m, 2H), 1,92 - 2,15 (m, 2H), 2,16 - 2,30 (m, 3H), 2,81 - 3,04 (m, 2H), 4,23 - 4,35 (m, 1H), 7,28 - 7,36 (m, 1H), 7,38 - 7,46 (m, 2H), 7,72 (s, 1H), 7,80 - 7,88 (m, 2H) ; RMN ¹³C (100 MHz, CDCl₃) : *δ* (ppm) 24,0 (CH₂), 24,3 (CH₂), 25,7 (CH₂), 29,0 (CH₂), 32,4 (CH₂), 56,0 (CH₂), 56,5 (CH₂), 64,0 (CH), 66,5 (CH), 118,8 (CHₐᵣₒ), 125,9 (2CHₐᵣₒ), 128,3 (CHₐᵣₒ), 129,0 (2CHₐᵣₒ), 130,9 (C_{q}), 147,6 (C_{q}) ; HRMS (EI-MS) : calculée pour C₁₇H₂₃N₄ m/z = 283,1923, trouvée m/z = 283,1928.
**Diastéréoisomère majoritaire** : R*_{f}* : 0,52 (CH₂Cl₂/MeOH/NH₄OH : 99/1/0,1) ; Mp : 140°C ; IR (ATR, Diamond) : *v* (cm⁻¹) : 974, 1049, 1077, 1134, 1196, 1222, 1267, 1433, 1460, 1482, 2780, 2862, 2928 ; RMN ¹H (250 MHz, CDCl₃) : *δ* (ppm) 1,39 - 1,56 (m, 1H), 1,60 - 1,93 (m, 7H), 1,95 - 2,08 (m, 1H), 2,20 - 2,35 (m, 1H), 2,41 - 2,66 (m, 2H), 3,02 - 3,22 (m, 3H), 4,36 - 4,53 (m, 1H), 7,28 - 7,36 (m, 1H), 7,38 - 7,46 (m, 2H), 7,74 (s, 1H), 7,80 - 7,89 (m, 2H) ; RMN ¹³C (100 MHz, CDCl₃) : *δ* (ppm) 22,7 (CH₂), 23,4 (CH₂), 29,5 (CH₂), 30,7 (CH₂), 35,7 (CH₂), 55,3 (CH₂), 58,2 (CH₂), 67,4 (CH), 67,9 (CH), 118,3 (CHₐᵣₒ), 125,8 (2CHₐᵣₒ), 128,2 (CHₐᵣₒ), 129,0 (2CHₐᵣₒ), 131,0 (C_{q}), 147,6 (C_{q}) ; HRMS (EI-MS): calculée pour C₁₇H₂₃N₄ m/z = 283,1923, trouvée m/z = 283,1928.

**1-[4-(4-Fluorophenyl)-1*H*-1,2,3-triazol-1-yl]octahydro-2*H*-quinolizine (35).** En suivant la procédure générale C, le produit est isolé sous la forme d'un solide blanc avec un rendement de 94% sous la forme de deux diastéréoisomères séparables dans des proportions 3/2.
**Diastéréoisomère minoritaire :** R*_{f}* : 0,39 (CH₂Cl₂/MeOH/NH₄OH : 99/1/0,1) ; Mp : 142°C ; IR (ATR, Diamond): *v* (cm⁻¹) : 1048, 1113, 1158, 1222, 1297, 1347, 1449, 1494, 1557, 1611, 2754, 2798, 2926, 3104 ; RMN ¹H (250 MHz, CDCl₃) : *δ* (ppm) 1,02 - 1,30 (m, 3H), 1,49 - 1,74 (m, 3H), 1,81 - 2,05 (m, 3H), 2,07 - 2,30 (m, 4H), 2,81 - 3,02 (m, 2H), 4,20 - 4,38 (m, 1H), 7,11 (t, 2H, *J* = 8,8 Hz), 7,68 (s, 1H), 7,80 (dd, 2H, *J* = 8,8 Hz et 5,3 Hz) ; RMN ¹³C (100 MHz, CDCl₃) : *δ* (ppm) 24,0 (CH₂), 24,3 (CH₂), 25,7 (CH₂), 29,0 (CH₂), 32,4 (CH₂), 55,9 (CH₂), 56,5 (CH₂), 64,0 (CH), 66,5 (CH), 116,0 (d, 2CHₐᵣₒ, *J* = 22 Hz), 118,6 (CHₐᵣₒ), 127,1 (d, C_{q}, *J* = 3 Hz), 127,6 (d, 2CHₐᵣₒ, *J* = 8 Hz), 146,7 (C_{q}), 162,8 (d, C_{q}, *J* = 247 Hz) ; HRMS (EI-MS) : calculée pour C₁₇H₂₂FN₄ m/z = 301,1829, trouvée m/z = 301,1826.
**Diastéréoisomère majoritaire** : R*_{f}* : 0,50 (CH₂Cl₂/MeOH/NH₄OH : 99/1/0,1) ; Mp : 136°C ; IR (ATR, Diamond): *v* (cm⁻¹) : 1050, 1159, 1223, 1346, 1448, 1493, 1557, 1612, 2794, 2927, 3104 ; RMN ¹H (250 MHz, CDCl₃) : *δ* (ppm) 1,39 - 1,57 (m, 1H), 1,60 - 1,95 (m, 7H), 1,96 - 2,09 (m, 1H), 2,18 - 2,38 (m, 1H), 2,42 - 2,66 (m, 2H), 3,02 - 3,23 (m, 3H), 4,36 - 4,53 (m, 1H), 7,11 (t, 2H, *J* = 8,7 Hz), 7,70 (s, 1H), 7,80 (d, 2H, *J* = 8,6 Hz et 5,4 Hz) ; RMN ¹³C (100 MHz, CDCl₃) : *δ* (ppm) 22,7 (CH₂), 23,4 (CH₂), 29,5 (CH₂), 30,7 (CH₂), 35,7 (CH₂), 55,3 (CH₂), 58,2 (CH₂), 67,4 (CH), 67,9 (CH), 116,0 (d, 2CHₐᵣₒ, *J* = 22 Hz), 118,1 (CHₐᵣₒ), 127,2 (d, C_{q}, *J* = 3 Hz), 127,6 (d, 2CHₐᵣₒ, *J* = 8 Hz), 146,8 (C_{q}), 162,8 (d, C_{q}, *J* = 247 Hz) ; HRMS (EI-MS) : calculée pour C₁₇H₂₂FN₄ m/z = 301,1829, trouvée m/z = 301,1831.

### Préparation des composés 37-57 et 80-83 de type quinuclidine (formule (II-2))

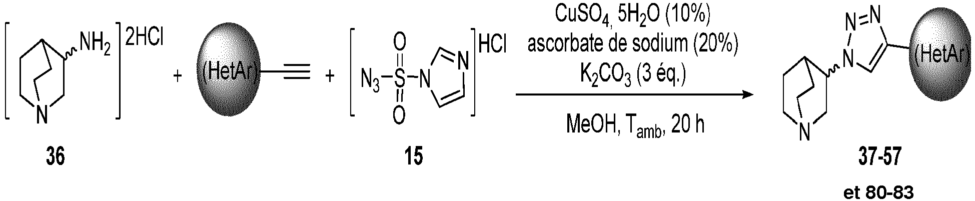

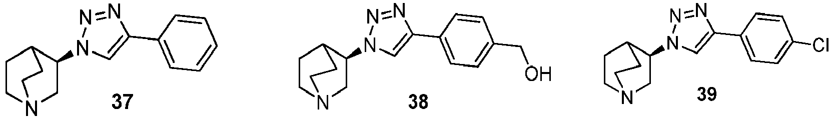

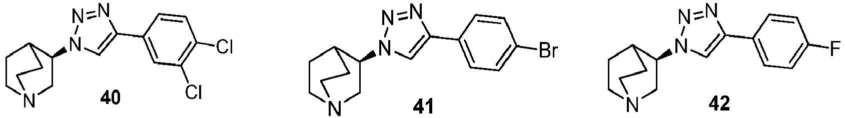

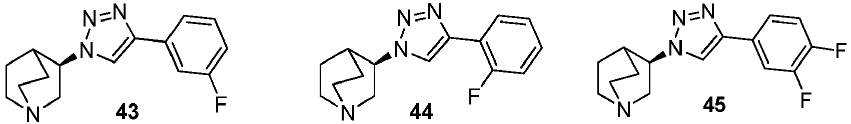

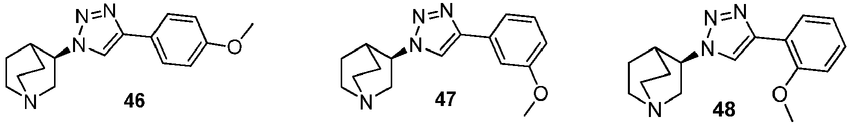

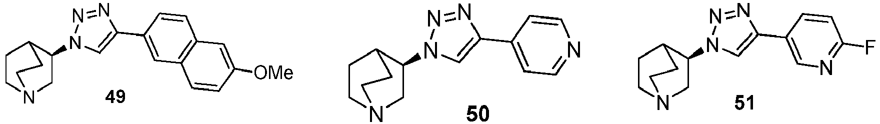

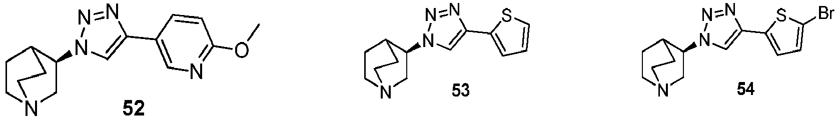

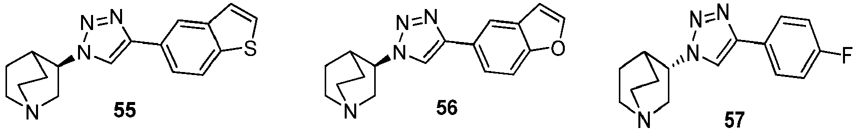

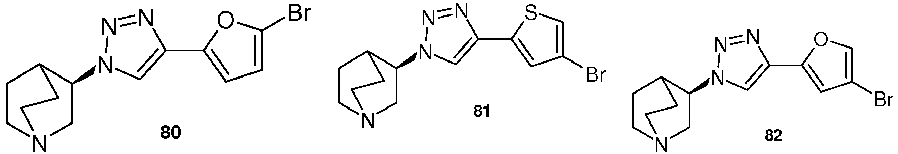

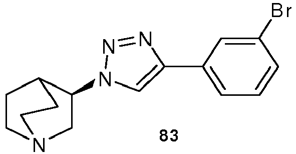

**Procédure générale D :** Sous argon, la 3-aminoquinuclidine **36** (212 mg, 1,00 mmol) et l'azoture du 1*H*-imidazole-1-sulfonyle **15** (232 mg, 1,10 mmol) sont solubilisés dans 6 mL de méthanol puis sont ajoutés successivement du K₂CO₃ (415 mg, 3,00 mmol) et une quantité catalytique de CuSO₄,5H₂O (25 mg, 0,100 mmol). Le milieu réactionnel est agité à température ambiante pendant 6 h puis concentré sous pression réduite. Le solide obtenu est alors repris dans 10 mL d'éther éthylique, filtré sous vide puis lavé deux fois avec 10 mL d'éther éthylique. Enfin, le filtrat est évaporé sous pression réduite et l'azoture ainsi obtenu est engagé dans l'étape suivant sans aucune autre purification.

Ce dernier est solubilisé dans 6 mL de méthanol puis sont ajoutés successivement l'alcyne désiré (1,00 mmol), du CuSO₄,5H₂O (25 mg, 0,100 mmol) et de l'ascorbate de sodium (40 mg, 0,200 mmol). Le milieu réactionnel est agité à température ambiante pendant 12 h. A l'issu du temps de réaction, le méthanol est évaporé sous pression réduite puis le résidu est chromatographié sur colonne de gel de silice en utilisant comme éluant un mélange CH₂Cl₂/MeOH/NH₄OH permettant une bonne séparation. Afin d'éliminer les traces d'imidazole souvent présent dans les produits triazoles chromatographiés, le mélange est solubilisé dans de l'acétate d'éthyle, lavé deux fois à l'eau, séché sur MgSO₄ anhydre et évaporé à sec.

**(*R*)-3-(4-Phényl-1*H*-1,2,3-triazol-1-yl)quinuclidine (37).** Le produit est isolé sous la forme d'un solide blanc avec un rendement de 33% en suivant la procédure générale D. R*_{f}* : 0,29 (CH₂Cl₂/MeOH/NH₄OH : 80/20/0,1) ; Mp : 132°C ; IR (ATR, Diamond) : *v* (cm⁻¹) : 973, 1023, 1043, 10,60, 1072, 1211, 1227, 1411, 1453, 1482, 2870, 2937 ; RMN ¹H (250 MHz, CDCl₃) : *δ* (ppm) 1,45 - 1,61 (m, 1H), 1,68 - 1,79 (m, 1H), 1,82 - 1,92 (m, 1H), 2,32 (q, 1H, *J* = 3,1 Hz), 2,90 - 3,10 (m, 3H), 3,15 - 3,35 (m, 1H), 3,56 (dd, 1H, *J* = 9,9 Hz et 2,2 Hz), 3,62 (dd, 1H, *J* = 9,9 Hz et 2,2 Hz), 3,81 (dd, 1H, *J* = 14,4 Hz et 5,2 Hz), 4,69 - 4,79 (m, 1H), 7,29 - 7,37 (m, 1H), 7,39 - 7,47 (m, 2H), 7,81 - 7,86 (m, 2H), 7,85 (s, 1H) ; RMN ¹³C (100 MHz, CDCl₃) : *δ* (ppm) 19,9 (CH₂), 25,6 (CH₂), 28,3 (CH), 47,0 (CH₂), 47,4 (CH₂), 52,4 (CH₂), 58,0 (CH), 119,4 (CHₐᵣₒ), 125,9 (2CHₐᵣₒ), 128,4 (CHₐᵣₒ), 129,1 (2CHₐᵣₒ), 130,7 (C_{q}), 147,9 (C_{q}) ; HRMS (EI-MS) : calculée pour C₁₅H₁₉N₄ m/z = 255,1610, trouvée m/z = 255,1613.

**(*R*)-(4-(1-(Quinuclidin-3-yl)-1*H*-1,2,3-triazol-4-yl)phényl)méthanol (38).** Le produit est isolé sous la forme d'un solide blanc avec un rendement de 32% en suivant la procédure générale D. R*_{f}* : 0,11 (CH₂Cl₂/MeOH/NH₄OH : 80/20/0,1) ; Mp : 200°C ; IR (ATR, Diamond) : *v* (cm⁻¹) : 978, 1017, 1041, 1059, 1221, 1337, 1428, 1450, 1610, 2878, 2939, 3127, 3353 ; RMN ¹H (250 MHz, DMSO-*d₆*) : *δ* (ppm) 1,39 - 1,56 (m, 2H), 1,71 - 1,82 (m, 2H), 2,22 (q, 1H, *J* = 3,0 Hz), 2,77 - 2,87 (m, 3H), 2,95 - 3,08 (m, 1H), 3,34 - 3,55 (m, 3H), 4,56 (d, 1H, *J* = 4,4 Hz), 4,74 - 4,83 (m, 1H), 5,25 (t, 1H, *J* = 4,4 Hz), 7,42 (d, 2H, *J* = 8,3 Hz), 7,87 (d, 2H, *J* = 8,3 Hz), 8,74 (s, 1H) ; RMN ¹³C (100 MHz, DMSO-*d₆*) : *δ* (ppm) 19,6 (CH₂), 25,3 (CH₂), 27,6 (CH), 46,3 (CH₂), 46,6 (CH₂), 51,9 (CH₂), 57,4 (CH), 62,6 (CH₂), 120,6 (CHₐᵣₒ), 124,9 (2CHₐᵣₒ), 126,8 (2CHₐᵣₒ), 129,3 (C_{q}), 142,1 (C_{q}), 146,1 (C_{q}) ; HRMS (EI-MS) : calculée pour C₁₆H₂₁N₄O m/z = 285,1715, trouvée m/z = 285,1702.

**(*R*)-3-(4-(4-Chlorophényl)-1*H*-1,2,3-triazol-1-yl)quinuclidine (39).** Le produit est isolé sous la forme d'un solide blanc avec un rendement de 28% en suivant la procédure générale D. R*_{f}* : 0,41 (CH₂Cl₂/MeOH/NH₄OH : 80/20/0,1) ; Mp : 144°C ; IR (ATR, Diamond) : *v* (cm⁻¹) : 971, 1014, 1060, 1092, 1190, 1202, 1324, 1401, 1433, 1452, 1468, 1484, 2868, 2939, 3110 ; RMN ¹H (250 MHz, CDCl₃) : *δ* (ppm) 1,40 - 1,50 (m, 1H), 1,60 - 1,73 (m, 1H), 1,73 - 1,87 (m, 2H), 2,28 (sext, 1H, *J* = 3,1 Hz), 2,84 - 3,00 (m, 3H), 3,08 - 3,22 (m, 1H), 3,49 (ddd, 1H, *J* = 14,3 Hz, 9,9 Hz et 2,2 Hz), 3,70 (dd, 1H, *J* = 14,3 Hz et 5,0 Hz), 4,55 - 4,71 (m, 1H), 7,40 (d, 2H, *J* = 8,7 Hz), 7,77 (d, 2H, *J* = 8,7 Hz), 7,80 (s, 1H) ; RMN ¹³C (100 MHz, CDCl₃) : *δ* (ppm) 20,2 (CH₂), 26,2 (CH₂), 28,4 (CH), 47,1 (CH₂), 47,5 (CH₂), 52,9 (CH₂), 58,7 (CH), 119,2 (CHₐᵣₒ), 127,1 (2CHₐᵣₒ), 129,3 (2CHₐᵣₒ), 129,4 (C_{q}), 134,1 (C_{q}), 146,7 (C_{q}) ; HRMS (EI-MS) : calculée pour C₁₅H₁₈N₄Cl m/z = 289,1220, trouvée m/z = 289,1211.

**(*R*)-3-(4-(3,4-Dichlorophényl)-1*H*-1,2,3-triazol-1-yl)quinuclidine (40).** Le produit est isolé sous la forme d'un solide blanc avec un rendement de 17% en suivant la procédure générale D. R*_{f}* : 0,33 (CH₂Cl₂/MeOH/NH₄OH : 80/20/0,1) ; Mp : 108°C ; IR (ATR, Diamond) : *v* (cm⁻¹) : 986, 1029, 1044, 1133, 1231, 1324, 1457, 1561, 1606, 2871, 2942, 3385 ; RMN ¹H (250 MHz, CDCl₃) : *δ* (ppm) 1,41 - 1,57 (m, 1H), 1,59 - 1,74 (m, 1H), 1,76 - 1,87 (m, 2H), 2,28 (q, 1H, *J* = 3,0 Hz), 2,86 - 3,00 (m, 3H), 3,08 - 3,22 (m, 1H), 3,44 - 3,57 (m, 1H), 3,70 (dd, 1H, *J* = 14,4 Hz et 5,0 Hz), 4,61 - 4,71 (m, 1H), 7,48 (d, 1H, *J* = 8,4 Hz), 7,65 (dd, 1H, *J* = 8,4 Hz et 1,9 Hz), 7,84 (s, 1H), 7,91 (d, 1H, *J* = 1,9 Hz) ; RMN ¹³C (100 MHz, CDCl₃) : *δ* (ppm) 20,1 (CH₂), 26,0 (CH₂), 28,3 (CH), 47,1 (CH₂), 47,4 (CH₂), 52,7 (CH₂), 58,7 (CH), 119,7 (CHₐᵣₒ), 125,0 (CHₐᵣₒ), 127,6 (CHₐᵣₒ), 130,9 (C_{q}), 131,0 (CHₐᵣₒ), 132,1 (C_{q}), 133,2 (C_{q}), 145,6 (C_{q}) ; HRMS (EI-MS) : calculée pour C₁₅H₁₇N₄Cl₂ m/z = 323,0830, trouvée m/z = 323,0827.

**(*R*)-3-(4-(4-Bromophényl)-1*H*-1,2,3-triazol-1-yl)quinuclidine (41).** Le produit est isolé sous la forme d'un solide blanc avec un rendement de 23% en suivant la procédure générale D. R*_{f}* : 0,32 (CH₂Cl₂/MeOH/NH₄OH : 80/20/0,1) ; Mp : 158°C ; IR (ATR, Diamond): *v* (cm⁻¹) : 972, 1009, 1043, 1060, 1070, 1186, 1218, 1315, 1424, 1451, 1479, 2867, 2936 ; RMN ¹H (250 MHz, CDCl₃) : *δ* (ppm) 1,40 - 1,57 (m, 1H), 1,60 - 1,72 (m, 1H), 1,72 - 1,86 (m, 2H), 2,28 (q, 1H, *J* = 3,1 Hz), 2,86 - 3,00 (m, 3H), 3,07 - 3,22 (m, 1H), 3,49 (ddd, 1H, *J* = 14,5 Hz, 9,8 Hz et 2,2 Hz), 3,69 (dd, 1H, *J* = 14,4 Hz et 5,0 Hz), 4,58 - 4,71 (m, 1H), 7,54 (d, 2H, *J* = 8,6 Hz), 7,71 (d, 2H, *J* = 8,6 Hz), 7,81 (s, 1H) ; RMN ¹³C (100 MHz, CDCl₃) : *δ* (ppm) 20,2 (CH₂), 26,2 (CH₂), 28,4 (CH), 47,1 (CH₂), 47,5 (CH₂), 52,9 (CH₂), 58,7 (CH), 119,3 (CHₐᵣₒ), 122,2 (C_{q}), 127,4 (2CHₐᵣₒ), 129,8 (C_{q}), 132,2 (2CHₐᵣₒ), 146,8 (C_{q}) ; HRMS (EI-MS) : calculée pour C₁₅H₁₈N₄Br m/z = 333,0715, trouvée m/z = 333,0722.

**(*R*)-3-(4-(4-Fluorophényl)-1*H*-1,2,3-triazol-1-yl)quinuclidine (42).** Le produit est isolé sous la forme d'un solide blanc avec un rendement de 29% en suivant la procédure générale D. R*_{f}* : 0,37 (CH₂Cl₂/MeOH/NH₄OH: 80/20/0,1); Mp : 180°C ; IR (ATR, Diamond) : *v* (cm⁻¹) : 973, 1014, 1044, 1060, 1160, 1225, 1453, 1495, 1560, 2375, 2869, 2936 ; RMN ¹H (250 MHz, DMSO-*d₆*) : *δ* (ppm) 1,49 - 1,62 (m, 2H), 1,81 - 1,94 (m, 2H), 2,31 - 2,36 (m, 1H), 2,94 - 3,08 (m, 3H), 3,12 - 3,26 (m, 1H), 3,57 - 3,71 (m, 2H), 3,91 - 5,02 (m, 1H), 7,34 (t, 2H, *J* = 8,6 Hz), 7,94 (dd, 2H, *J* = 8,6 Hz et 5,3 Hz), 8,83 (s, 1H) ; RMN ¹³C (100 MHz, DMSO-*d₆*) : *δ* (ppm) 19,7 (CH₂), 23,8 (CH₂), 27,1 (CH), 45,9 (CH₂), 46,1 (CH₂), 50,8 (CH₂), 56,3 (CH), 115,8 (d, 2CHₐᵣₒ, *J* = 22 Hz), 121,0 (CHₐᵣₒ), 127,1 (d, 2CHₐᵣₒ, *J* = 8 Hz), 127,3 (d, C_{q}, *J* = 3 Hz), 145,4 (C_{q}), 161,7 (d, C_{q}, *J* = 244 Hz) ; HRMS (EI-MS) : calculée pour C₁₅H₁₈FN₄ m/z = 273,1516, trouvée m/z = 273,1508.

**(*R*)-3-(4-(3-Fluorophényl)-1*H*-1,2,3-triazol-1-yl)quinuclidine (43).** Le produit est isolé sous la forme d'un solide blanc avec un rendement de 29% en suivant la procédure générale D. R*_{f}* : 0,31 (CH₂Cl₂/MeOH/NH₄OH : 80/20/0,1) ; Mp : 120°C ; IR (ATR, Diamond) : *v* (cm⁻¹) : 1041, 1061, 1148, 1214, 1266, 1315, 1345, 1449, 1487, 1589, 1620, 2869, 2936 ; RMN ¹H (250 MHz, CDCl₃) : *δ* (ppm) 1,40 - 1,55 (m, 1H), 1,60 - 1,72 (m, 1H), 1,72 - 1,86 (m, 2H), 2,28 (q, 1H, *J* = 3,1 Hz), 2,84 - 3,00 (m, 3H), 3,08 - 3,22 (m, 1H), 3,50 (ddd, 1H, *J* = 14,5 Hz, 9,8 Hz et 2,0 Hz), 3,70 (dd, 1H, *J* = 14,5 Hz et 5,0 Hz), 4,60 - 4,70 (m, 1H), 7,03 (td, 1H, *J* = 8,3 Hz et 2,3 Hz), 7,33 - 7,44 (m, 1H), 7,52 - 7,63 (m, 1H), 7,59 (s, 1H), 7,82 (s, 1H) ; RMN ¹³C (100 MHz, CDCl₃) : *δ* (ppm) 20,2 (CH₂), 26,2 (CH₂), 28,4 (CH), 47,1 (CH₂), 47,5 (CH₂), 52,9 (CH₂), 58,8 (CH), 112,8 (d, CHₐᵣₒ, *J* = 22 Hz), 115,1 (d, CHₐᵣₒ, *J* = 22 Hz), 119,6 (CHₐᵣₒ), 121,5 (d, CHₐᵣₒ, *J* = 3 Hz), 130,6 (d, CHₐᵣₒ, *J* = 8 Hz), 133,0 (d, C_{q}, *J* = 8 Hz), 146,8 (d, C_{q}, *J* = 3 Hz), 163,4 (d, Cq, *J* = 246 Hz) ; HRMS (EI-MS) : calculée pour C₁₅H₁₈FN₄ m/z = 273,1516, trouvée m/z = 273,1516.

**(*R*)-3-(4-(2-Fluorophényl)-1*H*-1,2,3-triazol-1-yl)quinuclidine (44).** Le produit est isolé sous la forme d'un solide blanc avec un rendement de 22% en suivant la procédure générale D. R*_{f}* : 0,42 (CH₂Cl₂/MeOH/NH₄OH : 80/20/0,1) ; Mp : 94°C ; IR (ATR, Diamond) : *v* (cm⁻¹) : 970, 1045, 1068, 1227, 1328, 1452, 1487, 1644, 2932, 3142, 3352 ; RMN ¹H (250 MHz, CDCl₃) : *δ* (ppm) 1,41 - 1,55 (m, 1H), 1,63 - 1,74 (m, 1H), 1,76 - 1,88 (m, 2H), 2,30 (q, 1H, *J* = 3,1 Hz), 2,87 - 3,00 (m, 3H), 3,10 - 3,24 (m, 1H), 3,50 (ddd, 1H, *J* = 14,4 Hz, 9,8 Hz et 2,2 Hz), 3,75 (dd, 1H, *J* = 14,4 Hz et 4,7 Hz), 4,63 - 4,72 (m, 1H), 7,09 - 7,18 (m, 1H), 7,22 - 7,34 (m, 2H), 7,98 (d, 1H, *J* = 3,7 Hz), 8,32 (dd, 1H, *J* = 7,5 Hz et 2,2 Hz) ; RMN ¹³C (100 MHz, CDCl₃) : *δ* (ppm) 20,2 (CH₂), 26,2 (CH₂), 28,4 (CH), 47,1 (CH₂), 47,5 (CH₂), 52,8 (CH₂), 58,6 (CH), 115,8 (d, CHₐᵣₒ, *J* = 22 Hz), 118,8 (d, C_{q}, *J* = 13 Hz), 122,4 (d, CHₐᵣₒ, *J* = 13 Hz), 124,8 (d, CHₐᵣₒ, *J* = 3 Hz), 128,0 (d, CHₐᵣₒ, *J* = 3 Hz), 129,5 (d, CHₐᵣₒ, *J* = 8 Hz), 141,2 (C_{q}), 159,4 (d, C_{q}, *J* = 248 Hz) ; HRMS (EI-MS) : calculée pour C₁₅H₁₈FN₄ m/z = 273,1516, trouvée m/z = 273,1529.

**(*R*)-3-(4-(3,4-Difluorophényl)-1*H*-1,2,3-triazol-1-yl)quinuclidine (45).** Le produit est isolé sous la forme d'un solide blanc avec un rendement de 24% en suivant la procédure générale D. R*_{f}* : 0,33 (CH₂Cl₂/MeOH/NH₄OH : 80/20/0,1) ; Mp : 122°C ; IR (ATR, Diamond) : *v* (cm⁻¹) : 969, 989, 1025, 1045, 1059, 1207, 1281, 1449, 1508, 1606, 2869, 2942 ; RMN ¹H (250 MHz, CDCl₃) : *δ* (ppm) 1,40 - 1,55 (m, 1H), 1,59- 1,74 (m, 1H), 1,74 - 1,88 (m, 2H), 2,26 (sext, 1H, *J* = 3,1 Hz), 2,85 - 2,99 (m, 3H), 3,07 - 3,20 (m, 1H), 3,48 (ddd, 1H, *J* = 14,4 Hz, 9,7 Hz et 2,1 Hz), 3,69 (dd, 1H, *J* = 14,4 Hz et 4,8 Hz), 4,59 - 4,68 (m, 1H), 7,14 - 7,26 (m, 1H), 7,50 - 7,57 (m, 1H), 7,66 (ddd, 1H, *J* = 11,2 Hz, 7,6 Hz et 2,1 Hz), 7,78 (s, 1H) ; RMN ¹³C (100 MHz, CDCl₃) : *δ* (ppm) 20,2 (CH₂), 26,2 (CH₂), 28,4 (CH), 47,1 (CH₂), 47,5 (CH₂), 52,8 (CH₂), 58,8 (CH), 114,9 (d, CHₐᵣₒ, *J* = 18 Hz), 117,9 (d, CHₐᵣₒ, *J* = 18 Hz), 119,4 (CHₐᵣₒ), 121,9 (q, CHₐᵣₒ, *J* = 6 Hz et 4 Hz), 128,0 (q, C_{q}, *J* = 6 Hz et 4 Hz), 146,0 (C_{q}), 150,3 (q, C_{q}, *J* = 248 Hz et 13 Hz), 150,8 (q, C_{q}, *J* = 248 Hz et 13 Hz) ; HRMS (EI-MS) : calculée pour C₁₅H₁₇F₂N₄ m/z = 291,1421, trouvée m/z = 291,1423.

**(*R*)-3-(4-(4-Méthoxyphényl)-1*H*-1,2,3-triazol-1-yl)quinuclidine (46).** Le produit est isolé sous la forme d'un solide blanc avec un rendement de 21% en suivant la procédure générale D. R*_{f}* : 0,33 (CH₂Cl₂/MeOH/NH₄OH : 80/20/0,1) ; Mp : 122°C ; IR (ATR, Diamond) : *v* (cm⁻¹) : 1029, 1060, 1106, 1177, 1249, 1307, 1454, 1497, 1561, 1618, 2870, 2937, 3399 ; RMN ¹H (250 MHz, DMSO-*d₆*) : *δ* (ppm) 1,38 - 1,50 (m, 2H), 1,72 - 1,82 (m, 2H), 2,19 - 2,26 (m, 1H), 2,74 - 2,92 (m, 3H), 2,96 - 3,17 (m, 1H), 3,33 - 3,62 (m, 2H), 3,83 (s, 3H), 4,74 - 4,83 (m, 1H), 7,05 (d, 2H, *J* = 8,5 Hz), 7,83 (d, 2H, *J* = 8,6 Hz), 8,66 (s, 1H) ; RMN ¹³C (100 MHz, DMSO-*d₆*) : *δ* (ppm) 19,6 (CH₂), 25,2 (CH₂), 27,6 (CH), 46,3 (CH₂), 46,6 (CH₂), 51,8 (CH₂), 55,1 (CH₃), 57,2 (CH), 114,2 (2CHₐᵣₒ), 119,9 (CHₐᵣₒ), 123,5 (C_{q}), 126,5 (2CHₐᵣₒ), 146,1 (C_{q}), 158,9 (C_{q}) ; HRMS (EI-MS) : calculée pour C₁₆H₂₁N₄O m/z = 285,1715, trouvée m/z = 285,1726.

**(*R*)-3-(4-(3-Méthoxyphényl)-1*H*-1,2,3-triazol-1-yl)quinuclidine (47).** Le produit est isolé sous la forme d'une huile jaunâtre avec un rendement de 32% en suivant la procédure générale D. R*_{f}* : 0,45 (CH₂Cl₂/MeOH/NH₄OH : 80/20/0,1) ; IR (ATR, Diamond) : *v* (cm⁻¹) : 1040, 1158, 1243, 1282, 1321, 1455, 1483, 1584, 1610, 2872, 2942 ; RMN ¹H (250 MHz, CDCl₃) : *δ* (ppm) 1,39 - 1,54 (m, 1H), 1,61 - 1,72 (m, 1H), 1,72 - 1,85 (m, 2H), 2,27 (sext, 1H, *J* = 3,1 Hz), 2,84 - 2,98 (m, 3H), 3,08 - 3,22 (m, 1H), 3,48 (ddd, 1H, *J* = 14,5 Hz, 9,8 Hz et 2,2 Hz), 3,71 (dd, 1H, *J* = 14,5 Hz et 4,8 Hz), 3,85 (s, 3H), 4,59 - 4,68 (m, 1H), 6,85 - 6,90 (m, 1H) 7,28 - 7,38 (m, 2H), 7,44 - 7,47 (m, 1H), 7,80 (s, 1H) ; RMN ¹³C (100 MHz, CDCl₃) : *δ* (ppm) 20,2 (CH₂), 26,2 (CH₂), 28,3 (CH), 47,1 (CH₂), 47,5 (CH₂), 52,8 (CH₂), 55,6 (CH₃), 58,6 (CH), 110,9 (CHₐᵣₒ), 114,4 (CHₐᵣₒ), 118,2 (CHₐᵣₒ), 119,4 (CHₐᵣₒ), 130,1 (CHₐᵣₒ), 132,1 (C_{q}), 147,6 (C_{q}), 160,2 (C_{q}) ; HRMS (EI-MS) : calculée pour C₁₆H₂₁N₄O m/z = 285,1715, trouvée m/z = 285,1719.

**(*R*)-3-(4-(2-Méthoxyphényl)-1*H*-1,2,3-triazol-1-yl)quinuclidine (48).** Le produit est isolé sous la forme d'un solide blanc avec un rendement de 28% en suivant la procédure générale D. R*_{f}* : 0,50 (CH₂Cl₂/MeOH/NH₄OH : 80/20/0,1) ; Mp : 120°C ; IR (ATR, Diamond) : *v* (cm⁻¹) : 969, 1017, 1043, 1067, 1120, 1240, 1322, 1434, 1488, 1583, 2864, 2927 ; RMN ¹H (250 MHz, CDCl₃) : *δ* (ppm) 1,37 - 1,52 (m, 1H), 1,63 - 1,74 (m, 1H), 1,74 - 1,85 (m, 2H), 2,28 (sext, 1H, *J* = 3,1 Hz), 2,85 - 2,99 (m, 3H), 3,10 - 3,24 (m, 1H), 3,46 (ddd, 1H, J= 14,3 Hz, 9,8 Hz et 2,2 Hz), 3,75 (dd, 1H, J= 14,3 Hz et 5,2 Hz), 3,94 (s, 3H), 4,59 - 4,68 (m, 1H), 6,97 (d, 1H, *J* = 8,3 Hz), 7,08 (td, 1H, *J* = 7,6 Hz et 1,1 Hz), 7,31 (ddd, 1H, *J* = 8,3 Hz, 7,6 Hz et 1,8 Hz), 8,08 (s, 1H), 8,35 (dd, 1H, *J* = 7,6 Hz et 1,8 Hz) ; RMN ¹³C (100 MHz, CDCl₃) : *δ* (ppm) 20,3 (CH₂), 26,2 (CH₂), 28,4 (CH), 47,2 (CH₂), 47,5 (CH₂), 52,8 (CH₂), 55,6 (CH₃), 58,4 (CH), 111,0 (CHₐᵣₒ), 120,0 (C_{q}), 121,3 (CHₐᵣₒ), 122,7 (CHₐᵣₒ), 127,8 (CHₐᵣₒ), 129,0 (CHₐᵣₒ), 143,1 (C_{q}), 155,8 (C_{q}) ; HRMS (EI-MS): calculée pour C₁₆H₂₁N₄O m/z = 285,1723, trouvée m/z = 285,1715.

**(*R*)-3-(4-(6-Méthoxynaphthalèn-2-yl)-1*H*-1,2,3-triazol-1-yl)quinuclidine (49).** Le produit est isolé sous la forme d'un solide blanc avec un rendement de 21% en suivant la procédure générale D. R*_{f}* : 0,29 (CH₂Cl₂/MeOH/NH₄OH : 80/20/0,1) ; Mp : 186°C ; IR (ATR, Diamond): *v* (cm⁻¹) : 906, 1025, 1123, 1162, 1210, 1262, 1344, 1394, 1454, 1479, 1612, 1630, 2869, 2932 ; RMN ¹H (250 MHz, CDCl₃) : *δ* (ppm) 1,42 - 1,56 (m, 1H), 1,65 - 1,76 (m, 1H), 1,76 - 1,88 (m, 2H), 2,31 (q, 1H, *J* = 3,1 Hz), 2,86 - 3,02 (m, 3H), 3,10 - 3,24 (m, 1H), 3,52 (ddd, 1H, *J* = 14,4 Hz, 9,7 Hz et 2,1 Hz), 3,73 (dd, 1H, *J* = 14,4 Hz et 5,2 Hz), 3,93 (s, 3H), 4,58 - 4,75 (m, 1H), 7,15 (s, 1H), 7,14 - 7,20 (m, 1H), 7,79 (d, 2H, *J* = 9,2 Hz), 7,89 (s, 1H), 7,91 (dd, 1H, *J* = 8,6 Hz et 1,7 Hz), 8,26 (s, 1H) ; RMN ¹³C (100 MHz, CDCl₃) : *δ* (ppm) 20,3 (CH₂), 26,2 (CH₂), 28,4 (CH), 47,2 (CH₂), 47,5 (CH₂), 52,9 (CH₂), 55,6 (CH₃), 58,7 (CH), 106,0 (CHₐᵣₒ), 119,0 (CHₐᵣₒ), 119,5 (CHₐᵣₒ), 124,5 (CHₐᵣₒ), 124,6 (CHₐᵣₒ), 126,1 (C_{q}), 127,6 (CHₐᵣₒ), 129,2 (C_{q}), 129,9 (CHₐᵣₒ), 134,6 (C_{q}), 148,0 (C_{q}), 158,1 (C_{q}) ; HRMS (EI-MS) : calculée pour C₂₀H₂₃N₄O m/z = 335,1872, trouvée m/z = 335,1866.

**(*R*)-3-(4-(Pyridin-4-yl)-1*H*-1,2,3-triazol-1-yl)quinuclidine (50).** Le produit est isolé sous la forme d'un solide brun avec un rendement de 30% en suivant la procédure générale D. R*_{f}* : 0,21 (CH₂Cl₂/MeOH/NH₄OH : 80/20/0,1) ; Mp : 150°C ; IR (ATR, Diamond) : *v* (cm⁻¹) : 991, 1041, 1058, 1073, 1208, 1227, 1325, 1413, 1430, 1562, 1613, 2869, 2935 ; RMN ¹H (250 MHz, CDCl₃) : *δ* (ppm) 1,42 - 1,56 (m, 1H), 1,60 - 1,76 (m, 1H), 1,76 - 1,88 (m, 2H), 2,29 (q, 1H, *J* = 3,1 Hz), 2,86 - 3,00 (m, 3H), 3,07 - 3,25 (m, 1H), 3,50 (ddd, 1H, *J* = 14,5 Hz, 9,7 Hz et 2,1 Hz), 3,71 (dd, 1H, *J* = 14,4 Hz et 5,0 Hz), 4,63 - 4,72 (m, 1H), 7,73 (d, 2H, *J* = 6,0 Hz), 7,95 (s, 1H), 8,66 (d, 2H, *J* = 6,0 Hz) ; RMN ¹³C (100 MHz, CDCl₃) : *δ* (ppm) 20,2 (CH₂), 26,2 (CH₂), 28,4 (CH), 47,1 (CH₂), 47,5 (CH₂), 52,9 (CH₂), 59,0 (CH), 120,1 (2CHₐᵣₒ), 120,7 (CHₐᵣₒ), 138,2 (C_{q}), 145,3 (C_{q}), 150,7 (2CHₐᵣₒ) ; HRMS (EI-MS): calculée pour C₁₄H₁₈N₅ m/z = 256,1562, trouvée m/z = 256,1550.

**(*R*)-3-(4-(6-Fluoropyridin-3-yl)-1*H*-1,2,3-triazol-1-yl)quinuclidine (51).** Le produit est isolé sous la forme d'un solide blanc avec un rendement de 18% en suivant la procédure générale D. R*_{f}* : 0,32 (CH₂Cl₂/MeOH/NH₄OH : 80/20/0,1) ; Mp : 147°C ; IR (ATR, Diamond) : *v* (cm⁻¹) : 976, 1024, 1044, 1060, 1237, 1316, 1429, 1471, 1552, 1593, 2870, 2935 ; RMN ¹H (400 MHz, CDCl₃) : *δ* (ppm) 1,45 - 1,54 (m, 1H), 1,63 - 1,72 (m, 1H), 1,74 - 1,90 (m, 2H), 2,29 (q, 1H, *J* = 3,1 Hz), 2,87 - 3,02 (m, 3H), 3,11 - 3,20 (m, 1H), 3,51 (ddd, 1H, *J* = 14,4 Hz, 9,8 Hz et 2,0 Hz), 3,71 (dd, 1H, *J* = 14,4 Hz et 5,1 Hz), 4,63 - 4,69 (m, 1H), 7,02 (dd, 1H, *J* = 8,5 Hz et 2,9 Hz), 7,87 (s, 1H), 8,28 - 8,37 (m, 1H), 8,60 (d, 1H, *J* = 2,5 Hz) ; RMN ¹³C (100 MHz, CDCl₃) : *δ* (ppm) 20,2 (CH₂), 26,2 (CH₂), 28,4 (CH), 47,1 (CH₂), 47,5 (CH₂), 52,9 (CH₂), 58,9 (CH), 110,1 (d, CHₐᵣₒ, *J* = 38 Hz), 119,4 (CHₐᵣₒ), 125,3 (d, C_{q}, *J* = 5 Hz), 138,7 (d, CHₐᵣₒ, *J* = 8 Hz), 143,8 (C_{q}), 144,9 (d, CHₐᵣₒ, *J* = 15 Hz), 163,5 (d, C_{q}, J= 240 Hz) ; HRMS (EI-MS) : calculée pour C₁₄H₁₇N₅F m/z = 274,1468, trouvée m/z = 274,1465.

**(*R*)-3-(4-(6-Méthoxypyridin-3-yl)-1*H*-1,2,3-triazol-1-yl)quinuclidine (52).** Le produit est isolé sous la forme d'un solide blanc avec un rendement de 28% en suivant la procédure générale D. R*_{f}* : 0,28 (CH₂Cl₂/MeOH/NH₄OH : 80/20/0,1) ; Mp : 155°C ; IR (ATR, Diamond) : *v* (cm⁻¹) : 973, 1027, 1215, 1254, 1282, 1325, 1420, 1481, 1555, 1614, 1729, 2869, 2938 ; RMN ¹H (250 MHz, CDCl₃) : *δ* (ppm) 1,40 - 1,55 (m, 1H), 1,62 - 1,74 (m, 1H), 1,74 - 1,88 (m, 2H), 2,27 (q, 1H, *J* = 3,0 Hz), 2,84 - 3,00 (m, 3H), 3,08 - 3,22 (m, 1H), 3,42 -3,56 (m, 1H), 3,72 (dd, 1H, *J =* 14,3 Hz et 4,3 Hz), 3,97 (s, 3H), 4,58 - 4,69 (m, 1H), 6,81 (d, 1H, *J* = 8,6 Hz), 7,77 (s, 1H), 8,07 (dd, 1H, *J =* 8,6 Hz et 2,2 Hz), 8,56 (d, 1H, *J =* 2,2 Hz) ; RMN ¹³C (100 MHz, CDCl₃) : *δ*(ppm) 20,3 (CH₂), 26,2 (CH₂), 28,4 (CH), 47,2 (CH₂), 47,5 (CH₂), 52,9 (CH₂), 53,8 (CH₃), 58,8 (CH), 111,3 (CHₐᵣₒ), 118,7 (CHₐᵣₒ), 120,5 (C_{q}), 136,5 (CHₐᵣₒ), 144,3 (CHₐᵣₒ), 145,0 (C_{q}), 164,2 (C_{q}) ; HRMS (EI-MS) : calculée pour C₁₅H₂₀N₅O m/z = 286,1668, trouvée m/z = 286,1681.

**(*R*)-3-(4-(Thiophèn-2-yl)-1*H*-1,2,3-triazol-1-yl)quinuclidine (53)**. Le produit est isolé sous la forme d'un solide brun avec un rendement de 38% en suivant la procédure générale D. R*_{f}*: 0,42 (CH₂Cl₂/MeOH/NH₄OH : 80/20/0,1) ; Mp : 137°C ; IR (ATR, Diamond) : *v* (cm⁻¹) : 931, 973, 1027, 1042, 1062, 1159, 1212, 1295, 1316, 1432, 1451, 2867, 2937, 3074 ; RMN ¹H (250 MHz, CDCl₃) : *δ*(ppm) 1,38 - 1,53 (m, 1H), 1,59 - 1,72 (m, 1H), 1,72 - 1,85 (m, 2H), 2,26 (q, 1H, *J* = 3,1 Hz), 2,83 - 2,98 (m, 3H), 3,06 - 3,20 (m, 1H), 3,47 (ddd, 1H, *J* = 14,5 Hz, 9,7 Hz et 2,0 Hz), 3,68 (dd, 1H, *J* = 14,5 Hz et 5,0 Hz), 4,57 - 4,66 (m, 1H), 7,07 (dd, 1H, *J* = 5,1 Hz et 3,6 Hz), 7,25 - 7,30 (m, 1H), 7,38 (dd, 1H, *J* = 3,6 Hz et 1,1 Hz), 7,73 (s, 1H) ; RMN ¹³C (100 MHz, CDCl₃) : *δ*(ppm) 20,2 (CH₂), 26,2 (CH₂), 28,3 (CH), 47,1 (CH₂), 47,4 (CH₂), 52,8 (CH₂), 58,7 (CH), 118,7 (CHₐᵣₒ), 124,3 (CHₐᵣₒ), 125,2 (CHₐᵣₒ), 127,8 (CHₐᵣₒ), 133,2 (C_{q}), 142,9 (C_{q}) ; HRMS (EI-MS) : calculée pour C₁₃H₁₇N₄S m/z = 261,1174, trouvée m/z = 261,1185.

**(*R*)-3-(4-(5-Bromothiophèn-2-yl)-1*H*-1,2,3-triazol-1-yl)quinuclidine (54).** Le produit est isolé sous la forme d'un solide blanc avec un rendement de 42% en suivant la procédure générale D. R*_{f}*: 0,46 (CH₂Cl₂/MeOH/NH₄OH : 80/20/0,1) ; Mp : 146°C ; IR (ATR, Diamond) : *v* (cm⁻¹) : 972, 1041, 1057, 1215, 1322, 1433, 1496, 1645, 2867, 2934, 3356 ; RMN ¹H (400 MHz, CDCl₃) : *δ*(ppm) 1,44 - 1,51 (m, 1H), 1,59 - 1,69 (m, 1H), 1,71 - 1,87 (m, 2H), 2,25 (q, 1H, J= 3,0 Hz), 2,83 - 2,99 (m, 3H), 3,07 - 3,16 (m, 1H), 3,47 (ddd, 1H, *J* = 14,4 Hz, 9,8 Hz et 2,2 Hz), 3,66 (ddd, 1H, *J* = 14,4 Hz, 5,1 Hz et 1,6 Hz), 4,57 - 4,63 (m, 1H), 7,00 (d, 1H, *J* = 3,8 Hz), 7,08 (d, 1H, *J* = 3,8 Hz), 7,69 (s, 1H) ; RMN ¹³C (100 MHz, CDCl₃) : *δ*(ppm) 20,1 (CH₂), 26,1 (CH₂), 28,3 (CH), 47,0 (CH₂), 47,4 (CH₂), 52,7 (CH₂), 58,7 (CH), 112,1 (C_{q}), 118,7 (CHₐᵣₒ), 124,3 (CHₐᵣₒ), 130,6 (CHₐᵣₒ), 134,8 (C_{q}), 142,1 (C_{q}) ; HRMS (EI-MS) : calculée pour C₁₃H₁₆N₄SBr m/z = 339,0279, trouvée m/z = 339,0283.

**(*R*)-3-(4-(Benzo[*b*]thiophèn-5-yl)-1*H*-1,2,3-triazol-1-yl)quinuclidine (55).** Le produit est isolé sous la forme d'un solide blanc avec un rendement de 32% en suivant la procédure générale D. R*_{f}*: 0,32 (CH₂Cl₂/MeOH/NH₄OH : 80/20/0,1) ; Mp : 178°C ; IR (ATR, Diamond) : *v*(cm⁻¹) : 1023, 1047, 1202, 1223, 1323, 1440, 2866, 2933 ; RMN ¹H (250 MHz, CDCl₃) : *δ*(ppm) 1,44 - 1,53 (m, 1H), 1,66 - 1,76 (m, 1H), 1,76 - 1,88 (m, 2H), 2,30 (q, 1H, *J* = 3,0 Hz), 2,86 - 3,00 (m, 3H), 3,11 - 3,21 (m, 1H), 3,50 (ddd, 1H, *J* = 14,5 Hz, 9,8 Hz et 2,1 Hz), 3,70 (dd, 1H, *J* = 14,5 Hz et 5,0 Hz), 4,62 - 4,69 (m, 1H), 7,37 (d, 1H, *J* = 5,4 Hz), 7,47 (d, 1H, *J* = 5,4 Hz), 7,80 (dd, 1H, *J* = 8,4 Hz et 1,1 Hz), 7,86 (s, 1H), 7,92 (d, 1H, *J* = 8,4 Hz), 8,33 (d, 1H, *J* = 1,1 Hz) ; RMN ¹³C (100 MHz, CDCl₃) : *δ*(ppm) 20,3 (CH₂), 26,2 (CH₂), 28,4 (CH), 47,2 (CH₂), 47,5 (CH₂), 52,9 (CH₂), 58,6 (CH), 119,1 (CHₐᵣₒ), 120,8 (CHₐᵣₒ), 122,4 (CHₐᵣₒ), 123,1 (CHₐᵣₒ), 124,2 (CHₐᵣₒ), 127,2 (C_{q}), 127,4 (CHₐᵣₒ), 139,6 (C_{q}), 140,2 (C_{q}), 147,9 (C_{q}) ; HRMS (EI-MS) : calculée pour C₁₇H₁₉N₄S m/z = 311,1330, trouvée m/z = 311,1322.

**(*R*)-3-(4-(Benzofuran-5-yl)-1*H*-1,2,3-triazol-1-yl)quinuclidine (56).** Le produit est isolé sous la forme d'un solide blanc avec un rendement de 34% en suivant la procédure générale D. R*_{f}*: 0,34 (CH₂Cl₂/MeOH/NH₄OH : 80/20/0,1) ; Mp : 178°C ; IR (ATR, Diamond) : *v* (cm⁻¹) : 989, 1029, 1042, 1062, 1109, 1194, 1221, 1321, 1455, 1497, 2870, 2935 ; RMN ¹H (250 MHz, CDCl₃) : *δ*(ppm) 1,44 - 1,53 (m, 1H), 1,67 - 1,77 (m, 1H), 1,77 - 1,89 (m, 2H), 2,30 (q, 1H, *J* = 3,0 Hz), 2,88 - 3,00 (m, 3H), 3,12 - 3,22 (m, 1H), 3,46 - 3,55 (m, 1H), 3,73 (dd, 1H, *J* = 14,3 Hz et 4,3 Hz), 4,63 - 4,69 (m, 1H), 6,81 (d, 1H, *J* = 2,1 Hz), 7,54 (d, 1H, *J* = 8,6 Hz), 7,64 (d, 1H, *J* = 2,1 Hz), 7,76 (dd, 1H, *J* = 8,6 Hz et 1,6 Hz), 7,82 (s, 1H), 8,10 (d, 1H, *J* = 1,6 Hz) ; RMN ¹³C (100 MHz, CDCl₃) : *δ*(ppm) 20,3 (CH₂), 26,2 (CH₂), 28,4 (CH), 47,2 (CH₂), 47,5 (CH₂), 52,9 (CH₂), 58,6 (CH), 107,0 (CHₐᵣₒ), 111,9 (CHₐᵣₒ), 118,6 (CHₐᵣₒ), 118,8 (CHₐᵣₒ), 122,6 (CHₐᵣₒ), 125,9 (C_{q}), 128,2 (C_{q}), 145,9 (CHₐᵣₒ), 148,2 (C_{q}), 155,1 (C_{q}) ; HRMS (EI-MS) : calculée pour C₁₇H₁₉N₄O m/z = 295,1559, trouvée m/z = 295,1557.

**(*S*)-3-(4-(4-Fluorophényl)-1*H*-1,2,3-triazol-1-yl)quinuclidine (57).** Le produit est isolé sous la forme d'un solide blanc avec un rendement de 33% en suivant la procédure générale D. R*_{f}*: 0,37 (CH₂Cl₂/MeOH/NH₄OH: 80/20/0,1); Mp : 139°C ; IR (ATR, Diamond) : *v* (cm⁻¹) : 974, 1022, 1042, 1060, 1160, 1225, 1330, 1400, 1452, 1494, 1560, 1612, 2869, 2936 ; RMN ¹H (250 MHz, CDCl₃) : *δ*(ppm) 1,39 - 1,54 (m, 1H), 1,60 - 1,74 (m, 1H), 1,74 - 1,88 (m, 2H), 2,26 (sext, 1H, *J* = 3,1 Hz), 2,84 - 2,98 (m, 3H), 3,07 - 3,21 (m, 1H), 3,48 (ddd, 1H, *J* = 14,4 Hz, 9,8 Hz et 2,3 Hz), 3,70 (dd, 1H, *J* = 14,4 Hz et 5,1 Hz), 4,59 - 4,68 (m, 1H), 7,11 (t, 2H, *J* = 8,6 Hz), 7,77 (s, 1H), 7,77 - 7,84 (m, 2H) ; RMN ¹³C (100 MHz, CDCl₃) : *δ*(ppm) 20,3 (CH₂), 26,2 (CH₂), 28,4 (CH), 47,2 (CH₂), 47,5 (CH₂), 52,9 (CH₂), 58,7 (CH), 116,0 (d, 2CHₐᵣₒ, *J* = 22 Hz), 118,9 (CHₐᵣₒ), 127,1 (d, C_{q}, *J* = 3 Hz), 127,6 (d, 2CHₐᵣₒ, *J* = 8 Hz), 146,9 (C_{q}), 162,8 (d, C_{q}, *J* = 247 Hz) ; HRMS (EI-MS) : calculée pour C₁₅H₁₈N₄F m/z = 273,1516, trouvée m/z = 273,1509.

**(R)-3-[4-(5-bromo-2-furyl)-1*H*-1,2,3-triazol-1-yl]quinuclidine (80).** Le produit est isolé sous la forme d'un solide blanc avec un rendement de 38% en suivant la procédure générale D. R*_{f}*: 0,26 (CH₂Cl₂/MeOH/NH₄OH : 95/5/1) ; Mp : 144°C ; IR (ATR, Diamond) : v (cm⁻¹) : 972, 1047, 1057, 1228, 1343, 1476, 1530, 1624, 2869, 2937, 3126 ; RMN ¹H (400 MHz, CDCl₃) : *δ* (ppm) 1.44 - 1.51 (m, 1H), 1.59 - 1.67 (m, 1H), 1.73 - 1.87 (m, 2H), 2.25 (q, 1H, *J* = 3.2 Hz), 2.85 - 3.00 (m, 3H), 3.07 - 3.15 (m, 1H), 3.49 (ddd, 1H, *J* = 14.4 Hz, 10.0 Hz et 2.4 Hz), 3.64 (dd, 1H, *J* = 14.4 Hz, 4.0 Hz), 4.63 - 4.66 (m, 1H), 6.40 (d, 1H, *J* = 3.6 Hz), 6.82 (d, 1H, *J* = 3.6 Hz), 7.8 (s, 1H) ; RMN ¹³C (100 MHz, CDCl₃) : *δ*(ppm) 19.9 (CH₂), 25.9 (CH₂), 28.0 (CH), 46.9 (CH₂), 47.2 (CH₂), 52.6 (CH₂), 58.5 (CH), 108.8 (CH), 113.2 (CH), 118.6 (CH), 121.4 (C_{q}), 139.4 (C_{q}), 148.3 (C_{q}) ; HRMS (EI-MS) : calculée pour C₁₃H₁₆N₄OBr m/z = 323.05020, trouvée m/z = 323.05015.

**(R)-3-[4-(4-bromo-2-thiènyl)-1*H*-1,2,3-triazol-1-yl]quinuclidine (81).** Le produit est isolé sous la forme d'un solide blanc avec un rendement de 50% en suivant la procédure générale D. R*_{f}*: 0,24 (CH₂Cl₂/MeOH/NH₄OH : 98/2/1) ; Mp : 106°C ; IR (ATR, Diamond) : *v* (cm⁻¹) : 786, 988, 1054, 1226, 1326, 1452, 1497, 2870, 2938, 3107; RMN ¹H (400 MHz, CDCl₃) : *δ*(ppm) 1.45 - 1.52 (m, 1H), 1.61 - 1.69 (m, 1H), 1.76 - 1.85 (m, 2H), 2.26 (q, 1H, *J* = *3.2* Hz), 2.85 - 3.00 (m, 3H), 3.09 - 3.16 (m, 1H), 3.49 (ddd, 1H, *J* = 14.4 Hz, 10.0 Hz et 2.1 Hz), 3.66 (dd, 1H, *J* = 14.4 Hz et 5.2 Hz), 4.60 - 4.65 (m, 1H), 7.19 (s, 1H), 7.26 (s, 1H), 7.74 (s, 1H) ; RMN ¹³C (100 MHz, CDCl₃) : *δ*(ppm) 19.9 (CH₂), 25.9 (CH₂), 28.1 (CH), 46.9 (CH₂), 47.2 (CH₂), 52.6 (CH₂), 58.6 (CH), 110.2 (C_{q}), 118.7 (CH), 122.1 (CH), 126.4 (CH), 134.3 (C_{q}), 141.5 (C_{q}) ; HRMS (EI-MS) : calculée pour C₁₃H₁₆N₄SBr m/z = 339.02766, trouvée m/z = 339.02736.

**(R)-3-[4-(4-bromo-2-furyl)-1*H*-1,2,3-triazol-1-yl]quinuclidine (82).** Le produit est isolé sous la forme d'un solide blanc avec un rendement de 43% en suivant la procédure générale D. R*_{f}*: 0,22 (CH₂Cl₂/MeOH/NH₄OH : 98/2/1) ; Mp : 129°C ; IR (ATR, Diamond) : *v* (cm⁻¹) : 785, 980, 1042, 1210, 1325, 1452, 1534, 2869, 2940, 3115; RMN ¹H (400 MHz, CDCl₃) : *δ*(ppm) 1.43 - 1.50 (m, 1H), 1.58 - 1.67 (m, 1H), 1.72 - 1.87 (m, 2H), 2.26 (q, 1H, *J* = 2.8 Hz), 2.85 - 2.99 (m, 3H), 3.07 - 3.14 (m, 1H), 3.48 (ddd, 1H, *J* = 14.4 Hz, 10.0 Hz et 2.2 Hz), 3.65 (dd, 1H, *J* = 14.4 Hz et 3.6 Hz), 4.62 - 4.65 (m, 1H), 6.87 (s, 1H), 7.44 (s, 1H), 7.78 (s, 1H) ; RMN ¹³C (100 MHz, CDCl₃) : *δ*(ppm) 19.9 (CH₂), 25.9 (CH₂), 28.0 (CH), 46.8 (CH₂), 47.2 (CH₂), 52.6 (CH₂), 58.5 (CH), 101.3 (C_{q}), 109.7 (CH), 119.0 (CH), 139.3 (C_{q}), 140.1 (CH), 147.2 (C_{q}) ; HRMS (EI-MS) : calculée pour C₁₃H₁₆N₄OBr m/z = 323.05045, trouvée m/z = 323.05020.

**(R)-3-[4-(3-bromophényl)-1*H*-1,2,3-triazol-1-yl]quinuclidine (83).** Le produit est isolé sous la forme d'un solide blanc avec un rendement de 50% en suivant la procédure générale D. R*_{f}*: 0,18 (CH₂Cl₂/MeOH/NH₄OH : 97/3/1) ; Mp : 188°C ; IR (ATR, Diamond) : *v* (cm⁻¹) : 970, 1020, 1041, 1060, 1069, 1233, 1341, 14237 1455, 1470, 1603, 2868, 2938 ; RMN ¹H (400 MHz, CDCl₃) : *δ*(ppm) 1.44 - 1.50 (m, 1H), 1.61 - 1.65 (m, 1H), 1.73 - 1.87 (m, 2H), 2.27 (q, 1H, *J* = 3.2 Hz), 2.86 - 3.00 (m, 3H), 3.09 - 3.17 (m, 1H), 3.49 (ddd, 1H, *J* = 14.4 Hz, 9.8 Hz et 2.2 Hz), 3.68 (dd, 1H, *J* = 14,4 Hz et 3.6 Hz), 4.63 - 4.65 (m, 1H), 7.29 (t, 1H, *J* = 7.6 Hz), 7.45 (d, 1H, *J* = 8.0 Hz), 7.78 (d, 1H, *J* = 8.0 Hz), 7.83 (s, 1H), 7.98 (s, 1H) ; RMN ¹³C (100 MHz, CDCl₃) : *δ*(ppm) 20.0 (CH₂), 26.0 (CH₂), 28.1 (CH), 46.9 (CH₂), 47.2 (CH₂), 52.6 (CH₂), 58.5 (CH), 119.3 (CH), 122.9 (C_{q}), 124.1 (CH), 128.6 (CH), 130.4 (CH), 131.0 (CH), 132.6 (C_{q}), 146.1 (C_{q}) ; HRMS (EI-MS) : calculée pour C₁₅H₁₈N₄Br m/z = 333.07111, trouvée m/z = 333.07094.

### Fluoration de l'alcool 38

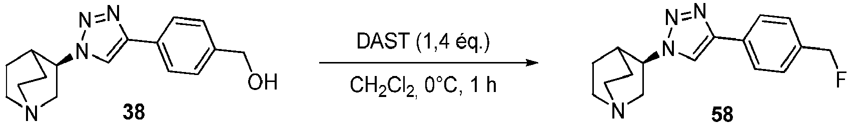

**(*R*)-3-(4-(4-(fluorométhyl)phényl)-1*H*-1,2,3-triazol-1-yl)quinuclidine (58).** L'alcool **38** (140 mg, 0,500 mmol) est solubilisé dans 10 mL de dichlorométhane puis à 0°C, du trifluorure de diethylaminosulfure (92 µL, 0,700 mmol) est ajouté goutte à goutte. La réaction est agitée à 0°C pendant une heure puis hydrolysée par l'ajout d'une solution saturée en NaHCO₃. La phase organique est séchée sur MgSO₄ anhydre, filtrée et concentrée sous pression réduite. Le composé fluoré 58 est purifié par chromatographie sur colonne de gel de silice en utilisant comme éluant un mélange CH₂Cl₂/MeOH/NH₄OH (90/10/0,1). Le produit 58 est isolé sous la forme d'un solide blanc avec un rendement de 51%. R*_{f}*: 0,41 (CH₂Cl₂/MeOH/NH₄OH : 80/20/0,1) ; Mp : 138°C ; IR (ATR, Diamond) : *v* (cm⁻¹) : 974, 1010, 1043, 1060, 1212, 1317, 1382, 1407, 1453, 1500, 2870, 2940 ; RMN ¹H (250 MHz, CDCl₃) : *δ*(ppm) 1,41 - 1,56 (m, 1H), 1,62 - 1,74 (m, 1H), 1,74 - 1,94 (m, 2H), 2,30 (q, 1H, *J* = 3,1 Hz), 2,86 - 3,00 (m, 3H), 3,09 - 3,24 (m, 1H), 3,50 (ddd, 1H, *J* = 14,6 Hz, 9,6 Hz et 2,0 Hz), 3,72 (dd, 1H, *J* = 14,5 Hz et 5,1 Hz), 4,61 - 4,70 (m, 1H), 5,41 (d, 2H, *J* = 47,8 Hz), 7,44 (dd, 2H, *J* = 8,6 Hz et 1,6 Hz), 7,84 (s, 1H), 7,88 (dd, 2H, *J* = 8,6 Hz et 1,0 Hz) ; RMN ¹³C (100 MHz, CDCl₃) : *δ*(ppm) 20,3 (CH₂), 26,2 (CH₂), 28,4 (CH), 47,1 (CH₂), 47,5 (CH₂), 52,9 (CH₂), 58,7 (CH), 84,5 (d, CH₂, *J* = 166 Hz), 119,4 (CHₐᵣₒ), 126,1 (2CHₐᵣₒ), 128,2 (d, 2CHₐᵣₒ, *J* = 6 Hz), 131,4 (d, C_{q}, *J* = 3 Hz), 136,2 (d, C_{q}, *J* = 17 Hz), 147,3 (C_{q}) ; HRMS (EI-MS) : calculée pour C₁₆H₂₀N₄F m/z = 287,1672, trouvée m/z = 287,1660.

### Préparation du composé 60 de type quinuclidine comportant un noyau triazole substitué en position 4 et 5

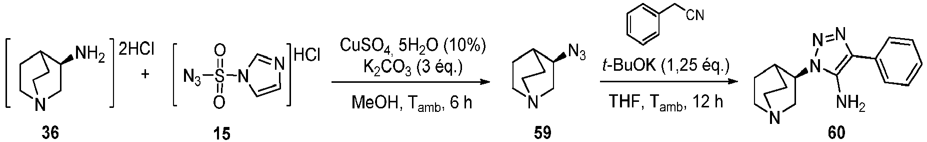

**(*R*)-4-Phényl-1-(quinuclidin-3-yl)-1*H*-1,2,3-triazol-5-amine (60)**. Sous argon, la 3(R)-aminoquinuclidine **36** (400 mg, 2,00 mmol) et l'azoture du 1*H*-imidazole-1-sulfonyle **15** (464 mg, 2,20 mmol) sont solubilisés dans 12 mL de méthanol puis sont ajoutés successivement du K₂CO₃ (830 mg, 6,00 mmol) et une quantité catalytique de CuSO₄,5H₂O (50 mg, 0,200 mmol). Le milieu réactionnel est agité à température ambiante pendant 6 h puis concentré sous pression réduite. Le solide obtenu est alors repris dans 20 mL d'éther éthylique, filtré sous vide puis lavé deux fois avec 20 mL d'éther éthylique. Après évaporation du filtrat sous pression réduite, L'azoture **59** est engagé dans l'étape suivant sans aucune autre purification.

A une solution de l'azoture **59** (2,00 mmol) dans 10 mL de THF anhydre sont ajoutés à température ambiante du phénylacétonitrile (0,28 mL, 2,40 mmol) et du *t*-BuOK (337 mg, 3,00 mmol). Le milieu réactionnel est agité à température ambiante et sous argon pendant 12 heures. Ensuite, le solvant est évaporé puis le résidu est repris dans du dichlorométhane et laver avec de l'eau. La phase organique est séchée sur MgSO₄ anhydre, filtrée et évaporée sous pression réduite. Le produit est purifié par chromatographie sur colonne de gel de silice en utilisant comme éluant un mélange CH₂Cl₂/MeOH/NH₄OH (90/10/0,1) puis (70/30/0,1). L'amine **60** est isolée sous la forme d'un solide blanc avec un rendement de 43%. R*_{f}*: 0,11 (CH₂Cl₂/MeOH/NH₄OH : 70/30/0,1) ; Mp: 120°C ; IR (ATR, Diamond): *v* (cm⁻¹) : 982, 1007, 1056, 1266, 1323, 1360, 1445, 1510, 1585, 1607, 1633, 2870, 2940, 3175, 3307 ; RMN ¹H (250 MHz, CDCl₃) : *δ*(ppm) 1,33 - 1,48 (m, 1H), 1,72 - 2,00 (m, 3H), 2,12 (q, 1H, *J* = 3,1 Hz), 2,82 - 3,01 (m, 3H), 3,26 - 3,40 (m, 2H), 3,71 - 3,80 (m, 2H), 4,01 (dd, 1H, *J* = 14,2 Hz, 4,8 Hz), 4,23 - 4,32 (m, 1H), 7,27 - 7,35 (m, 1H), 7,41 - 7,49 (m, 2H), 7,67 - 7,72 (m, 2H) ; RMN ¹³C (100 MHz, CDCl₃) : *δ*(ppm) 19,9 (CH₂), 26,1 (CH₂), 26,9 (CH), 47,2 (CH₂), 47,4 (CH₂), 51,5 (CH₂), 54,7 (CH), 125,9 (2CHₐᵣₒ), 127,2 (CHₐᵣₒ), 129,2 (2CHₐᵣₒ), 131,7 (C_{q}), 131,9 (C_{q}), 137,3 (C_{q}) ; HRMS (EI-MS) : calculée pour C₁₅H₂₀N₅ m/z = 270,1719, trouvée m/z = 270,1732.

### Préparation des composés 61-63 et 84-111 de type quinuclidine

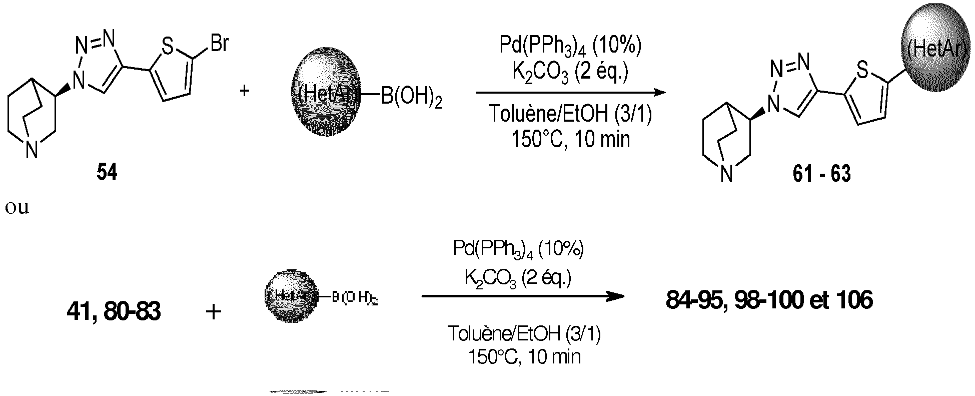

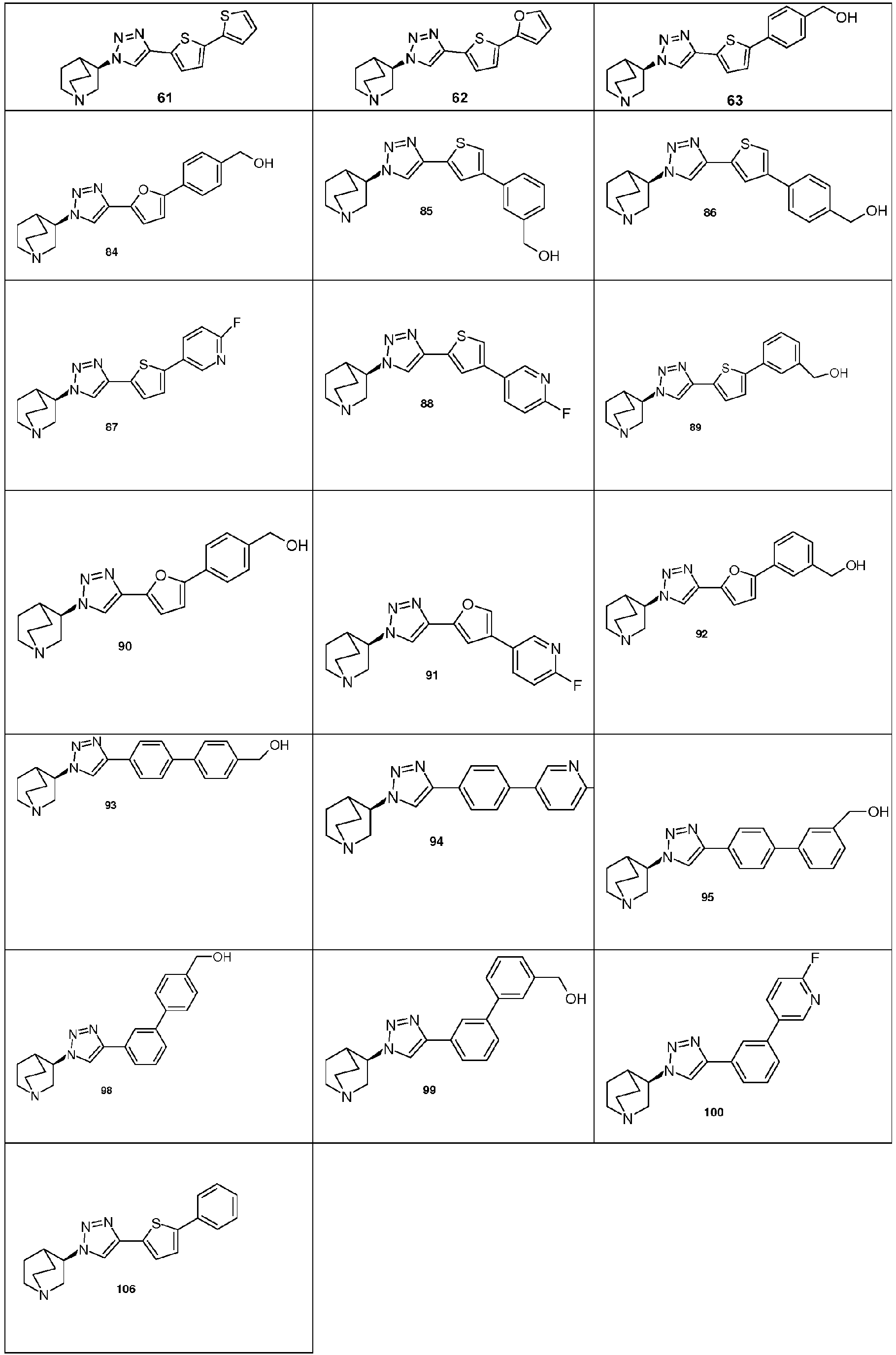

**Procédure générale E1** : A une solution d'un dérivé bromé choisi parmi les composés **54, 41** et **80-83** (50 mg, 0,147 mmol) dans un mélange de 1,5 mL de toluène et 0,5 mL d'éthanol sont ajoutés l'acide boronique souhaité (0,176 mmol), du K₂CO₃ (41 mg, 0,294 mmol) et du tetrakis(triphenylphosphine)palladium (17 mg, 0,0147 mmol). Après dégazage, la réaction est irradiée par micro-onde à 150°C pendant 10 minutes. Le solvant est ensuite évaporé puis le résidu est purifié par chromatographie sur colonne de gel de silice en utilisant comme éluant un mélange CH₂Cl₂/MeOH/NH₄OH (80/20/0,1).

**(*R*)-3-(4-(2,2'-Bithiophèn-5-yl)-1*H*-1,2,3-triazol-1-yl)quinuclidine (61)**. Le produit est isolé sous la forme d'un solide brun avec un rendement de 73% en suivant la procédure générale E1. R*_{f}*: 0,25 (CH₂Cl₂/MeOH/NH₄OH : 80/20/0,1) ; Mp : 182°C; IR (ATR, Diamond) : *v* (cm⁻¹) : 1042, 1067, 1210, 1340, 1425, 1454, 1503, 1584, 2867, 2937 ; RMN ¹H (400 MHz, CDCl₃) : *δ*(ppm) 1,44 - 1,54 (m, 1H), 1,63 - 1,74 (m, 1H), 1,76 - 1,88 (m, 2H), 2,26 - 2,30 (m, 1H), 2,86 - 3,00 (m, 3H), 3,10 - 3,20 (m, 1H), 3,45 - 3,54 (m, 1H), 3,70 (dd, 1H, *J* = 14,3 Hz et 4,6 Hz), 4,60 - 4,67 (m, 1H), 7,03 (dd, 1H, *J* = 4,9 Hz et 3,8 Hz), 7,14 (d, 1H, *J* = 3,6 Hz), 7,19 - 7,24 (m, 2H), 7,28 (d, 1H, *J* = 3,6 Hz), 7,73 (s, 1H) ; RMN ¹³C (100 MHz, CDCl₃) : *δ*(ppm) 20,1 (CH₂), 26,1 (CH₂), 28,3 (CH), 47,1 (CH₂), 47,4 (CH₂), 52,7 (CH₂), 58,7 (CH), 118,6 (CHₐᵣₒ), 124,0 (CHₐᵣₒ), 124,3 (CHₐᵣₒ), 124,7 (CHₐᵣₒ), 124,8 (CHₐᵣₒ), 128,1 (CHₐᵣₒ), 131,8 (C_{q}), 137,1 (C_{q}), 137,3 (C_{q}), 142,6 (C_{q}) ; HRMS (EI-MS) : calculée pour C₁₇H₁₉N₄S₂ m/z = 343,1051, trouvée m/z = 343,1055.

**(*R*)-3-(4-(5-(Furan-2-yl)thiophèn-2-yl)-1*H*-1,2,3-triazol-1-yl)quinuclidine (62).** Le produit est isolé sous la forme d'un solide blanc avec un rendement de 83% en suivant la procédure générale E1. R*_{f}*: 0,27 (CH₂Cl₂/MeOH/NH₄OH : 80/20/0,1) ; Mp : 180°C ; IR (ATR, Diamond): *v* (cm⁻¹) : 977, 1070, 1442, 1647, 2939, 3243 ; RMN ¹H (400 MHz, CDCl₃) : *δ*(ppm) 1,44 - 1,53 (m, 1H), 1,64 - 1,73 (m, 1H), 1,76 - 1,88 (m, 2H), 2,28 (q, 1H, *J* = 3,0 Hz), 2,87 - 3,01 (m, 3H), 3,10 - 3,20 (m, 1H), 3,49 (ddd, 1H, *J* = 14,4 Hz, 9,8 Hz et 2,1 Hz), 3,68 (ddd, 1H, *J* = 14,4 Hz, 5,0 Hz et 1,5 Hz), 4,61 - 4,66 (m, 1H), 6,46 (dd, 1H, *J* = 3,4 Hz et 1,8 Hz), 6,54 (d, 1H, *J* = 3,4 Hz), 7,21 (d, 1H, *J* = 3,8 Hz), 7,31 (d, 1H, *J* = 3,8 Hz), 7,41 - 7,42 (m, 1H), 7,73 (s, 1H) ; RMN ¹³C (100 MHz, CDCl₃) : *δ*(ppm) 20,1 (CH₂), 26,1 (CH₂), 28,3 (CH), 47,1 (CH₂), 47,4 (CH₂), 52,8 (CH₂), 58,7 (CH), 105,6 (CHₐᵣₒ), 112,0 (CHₐᵣₒ), 118,6 (CHₐᵣₒ), 123,1 (CHₐᵣₒ), 124,8 (CHₐᵣₒ), 131,8 (C_{q}), 133,4 (C_{q}), 142,0 (CHₐᵣₒ), 142,6 (C_{q}), 149,3 (C_{q}) ; HRMS (EI-MS) : calculée pour C₁₇H₁₉N₄OS m/z = 327,1280, trouvée m/z = 327,1284.

**(*R*)-(4-(5-(1-(quinuclidin-3-yl)-1*H*-1,2,3-triazol-4-yl)thiophèn-2-yl)phényl)méthanol (63).** Le produit est isolé sous la forme d'un solide blanc avec un rendement de 72% en suivant la procédure générale E1. R*_{f}*: 0,17 (CH₂Cl₂/MeOH/NH₄OH : 80/20/0,1) ; Mp : 253°C ; IR (ATR, Diamond) : *v* (cm⁻¹) : 983, 1041, 1214, 1306, 1355, 1416, 1454, 1502, 2823, 2872, 2941, 3110 ; RMN ¹H (400 MHz, DMSO-*d₆*) : *δ*(ppm) 1,38 - 1,52 (m, 2H), 1,72 - 1,80 (m, 2H), 2,20 - 2,26 (m, 1H), 2,76 - 2,85 (m, 3H), 2,96 - 3,05 (m, 1H), 3,34 - 3,51 (m, 2H), 4,56 (d, 2H, *J* = 4,8 Hz), 4,76 - 7,82 (m, 1H), 5,27 (t, 1H, *J* = 4,8 Hz), 7,41 (d, 2H, *J* = 7,8 Hz), 7,47 (d, 1H, *J =* 3,1 Hz), 7,54 (d, 1H, *J =* 3,1 Hz), 7,69 (d, 2H, *J* = 7,8 Hz), 8,72 (s, 1H) ; RMN ¹³C (100 MHz, DMSO-*d₆*) : *δ*(ppm) 19,6 (CH₂), 25,3 (CH₂), 27,6 (CH), 46,3 (CH₂), 46,6 (CH₂), 51,9 (CH₂), 57,6 (CH), 62,5 (CH₂), 120,3 (CHₐᵣₒ), 123,9 (CHₐᵣₒ), 124,9 (2CHₐᵣₒ), 125,1 (CHₐᵣₒ), 127,1 (2CHₐᵣₒ), 131,9 (C_{q}), 132,1 (C_{q}), 141,4 (C_{q}), 142,2 (2C_{q}) ; HRMS (EI-MS) : calculée pour C₂₀H₂₃N₄OS m/z = 367,1593, trouvée m/z = 367,1609.

**(R)-[4-[5-[1-[quinuclidin-3-yl]-1*H*-1,2,3-triazol-4-yl]-2-furyl]phenyl]méthanol (84).** Le produit est isolé sous la forme d'un solide blanc avec un rendement de 72% en suivant la procédure générale E1. R*_{f}*: 0,17 (CH₂Cl₂/MeOH/NH₄OH : 96/4/1) ; Mp : 209°C ; IR (ATR, Diamond) : *v* (cm⁻¹) : 792, 980, 1042, 1201, 1327, 1413, 1457, 1502, 2879, 2946, 3116 ; RMN ¹H (400 MHz, DMSO-*d₆*) : *δ*(ppm) 1.31 - 1.46 (m, 2H), 1.65 - 1.77 (m, 2H), 2.20 - 2.22 (m, 1H), 2.71 - 2.78 (m, 3H), 2.93 - 3.01 (m, 1H), 3.34 - 3.50 (m, 2H), 4.51 (d, 2H, *J* = 5.5 Hz), 4.72 - 4.81 (m, 1H), 5.23 (t, 1H, *J* = 5.5 Hz), 6.88 (d, 2H, *J* = 3.2 Hz), 7.02 (d, 1H, *J* = 3.2 Hz), 7.38 (d, 1H, *J* = 8.2 Hz), 7.74 (d, 2H, *J* = 8.2 Hz), 8.67 (s, 1H) ; RMN ¹³C (100 MHz, DMSO-*d₆*) : *δ*(ppm) 20.0 (CH₂), 25.7 (CH₂), 28.1 (CH), 46.8 (CH₂), 47.0 (CH₂), 52.1 (CH₂), 57.9 (CH), 63.0 (CH₂), 107.6 (CH), 108.7 (CH), 121.0 (CH), 123.6 (2CH), 127.3 (2CH), 128.9 (C_{q}), 139.3 (C_{q}), 142.5 (C_{q}), 146.0 (C_{q}), 152.9 (C_{q}) ; HRMS (EI-MS) : calculée pour C₂₀H₂₃N₄O₂ m/z = 351.18155, trouvée m/z = 351.18154.

**(R)-[3-[5-[1-[quinuclidin-3-yl]-1*H*-1,2,3-triazol-4-yl]-2-thiènyl]phényl]méthanol (85).** Le produit est isolé sous la forme d'un solide blanc avec un rendement de 75% en suivant la procédure générale E1. R*_{f}*: 0,17 (CH₂Cl₂/MeOH/NH₄OH : 98/2/1) ; Mp : 181°C ; IR (ATR, Diamond) : *v* (cm⁻¹) : 796, 988, 1040, 1226, 1323, 1452, 1488, 2870, 2938, 3108 ; RMN ¹H (400 MHz, DMSO-*d₆*) : *δ*(ppm) 1.35 - 1.50 (m, 2H), 1.66 - 1.81 (m, 2H), 2.20 - 2.22 (m, 1H), 2.72 - 2.87 (m, 3H), 2.93 - 3.05 (m, 1H), 3.34 - 3.51 (m, 2H), 4.57 (s, 2H), 4.74 - 4.81 (m, 1H), 5.33 (bl, 1H), 7.28 (d, 1H, *J* = 12.0 Hz), 7.39 (t, 1H, *J* = 12.0 Hz), 7.46 (d, 1H, *J* = 6.0 Hz), 7.53 (d, 1H, *J* = 6.0 Hz), 7.58 (d, 1H, *J* = 12.0 Hz), 7.65 (s, 1H), 8.70 (s, 1H) ; RMN ¹³C (100 MHz, DMSO-*d₆*) : *δ*(ppm) 20.0 (CH₂), 25.7 (CH₂), 28.0 (CH), 46.7 (CH₂), 47.0 (CH₂), 52.2 (CH₂), 57.9 (CH), 63.0 (CH₂), 120.8 (CH), 123.5 (CH), 123.9 (CH), 124.6 (CH), 125.6 (CH), 126.2 (CH), 129.3 (CH), 132.8 (C_{q}), 133.6 (C_{q}), 141.8 (C_{q}), 142.8 (C_{q}), 144.0 (C_{q}) ; HRMS (EI-MS) : calculée pour C₂₀H₂₃N₄OS m/z = 367.15871, trouvée m/z = 367.15904.

**(R)-[4-[5-[1-[quinuclidin-3-yl]-1*H*-1,2,3-triazol-4-yl]-3-thiènyl]phényl]méthanol (86).** Le produit est isolé sous la forme d'un solide blanc avec un rendement de 72% en suivant la procédure générale E1. R*_{f}*: 0,18 (CH₂Cl₂/MeOH/NH₄OH : 98/2/1) ; Mp : 196°C ; IR (ATR, Diamond): *v* (cm⁻¹) : 749, 785, 981, 1042, 1061, 1211, 1325, 1413, 1452, 1534, 2870, 2941, 3116 ; RMN ¹H (400 MHz, DMSO-*d₆*) : *δ* ppm) 1.34 - 1.50 (m, 2H), 1.66 - 1.78 (m, 2H), 2.19 (q, 1H, *J* = 2.8 Hz), 2.74 - 2.83 (m, 3H), 2.94 - 3.01 (m, 1H), 3.40 (d, 2H, *J =* 7.2 Hz), 4.51 (s, 2H), 4.75 - 4.79 (m, 1H), 5.20 (bl, 1H), 7.35 (d, 2H, *J* = 8.2 Hz), 7.68 (d, 2H, *J* = 8.2 Hz), 7.80 (d, 1H, *J* = 0.8 Hz), 7.89 (d, 1H, *J =* 0.8 Hz), 8.70 (s, 1H) ; RMN ¹³C (100 MHz, DMSO-*d₆*) : *δ*(ppm) 19.9 (CH₂), 25.6 (CH₂), 28.0 (CH), 46.7 (CH₂), 47.0 (CH₂), 52.3 (CH₂), 57.8 (CH), 63.0 (CH₂), 120.1 (CH), 120.7 (CH), 123.3 (CH), 126.0 (2CH), 127.4 (2CH), 133.7 (C_{q}), 134.3 (C_{q}), 142.0 (C_{q}), 142.1 (C_{q}), 142.2 (C_{q}) ; HRMS (EI-MS) : calculée pour C₂₀H₂₃N₄OS m/z = 367.15871, trouvée m/z = 367.15889.

**(R)-3-[4-[5-(6-fluoro-3-pyridyl)-2-thiènyl]-1*H*,1,2,3-triazol-1-yl]quinuclidine (87).** Le produit est isolé sous la forme d'un solide blanc avec un rendement de 80% en suivant la procédure générale E1. R*_{f}*: 0,20 (CH₂Cl₂/MeOH/NH₄OH : 98/2/1) ; Mp : 163°C ; IR (ATR, Diamond): *v* (cm⁻¹) : 784, 801, 986, 1077, 1241, 1320, 1387, 1447, 1526, 1581, 2871, 2939, 3127 ; RMN ¹H (400 MHz, CDCl₃) : *δ*(ppm) 1.46 - 1.52 (m, 1H), 1.64 - 1.71 (m, 1H), 1.74 - 1.87 (m, 2H), 2.28 (q, 1H, *J* = 2.8 Hz), 2.86 - 3.00 (m, 3H), 3.10 - 3.14 (m, 1H), 3.46 (ddd, 1H, *J* = 14.4 Hz, 10.0 Hz et 2.0 Hz), 3.66 (dd, 1H, *J* = 14.4 Hz, 4.0 Hz), 4.63 - 4.65 (m, 1H), 6.96 (dd, 1H, *J* = 8.4 Hz et 2.4 Hz), 7.26 (d, 1H, *J* = 3.8 Hz), 7.35 (d, 1H, *J* = 3.8 Hz), 7.78 (s, 1H), 7.97 (td, 1H, *J* = 8.4 Hz et 2.2 Hz), 8.46 (d, 1H, *J* = 2.2 Hz) ; RMN ¹³C (100 MHz, CDCl₃) : *δ*(ppm) 20.0 (CH₂), 25.9 (CH₂), 28.1 (CH), 46.9 (CH₂), 47.2 (CH₂), 52.6 (CH₂), 58.6 (CH), 109.7 (d, CH, *J* = 38 Hz), 118.6 (CH), 124.7 (CH), 124.9 (CH), 128.4 (d, C_{q}, *J* = 5 Hz), 133.6 (C_{q}), 138.2 (d, CH, *J* = 8 Hz), 138.3 (C_{q}), 142.1 (C_{q}), 144.3 (d, CH, *J* = 14 Hz), 162.8 (d, C_{q}, *J* = 239 Hz) ; HRMS (EI-MS) : calculée pour C₁₈H₁₉N₅S m/z = 356.13397, trouvée m/z = 356.13432.

**(R)-3-[4-[4-(6-fluoro-3-pyridyl)-2-thiènyl]-1*H*-1,2,3-triazol-1-yl]quinuclidine (88).** Le produit est isolé sous la forme d'un solide blanc avec un rendement de 79% en suivant la procédure générale E1. R*_{f}*: 0,20 (CH₂Cl₂/MeOH/NH₄OH : 98/2/1) ; Mp : 136°C ; IR (ATR, Diamond) : *v* (cm⁻¹) : 793, 972, 1055, 1219, 1310, 1402, 1459, 1589, 2868, 2938, 3115 ; RMN ¹H (400 MHz, CDCl₃) : *δ*(ppm) 1.46 - 1.53 (m, 1H), 1.65 - 1.73 (m, 1H), 1.77 - 1.87 (m, 2H), 2.30 (q, 1H, *J*= 2.8 Hz), 2.87 - 3.01 (m, 3H), 3.11 - 3.18 (m, 1H), 3.51 (ddd, 1H, *J* = 14.4 Hz, 10.0 Hz et 2.0 Hz), 3.70 (dd, 1H, *J* = 14.4 Hz et 4.0 Hz), 4.65 - 4.68 (m, 1H), 6.98 (dd, 1H, *J* = 8.4 Hz et 2.8 Hz), 7.41 (s, 1H), 7.63 (s, 1H), 7.81 (s, 1H), 7.98 (td, 1H, *J* = 8.4 Hz et 2.8 Hz), 8.46 (d, 1H, *J* = 1.6 Hz) ; RMN ¹³C (100 MHz, CDCl₃) : *δ*(ppm) 19.9 (CH₂), 25.9 (CH₂), 28.1 (CH), 46.9 (CH₂), 47.2 (CH₂), 52.6 (CH₂), 58.6 (CH), 109.6 (d, CH, *J* = 38 Hz), 118.7 (CH), 120.7 (CH), 122.5 (CH), 129.5 (d, C_{q}, *J* = 5 Hz), 134.7 (C_{q}), 138.0 (C_{q}), 138.8 (d, CH, *J* = 7 Hz), 142.1 (C_{q}), 145.0 (d, CH, *J* = 15 Hz), 162.8 (d, C_{q}, *J* = 238 Hz) ; HRMS (EI-MS) : calculée pour C₁₈H₁₉N₅SF m/z = 356.13397, trouvée m/z = 356.13430.

**(R)-[3-[5-[1-[quinuclidin-3-yl]-1*H*-1,2,3-triazol-1,2,3-triazol-4-yl]-3-thiènyl]phényl]méthanol (89).** Le produit est isolé sous la forme d'un solide blanc avec un rendement de 77% en suivant la procédure générale E1. R*_{f}*: 0,21 (CH₂Cl₂/MeOH/NH₄OH : 98/2/1) ; Mp : 211°C ; IR (ATR, Diamond) : *v* (cm⁻¹) : 787, 996, 1041, 1162, 1233, 1324, 1437, 1455, 1603, 2868, 2937, 3367; RMN ¹H (400 MHz, CDCl₃) : *δ*(ppm) 1.43 - 1.58 (m, 1H), 1.62 - 1.90 (m, 3H), 2.26 - 2.32 (m, 1H), 2.47 (bl, 1H), 2.83 - 3.01 (m, 3H), 3.09 - 3.19 (m, 1H), 3.46 (ddd, 1H, *J* = 15.6 Hz, 12.0 Hz et 3.6 Hz), 3.66 (dd, 1H, *J* = 15.6 Hz et 7.6 Hz), 4.60 - 4.69 (m, 1H), 4.76 (s, 2H), 7.32 (d, 1H, *J* = 12.2 Hz), 7.38 - 7.44 (m, 2H), 7.56 (dd, 1H, *J* = 12.2 Hz et 2.2 Hz), 7.66 (s, 1H), 7.70 (d, 1H, *J* = 2.2 Hz), 7.78 (s, 1H) ; RMN ¹³C (100 MHz, CDCl₃) : *δ*(ppm) 19.9 (CH₂), 25.8 (CH₂), 28.0 (CH), 46.8 (CH₂), 47.1 (CH₂), 52.5 (CH₂), 58.4 (CH), 65.0 (CH₂), 118.5 (CH), 119.9 (CH), 123.2 (CH), 124.8 (CH), 125.3 (CH), 125.8 (CH), 129.0 (CH), 133.7 (C_{q}), 135.7 (C_{q}), 141.8 (C_{q}), 142.5 (2C_{q}) ; HRMS (EI-MS) : calculée pour C₂₀H₂₃N₄OS m/z = 367.15871, trouvée m/z = 367.15909.

**(R)-[4-[5-[1-[quinuclidin-3-yl]-1*H*-1,2,3-triazol-1,2,3-triazol-4-yl]-3-furyl]phényl]méthanol (90).** Le produit est isolé sous la forme d'un solide blanc avec un rendement de 71% en suivant la procédure générale E1. R*_{f}*: 0,2 (CH₂Cl₂/MeOH/NH₄OH : 96/4/1) ; Mp : 202°C ; IR (ATR, Diamond) : *v* (cm⁻¹) : 795, 986, 1039, 1163, 1273, 1351, 1462, 1528, 1579, 2185, 2946 ; RMN ¹H (400 MHz, DMSO-*d₆*) : *δ*(ppm) 1.34 - 1.46 (m, 2H), 1.69 - 1.73 (m, 2H), 2.19 (q, 1H, *J* = 2.8 Hz), 2.74 - 2.83 (m, 3H), 2.94 - 3.01 (m, 1H), 3.35 - 3.51 (m, 3H), 4.49 (s, 2H), 4.76 - 4.80 (m, 1H), 7.250(s, 1H), 7.33 (d, 2H, *J* = 8.2 Hz), 7.62 (d, 2H, *J* = 8.2 Hz), 7.24 (s, 1H), 8.59 (s, 1H) ; RMN ¹³C (100 MHz, DMSO-*d₆*) : *δ*(ppm) 19.9 (CH₂), 25.5 (CH₂), 28.0 (CH), 46.7 (CH₂), 46.9 (CH₂), 52.0 (CH₂), 57.8 (CH), 63.1 (CH₂), 105.2 (CH), 121.3 (CH), 125.7 (2CH), 127.3 (2CH), 127.7 (C_{q}), 130.3 (C_{q}), 139.1 (CH), 139.2 (C_{q}), 141.9 (C_{q}), 147.5 (C_{q}) ; HRMS (EI-MS) : calculée pour C₂₀H₂₃N₄O₂ m/z = 351.18155, trouvée m/z = 351.18173.

**(R)-3-[4-[4-(6-fluoro-3-pyridyl)-2-furyl]-1*H*-1,2,3-triazol-1-yl]quinuclidine (91).** Le produit est isolé sous la forme d'un solide blanc avec un rendement de 73% en suivant la procédure générale E1. R*_{f}* : 0,22 (CH₂Cl₂/MeOH/NH₄OH : 96/4/1) ; Mp : 210°C ; IR (ATR, Diamond) : *v* (cm⁻¹) : 791, 986, 1058, 1247, 1310, 1418, 1491, 1564, 2872, 2944, 3091 ; RMN ¹H (400 MHz, CDCl₃) : *δ* (ppm) 1.45 - 1.53 (m, 1H), 1.62 - 1.68 (m, 1H), 1.76 - 1.89 (m, 2H), 2.29 (q, 1H, *J* = 3.2 Hz), 2.87 - 3.01 (m, 3H), 3.10 - 3.18 (m, 1H), 3.51 (ddd, 1H, *J* = 14.4 Hz, 10.0 Hz et 3.2 Hz), 3.70 (dd, 1H, *J* = 14.4 Hz et 4.8 Hz), 4.66 - 4.69 (m, 1H), 6.98 (dd, 1H, *J* = 8.4 Hz et 2.8 Hz), 7.13 (s, 1H), 7.74 (s, 1H), 7.85 (s, 1H), 7.90 (td, 1H, *J* = 8.4 Hz et 2.4 Hz), 8.40 (d, 1H, *J* = 2.4 Hz) ; RMN ¹³C (100 MHz, CDCl₃) : *δ* (ppm) 19.9 (CH₂), 25.9 (CH₂), 28.1 (CH), 46.9 (CH₂), 47.2 (CH₂), 52.7 (CH₂), 58.6 (CH), 105.0 (CH), 109.7 (d, CH, *J* = 38 Hz), 118.9 (CH), 124.0 (C_{q}), 126.1 (d, C_{q}, *J* = 4 Hz), 137.8 (CH), 138.4 (d, CH, *J* = 8 Hz), 139.7 (C_{q}), 144.6 (d, CH, *J* = 15 Hz), 148.0 (C_{q}), 162.8 (d, C_{q}, *J* = 238 Hz) ; HRMS (EI-MS) : calculée pour C₁₈H₁₉N₅FO m/z = 340.15681, trouvée m/z = 340.15696.

**(R)-[3-[5-[1-[quinuclidin-3-yl]-1*H*-1,2,3-triazol-4-yl]-3-furyl]phényl]méthanol (92).** Le produit est isolé sous la forme d'un solide blanc avec un rendement de 72% en suivant la procédure générale E1. R*_{f}* : 0,19 (CH₂Cl₂/MeOH/NH₄OH : 96/4/1) ; Mp : 212°C ; IR (ATR, Diamond) : *v* (cm⁻¹) : 798, 970, 1041, 1205, 1343, 1438, 1455, 1567, 2868, 2939, 3397 ; RMN ¹H (400 MHz, CDCl₃) : *δ* (ppm) 1.40 - 1.53 (m, 1H), 1.60 - 1.66 (m, 1H), 1.74 - 1.84 (m, 2H), 2.26 (q, 1H, *J* = 2.8 Hz), 2.82 - 2.96 (m, 3H), 3.03 - 3.10 (m, 2H), 3.44 (ddd, 1H, *J* = 15.6 Hz, 12.0 Hz et 3.6 Hz), 3.58 (dd, 1H, *J* = 15.6 Hz et 7.6 Hz), 4.62 - 4.64 (m, 1H), 4.71 (s, 2H), 7.15 (s, 1H), 7.28 (d, 1H, *J* = 7.6 Hz), 7.56 (t, 1H, *J* = 7.6 Hz), 7.43 (d, 1H, *J* = 7.6 Hz), 7.54 (s, 1H), 7.73 (s, 1H), 7.78 (s, 1H) ; RMN ¹³C (100 MHz, CDCl₃) : *δ* (ppm) 19.8 (CH₂), 25.8 (CH₂), 27.9 (CH), 46.7 (CH₂), 47.0 (CH₂), 52.4 (CH₂), 58.3 (CH), 64.8 (CH₂), 105.5 (CH), 118.7 (CH), 124.3 (CH), 124.8 (CH), 125.8 (CH), 128.0 (C_{q}), 129.0 (CH), 132.1 (C_{q}), 137.9 (CH), 140.0 (C_{q}), 142.0 (C_{q}), 147.2 (C_{q}) ; HRMS (EI-MS) : calculée pour C₂₀H₂₃N₄O₂ m/z = 351.18155, trouvée m/z = 351.18165.

**(R)-[4-[4-[1-[quinuclidin-3-yl]-1*H*-1,2,3-triazol-4-yl]phényl]phényl]méthanol (93).** Le produit est isolé sous la forme d'un solide blanc avec un rendement de 81% en suivant la procédure générale E1. R*_{f}* : 0,18 (CH₂Cl₂/MeOH/NH₄OH : 97/3/1) ; Mp : 250°C ; IR (ATR, Diamond) : *v* (cm⁻¹) : 798, 973, 1042, 1223, 1344, 1429, 1451, 1661, 2869, 2938, 3119 ; RMN ¹H (400 MHz, DMSO-*d₆*) : *δ* (ppm) 1.36 - 1.49 (m, 2H), 1.69 - 1.75 (m, 2H), 2.20 (q, 1H, *J* = 2.8 Hz), 2.73 - 2.80 (m, 3H), 2.96 - 3.03 (m, 1H), 3.35 - 3.49 (m, 2H), 4.54 (d, 2H, *J* = 3.2 Hz), 4.75 - 4.77 (m, 1H), 5.21 (bl, 1H), 7.40 (d, 2H, *J* = 8.2 Hz), 7.68 (d, 2H, *J* = 8.2 Hz), 7.76 (d, 2H, *J* = 8.4 Hz), 7.96 (d, 2H, *J* = 8.4 Hz), 8.78 (s, 1H) ; RMN ¹³C (100 MHz, DMSO-*d₆*) : *δ* (ppm) 20.0 (CH₂), 25.7 (CH₂), 28.1 (CH), 46.8 (CH₂), 47.0 (CH₂), 52.3 (CH₂), 57.8 (CH), 63.0 (CH₂), 121.3 (CH), 126.1 (2CH), 126.6 (2CH), 127.3 (2CH), 127.4 (2CH), 130.2 (C_{q}), 138.3 (C_{q}), 139.7 (C_{q}), 142.3 (C_{q}), 146.2 (C_{q}) ; HRMS (EI-MS) : calculée pour C₂₂H₂₅N₄O m/z = 361.20229, trouvée m/z = 361.20277.

**(R)-3-[4-[4-(6-fluoro-3-pyridyl)phényl]-1*H*-1,2,3-triazol-1-yl]quinuclidine (94).** Le produit est isolé sous la forme d'un solide jaune avec un rendement de 85% en suivant la procédure générale E1. R*_{f}* : 0,22 (CH₂Cl₂/MeOH/NH₄OH : 97/3/1) ; Mp : 181°C ; IR (ATR, Diamond) : *v* (cm⁻¹) : 811, 987, 1039, 1253, 1372, 1474, 1590, 2871, 2942, 3115 ; RMN ¹H (400 MHz, CDCl₃) : *δ* (ppm) 1.47 - 1.53 (m, 1H), 1.67 - 1.85 (m, 3H), 2.30 (q, 1H, *J* = 3.2 Hz), 2.88 - 3.02 (m, 3H), 3.13 - 3.20 (m, 1H), 3.51 (ddd, 1H, *J* = 14.2 Hz, 9.8 Hz et 2.2 Hz), 3.72 (dd, 1H, *J* = 14.2 Hz et 4.0 Hz), 4.66 - 4.69 (m, 1H), 7.01 (dd, 1H, *J* = 8.4 Hz et 2.8 Hz), 7.61 (d, 2H, *J* = 8.2 Hz), 7.88 (s, 1H), 7.95 (d, 2H, *J* = 8.4 Hz), 8.01 (td, 1H, *J* = 8.2 Hz et 2.4 Hz), 8.45 (d, 1H, *J* = 2.4 Hz) ; RMN ¹³C (100 MHz, CDCl₃) : *δ* (ppm) 20.0 (CH₂), 26.0 (CH₂), 28.1 (CH), 46.9 (CH₂), 47.3 (CH₂), 52.7 (CH₂), 58.5 (CH), 109.5 (d, CH, *J* = 37 Hz), 119.2 (CH), 126.3 (2CH), 127.4 (2CH), 130.6 (C_{q}), 134.2 (d, C_{q}, *J* = 4 Hz), 136.2 (C_{q}), 139.5 (d, CH, *J* = 8 Hz), 145.6 (d, CH, *J* = 15 Hz), 146.8 (C_{q}), 163.1 (d, C_{q}, *J* = 238 Hz) ; HRMS (EI-MS) : calculée pour C₂₀H₂₁N₅F m/z = 350.17755, trouvée m/z = 350.17782.

**(R)-[3-[4-[1-[quinuclidin-3-yl]-1*H*-1,2,3-triazol-4-yl]phényl]phényl]méthanol (95).** Le produit est isolé sous la forme d'un solide blanc avec un rendement de 80% en suivant la procédure générale E1. R*_{f}* : 0,18 (CH₂Cl₂/MeOH/NH₄OH : 97/3/1) ; Mp : 207°C ; IR (ATR, Diamond) : *v* (cm⁻¹) : 792, 982, 1038, 1225, 1324, 1437, 1455, 1603, 2874, 2941, 3367 ; RMN ¹H (400 MHz, DMSO-*d₆*) : *δ* (ppm) 1.32 - 1.52 (m, 2H), 1.67 - 1.74 (m, 2H), 2.20 (q, 1H, *J* = 2.8 Hz), 2.73 - 2.80 (m, 3H), 2.96 - 3.05 (m, 1H), 3.36 - 3.50 (m, 2H), 4.58 (s, 2H), 4.76 - 4.78 (m, 1H), 5.25 (bl, 1H), 7.32 (d, 1H, *J* = 7.2 Hz), 7.43 (t, 1H, *J* = 7.6 Hz), 7.58 (d, 1H, *J* = 7.6 Hz), 7.67 (s, 1H), 7.75 (d, 2H, *J* = 8.2 Hz), 7.97 (d, 2H, *J* = 8.2 Hz), 8.79 (s, 1H) ; RMN ¹³C (100 MHz, DMSO-*d₆*) : *δ* (ppm) 20.0 (CH₂), 25.7 (CH₂), 28.1 (CH), 46.8 (CH₂), 47.0 (CH₂), 52.2 (CH₂), 57.8 (CH), 63.3 (CH₂), 121.4 (CH), 124.9 (CH), 125.2 (CH), 126.1 (3CH), 127.4 (2CH), 129.1 (CH), 130.4 (C_{q}), 139.8 (C_{q}), 139.9 (C_{q}), 143.7 (C_{q}), 146.2 (C_{q}) ; HRMS (EI-MS) : calculée pour C₂₂H₂₅N₄O m/z = 361.20229, trouvée m/z = 361.20262.

**(R)-[4-[3-[1-[quinuclidin-3-yl]-1*H*-1,2,3-triazol-4-yl]phényl]phényl]méthanol (98).** Le produit est isolé sous la forme d'un solide blanc avec un rendement de 87% en suivant la procédure générale E1. R*_{f}* : 0,18 (CH₂Cl₂/MeOH/NH₄OH : 97/3/1) ; Mp : 214°C ; IR (ATR, Diamond) : *v* (cm⁻¹) : 793, 972, 1041, 1222, 1320, 1451, 1481, 1660, 2868, 2939, 3077 ; RMN ¹H (400 MHz, DMSO-*d₆*) : *δ* (ppm) 1.31 - 1.49 (m, 2H), 1.70 - 1.74 (m, 2H), 2.20 (q, 1H, *J* = 2.8 Hz), 2.74 - 2.78 (m, 3H), 2.94 - 3.01 (m, 1H), 3.38 - 3.46 (m, 2H), 4.55 (s, 2H), 4.74 - 4.78 (m, 1H), 5.23 (bl, 1H), 7.42 (d, 2H, *J* = 7.6 Hz), 7.51 (t, 1H, *J* = 7.6 Hz), 7.61 (d, 1H, *J* = 7.6 Hz), 7.69 (d, 2H, *J* = 7.6 Hz), 7.87 (d, 1H, *J* = 7.6 Hz), 8.14 (s, 1H), 8.85 (s, 1H) ; RMN ¹³C (100 MHz, DMSO-*d₆*) : *δ* (ppm) 20.0 (CH₂), 25.7 (CH₂), 28.1 (CH), 46.8 (CH₂), 47.0 (CH₂), 52.3 (CH₂), 57.8 (CH), 63.0 (CH₂), 121.5 (CH), 123.7 (CH), 124.3 (CH), 126.3 (CH), 126.8 (2CH), 127.4 (2CH), 129.9 (CH), 131.9 (C_{q}), 138.6 (C_{q}), 141.0 (C_{q}), 142.5 (C_{q}), 146.5 (C_{q}) ; HRMS (EI-MS) : calculée pour C₂₂H₂₅N₄O m/z = 361.20229, trouvée m/z = 361.20237.

**(R)-[3-[3-[1-[quinuclidin-3-yl]-1*H*-1,2,3-triazol-4-yl]phényl]phényl]méthanol (99).** Le produit est isolé sous la forme d'un solide blanc avec un rendement de 80% en suivant la procédure générale E1. R*_{f}* : 0,18 (CH₂Cl₂/MeOH/NH₄OH : 97/3/1) ; Mp : 201°C ; IR (ATR, Diamond) : *v* (cm⁻¹) : 793, 984, 1042, 1262, 1326, 1423, 1436, 1612, 1697, 1719, 2870, 2943, 3133 ; RMN ¹H (400 MHz, DMSO-*d₆*) : *δ* (ppm) 1.34 - 1.48 (m, 2H), 1.69 - 1.75 (m, 2H), 2.20 (q, 1H, *J* = 2.8 Hz), 2.75 - 2.79 (m, 3H), 2.94 - 3.01 (m, 1H), 3.35 - 3.48 (m, 2H), 4.58 (d, 2H, *J* = 5.6 Hz), 4.72 - 4.79 (m, 1H), 5.25 (d, 1H, *J* = 5.6 Hz), 7.34 (d, 1H, *J* = 7.6 Hz), 7.44 (t, 1H, *J* = 7.6 Hz), 7.51 - 7.63 (m, 3H), 7.66 (s, 1H), 7.87 (d, 1H, *J* = 7.6 Hz), 8.14 (s, 1H), 8.85 (s, 1H) ; RMN ¹³C (100 MHz, DMSO-*d₆*) : *δ* (ppm) 20.0 (CH₂), 25.7 (CH₂), 28.1 (CH), 46.8 (CH₂), 47.0 (CH₂), 52.2 (CH₂), 57.8 (CH), 63.3 (CH₂), 121.5 (CH), 123.8 (CH), 124.5 (CH), 125.2 (CH), 125.5 (CH), 126.2 (CH), 126.5 (CH), 129.1 (CH), 129.9 (CH), 131.9 (C_{q}), 140.1 (C_{q}), 141.3 (C_{q}), 143.7 (C_{q}), 146.5 (C_{q}) ; HRMS (EI-MS) : calculée pour C₂₂H₂₅N₄O m/z = 361.20229, trouvée m/z = 361.20245.

**(R)-3-[4-[3-(6-fluoro-3-pyridyl)phényl]-1*H*-1,2,3-triazol-1-yl]quinuclidine (100).** Le produit est isolé sous la forme d'un solide blanc avec un rendement de 82% en suivant la procédure générale E1. R*_{f}* : 0,23 (CH₂Cl₂/MeOH/NH₄OH : 97/3/1) ; Mp : 164°C ; IR (ATR, Diamond) : *v* (cm⁻¹) : 789, 980, 1061, 1208, 1344, 1454, 1473, 1590, 2865, 2940, 3059 ; RMN ¹H (400 MHz, CDCl₃) : *δ* (ppm) 1.42 - 1.55 (m, 1H), 1.65 - 1.70 (m, 1H), 1.75 - 1.87 (m, 2H), 2.30 (q, 1H, *J* = 3.2 Hz), 2.88 - 2.97 (m, 3H), 3.12 - 3.19 (m, 1H), 3.51 (ddd, 1H, *J* = 14.4 Hz, 9.8 Hz et 2.2 Hz), 3.72 (dd, 1H, *J* = 14.4 Hz et 4.0 Hz), 4.66 - 4.68 (m, 1H), 7.01 (dd, 1H, *J* = 8.4 Hz et 2.8 Hz), 7.48 - 7.54 (m, 2H), 7.82 (d, 1H, *J* = 7.2 Hz), 7.89 (s, 1H), 8.01 - 8.06 (m, 2H), 8.46 (d, 1H, *J* = 2.0 Hz) ; RMN ¹³C (100 MHz, CDCl₃) : *δ* (ppm) 20.0 (CH₂), 25.9 (CH₂), 28.1 (CH), 46.9 (CH₂), 47.2 (CH₂), 52.6 (CH₂), 58.5 (CH), 109.4 (d, CH, *J* = 37 Hz), 119.2 (CH), 124.3 (CH), 125.3 (CH), 126.7 (CH), 129.6 (CH), 131.6 (C_{q}), 134.5 (d, C_{q}, *J* = 4 Hz), 137.4 (C_{q}), 139.8 (d, CH, *J* = 8 Hz), 145.8 (d, CH, *J* = 15 Hz), 147.0 (C_{q}), 163.2 (d, C_{q}, *J* = 238 Hz) ; HRMS (EI-MS) : calculée pour C₂₀H₂₁N₅F m/z = 350.17755, trouvée m/z = 350.17766.

**(R)-3-[4-(5-phenyl-2-thiènyl)-1H-1,2,3-triazol-1-yl]quinuclidine (106).** Le produit est isolé sous la forme d'un solide blanc avec un rendement de 80% en suivant la procédure générale E1. R*_{f}* : 0,25 (CH₂Cl₂/MeOH/NH₄OH : 98/2/1) ; Mp : 184°C ; IR (ATR, Diamond) : *v* (cm⁻¹) : 789, 982, 1060, 1209, 1323, 1404, 1455, 1498, 1590, 2866, 2940, 3059 ; RMN ¹H (400 MHz, CDCl₃) : *δ* (ppm) 1.43 - 1.55 (m, 1H), 1.62 - 1.80 (m, 3H), 2.28 (q, 1H, *J* = 2.8 Hz), 2.85 - 3.03 (m, 3H), 3.12 - 3.19 (m, 1H), 3.50 (ddd, 1H, *J* = 14.4 Hz, 10.0 Hz et 2.8 Hz), 4.70 (dd, 1H, *J* = 14.4 Hz et 4.0 Hz), 4.62 - 4.65 (m, 1H), 7.27 - 7.32 (m, 2H), 7.35 - 7.41 (m, 3H), 7.63 (t, 2H, *J* = 7.6 Hz), 7.75 (s, 1H) ; RMN ¹³C (100 MHz, CDCl₃) : *δ* (ppm) 20.0 (CH₂), 26.0 (CH₂), 28.1 (CH), 46.9 (CH₂), 47.2 (CH₂), 52.6 (CH₂), 58.3 (CH), 118.4 (CH), 123.5 (CH), 124.9 (CH), 125.6 (2CH), 127.6 (CH), 128.9 (2CH), 132.2 (C_{q}), 134.0 (C_{q}), 142.6 (C_{q}), 143.8 (C_{q}) ; HRMS (EI-MS) : calculée pour C₁₉H₂₁N₄S m/z = 337.148144, trouvée m/z = 337.148472.

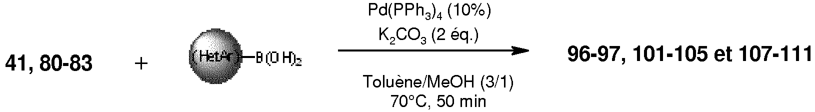

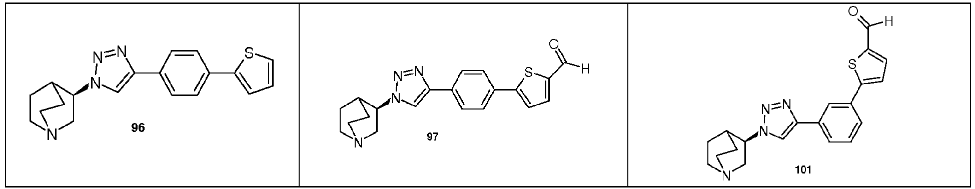

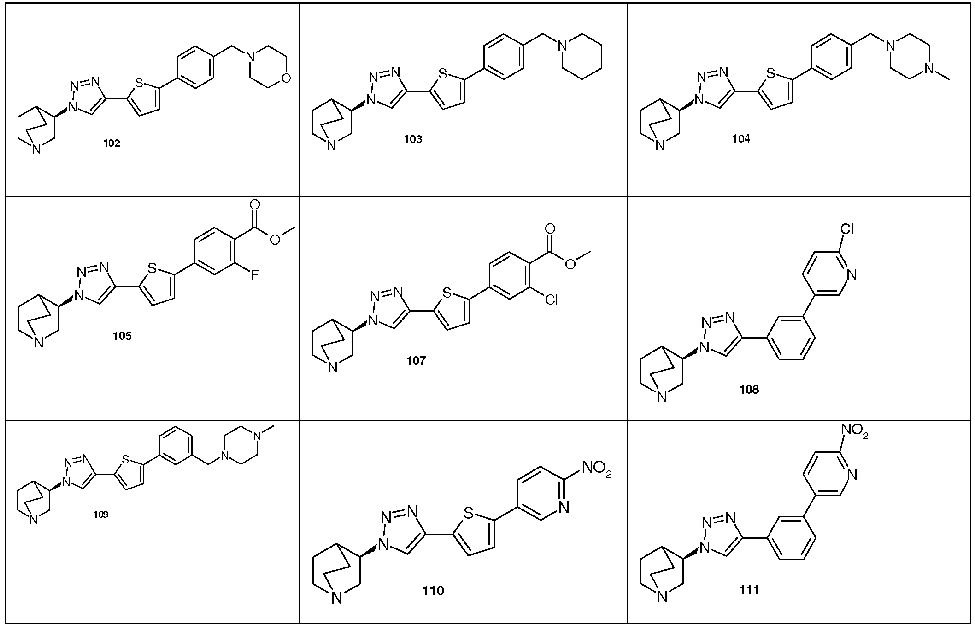

**Procédure générale E2** : A une solution de dérivé bromé (**composé 41, 54, 80, 81,82 ou 83**) (1,0 mmol) dans un mélange de 3 mL de toluène et 1 mL de méthanol sont ajoutés l'acide boronique souhaité (1,2 mmol), du K₂CO₃ (2,0 mmol) et du [1,1-bis(diph »nylphosphino)ferrocène]dichloropalladium (II) PdCl₂(dppf) (0,01 mmol). Après dégazage, la réaction est irradiée par micro-onde à 70°C pendant 50 minutes. Le solvant est ensuite évaporé puis le résidu est purifié par chromatographie sur colonne de gel de silice en utilisant comme éluant un mélange CH₂Cl₂/MeOH/NH₄OH.

**(R)-3-[4-[4-(2-thiènyl)phényl]-1*H*-1,2,3-triazol-1-yl]quinuclidine (96).** Le produit est isolé sous la forme d'un solide blanc avec un rendement de 86% en suivant la procédure générale E2. R*_{f}* : 0,17 (CH₂Cl₂/MeOH/NH₄OH : 97/3/1) ; Mp : 214°C ; IR (ATR, Diamond) : *v* (cm⁻¹) : 791, 988, 1042, 1222, 1314, 1404, 1450, 1493, 2867, 2937, 3120 ; RMN ¹H (400 MHz, CDCl₃) : *δ* (ppm) 1.45 - 1.54 (m, 1H), 1.65 - 1.83 (m, 3H), 2.29 (q, 1H, *J* = 2.8 Hz), 2.87 - 3.01 (m, 3H), 3.12 - 3.20 (m, 1H), 3.50 (ddd, 1H, *J* = 14.4 Hz, 10.0 Hz et 3.2 Hz), 3.72 (dd, 1H, *J* = 14.4 Hz et 3.2 Hz), 4.64 - 4.66 (m, 1H), 7.10 (dd, 1H, *J* = 5.0 Hz et 3.6 Hz), 7.29 (dd, 1H, *J* = 5.0 Hz et 1.2 Hz), 7.33 - 7.37 (m, 1H), 7.68 (d, 2H, *J* = 8.4 Hz), 7.83 - 7.87 (m, 3H) ; RMN ¹³C (100 MHz, CDCl₃) : *δ* (ppm) 20.0 (CH₂), 26.0 (CH₂), 28.1 (CH), 46.9 (CH₂), 47.3 (CH₂), 52.6 (CH₂), 58.4 (CH), 118.9 (CH), 123.2 (CH), 124.9 (CH), 126.0 (2CH), 126.2 (2CH), 128.1 (CH), 129.6 (C_{q}), 134.1 (C_{q}), 143.9 (C_{q}), 147.1 (C_{q}) ; HRMS (EI-MS) : calculée pour C₁₉H₂₁N₄S m/z = 337.14814, trouvée m/z = 337.14848.

**(R)-5-[4-[1-[quinuclidin-3-yl]-1*H*-1,2,3-triazol-4-yl]phényl]thiophène-2-carbaldéhyde (97).** Le produit est isolé sous la forme d'un solide blanc avec un rendement de 83% en suivant la procédure générale E2. R*_{f}* : 0,19 (CH₂Cl₂/MeOH/NH₄OH : 97/3/1) ; Mp : 228°C ; IR (ATR, Diamond) : *v* (cm⁻¹) : 806, 972, 1041, 1223, 1315, 1404, 1450, 1660, 2868, 2938, 3117 ; RMN ¹H (400 MHz, CDCl₃) : *δ* (ppm) 1.42 - 1.54 (m, 1H), 1.65 - 1.74 (m, 1H), 1.77 - 1.88 (m, 2H), 2.29 (q, 1H, *J* = 3.2 Hz), 2.86 - 3.00 (m, 3H), 3.11 - 3.18 (m, 1H), 3.50 (ddd, 1H, *J* = 14.4 Hz, 10.0 Hz et 3.6 Hz), 3.71 (dd, 1H, *J* = 14.4 Hz et 3.6 Hz), 4.64 - 4.67 (m, 1H), 7.42 (d, 1H, *J* = 4.0 Hz), 7.71 - 7.74 (m, 3H), 7.88 - 7.91 (m, 3H), 9.88 (s, 1H) ; RMN ¹³C (100 MHz, CDCl₃) : *δ* (ppm) 20.0 (CH₂), 26.0 (CH₂), 28.1 (CH), 46.9 (CH₂), 47.2 (CH₂), 52.6 (CH₂), 58.5 (CH), 119.4 (CH), 124.1 (CH), 126.2 (2CH), 126.8 (2CH), 131.7 (C_{q}), 132.6 (C_{q}), 137.4 (CH), 142.4 (C_{q}), 146.6 (C_{q}), 153.6 (C_{q}), 182.7 (CH) ; HRMS (EI-MS) : calculée pour C₂₀H₂₁N₄SO m/z = 365.14306, trouvée m/z = 365.14334.

**(R)-5-[3-[1-[quinuclidin-3-yl]-1*H*-1,2,3-triazol-4-yl]phényl]thiophène-2-carbaldéhyde (101).** Le produit est isolé sous la forme d'un solide blanc avec un rendement de 80% en suivant la procédure générale E2. R*_{f}* : 0,24 (CH₂Cl₂/MeOH/NH₄OH : 97/3/1) ; Mp : 150°C ; IR (ATR, Diamond) : *v* (cm⁻¹) : 792, 986, 1070, 1223, 1316, 1405, 1450, 1659, 2868, 2938, 3120 ; RMN ¹H (400 MHz, CDCl₃) : *δ* (ppm) 1.46 - 1.56 (m, 1H), 1.66 - 1.90 (m, 3H), 2.29 (q, 1H, *J* = 2.8 Hz), 2.88 - 3.02 (m, 3H), 3.13 - 3.20 (m, 1H), 3.50 (ddd, 1H, *J* = 14.4 Hz, 10.0 Hz et 3.6 Hz), 3.71 (dd, 1H, *J* = 14.4 Hz et 4.2 Hz), 4.67 - 4.69 (m, 1H), 7.47 - 7.51 (m, 2H), 7.63 (d, 1H, *J* = 7.2 Hz), 7.76 (d, 1H, *J* = 4.2 Hz), 7.85 (d, 1H, *J* = 8.0 Hz), 7.91 (s, 1H), 8.17 (s, 1H), 9.91 (s, 1H) ; RMN ¹³C (100 MHz, CDCl₃) : *δ* (ppm) 20.0 (CH₂), 25.9 (CH₂), 28.1 (CH), 46.9 (CH₂), 47.2 (CH₂), 52.6 (CH₂), 58.5 (CH), 119.4 (CH), 123.5 (CH), 124.5 (CH), 126.0 (CH), 126.5 (CH), 129.7 (CH), 131.7 (C_{q}), 133.6 (C_{q}), 137.3 (CH), 142.6 (C_{q}), 146.7 (C_{q}), 153.7 (C_{q}), 182.7 (CH) ; HRMS (EI-MS) : calculée pour C₂₀H₂₁N₄SO m/z = 365.14306, trouvée m/z = 365.14324.

**(R)-4-[4-[5-[1-[quinuclidin-3-yl]-1*H*-1,2,3-triazol-4yl]-2-thiènyl]phényl]méthyl]morpholine (102).** Le produit est isolé sous la forme d'un solide blanc avec un rendement de 83% en suivant la procédure générale E2. R*_{f}* : 0,21 (CH₂Cl₂/MeOH/NH₄OH : 97/3/1) ; Mp : 222°C ; IR (ATR, Diamond) : *v* (cm⁻¹) : 791, 987, 1040, 1220, 1345, 1451, 1498, 1662, 2803, 2868, 2938 ; RMN ¹H (400 MHz, CDCl₃) : *δ* (ppm) 1.41 - 1.53 (m, 1H), 1.62 - 1.86 (m, 3H), 2.27 (q, 1H, *J* = 2.8 Hz,), 2.45 - 2.28 (m, 4H), 2.28 - 3.00 (m, 3H), 3.10 - 3.19 (m, 1H), 3.46 - 3.50 (m, 3H), 3.66 - 3.72 (m, 5H), 4.61 - 4.64 (m, 1H), 7.27 (d, 1H, *J* = 8.0 Hz), 7.33 - 7.36 (m, 3H), 7.57 (d, 2H, *J* = 8.0 Hz), 7.73 (s, 1H) ; RMN ¹³C (100 MHz, CDCl₃) : *δ* (ppm) 20.0 (CH₂), 26.0 (CH₂), 28.1 (CH), 46.9 (CH₂), 47.2 (CH₂), 52.6 (CH₂), 53.6 (2CH₂), 58.5 (CH), 63.0 (CH₂), 37.0 (2CH₂), 118.3 (CH), 123.4 (CH), 124.9 (CH), 125.5 (2CH), 129.7 (2CH), 132.0 (C_{q}), 133.0 (C_{q}), 137.4 (C_{q}), 142.6 (Cq), 143.6 (C_{q}) ; HRMS (EI-MS) : calculée pour C₂₄H₃₀N₅OS m/z = 436.216558, trouvée m/z = 436.216563.

**(R)-3-[4-[5-[4-(1-pipéridylmethyl)phényl]-2-thiènyl]-1H-1,2,3-triazol-1-yl] quinuclidine (103).** Le produit est isolé sous la forme d'un solide blanc avec un rendement de 80% en suivant la procédure générale E2. R*_{f}* : 0,21 (CH₂Cl₂/MeOH/NH₄OH : 97/3/1) ; Mp : 230°C ; IR (ATR, Diamond) : *v* (cm⁻¹) : 792, 995, 1103, 1215, 1366, 1415, 1451, 1662, 2866, 2934, 3120 ; RMN ¹H (400 MHz, CDCl₃) : *δ* (ppm) 1.38 - 1.52 (m, 3H), 1.56 - 1.62 (m, 4H), 1.64 - 1.88 (m, 3H), 2.28 (q, 1H, *J* = 2.8 Hz), 2.32 - 2.49 (m, 4H), 2.86 - 3.01 (m, 3H), 3.11 - 3.18 (m, 1H), 3.45 - 3.52 (m, 3H), 3.69 (dd, 1H, *J* = 14.4 Hz et 5.2 Hz), 4.62 - 4.64 (m, 1H), 7.27 (d, 1H, *J* = 8.0 Hz), 7.33 - 7.35 (m, 3H), 7.57 (d, 2H, *J* = 8.0 Hz), 7.74 (s, 1H) ; RMN ¹³C (100 MHz, CDCl₃) : *δ* (ppm) 20.0 (CH₂), 24.3 (CH₂), 25.9 (2CH₂), 26.0 (CH₂), 28.1 (CH), 46.9 (CH₂), 47.2 (CH₂), 52.6 (CH₂), 54.5 (2CH₂), 58.5 (CH), 63.4 (CH₂), 118.3 (CH), 123.2 (CH), 124.9 (CH), 125.4 (2CH), 129.7 (2CH), 131.8 (C_{q}), 132.6 (C_{q}), 138.3 (C_{q}), 142.6 (C_{q}), 143.8 (C_{q}) ; HRMS (EI-MS) : calculée pour C₂₅H₃₂N₅S m/z = 434.237293, trouvée m/z = 434.237451.

**(R)-3-[4-[5-[4-[(4-méthylpipérazin-1-yl)méthyl]phényl]-2-thiènyl]-1H-1,2,3-triazol-1-yl] quinuclidine (104).** Le produit est isolé sous la forme d'un solide blanc avec un rendement de 75% en suivant la procédure générale E2. R*_{f}* : 0,20 (CH₂Cl₂/MeOH/NH₄OH : 97/3/1) ; Mp : 214°C ; IR (ATR, Diamond) : *v* (cm⁻¹) : 792, 973, 1040, 1222, 1347, 1450, 1661, 2869, 2939, 3120 ; RMN ¹H (400 MHz, CDCl₃) : *δ* (ppm) 1.42 - 1.53 (m, 1H), 1.62 - 1.85 (m, 3H), 2.27 - 2.70 (m, 12H), 2.84 - 3.00 (m, 3H), 3.10 - 3.18 (m, 1H), 3.45 - 3.52 (m, 3H), 3.69 (dd, 1H, *J* = 14.4 Hz et 5.2 Hz), 4.61 - 4.64 (m, 1H), 7.27 (d, 1H, *J* = 8.0 Hz), 7.33 - 7.35 (m, 3H), 7.57 (d, 2H, *J* = 8.0 Hz), 7.74 (s, 1H) ; RMN ¹³C (100 MHz, CDCl₃) : *δ* (ppm) 20.0 (CH₂), 26.0 (CH₂), 28.1 (CH), 46.0 (CH₃), 46.9 (CH₂), 47.2 (CH₂), 52.6 (2CH₂), 53.1 (2CH₂), 55.1 (CH₂), 58.5 (CH), 62.6 (CH₂), 118.3 (CH), 123.3 (CH), 124.9 (CH), 125.5 (2CH), 129.7 (2CH), 131.9 (C_{q}), 132.8 (C_{q}), 137.9 (C_{q}), 142.6 (Cq), 143.7 (C_{q}) ; HRMS (EI-MS) : calculée pour C₂₅H₃₃N₆S m/z = 449.248193, trouvée m/z = 449.248404.

**Méthyl-2-fluoro-4-[5-[1-[(3R)-quinuclidin-3-yl]-1H-1,2,3-triazol-4-yl]-2-thiènyl]benzoate (105).** Le produit est isolé sous la forme d'un solide blanc avec un rendement de 85% en suivant la procédure générale E2. R*_{f}* : 0,20 (CH₂Cl₂/MeOH/NH₄OH : 97/3/1) ; Mp : 250°C ; IR (ATR, Diamond) : *v* (cm⁻¹) : 785, 926, 1090, 1262, 1326, 1422, 1472, 1612, 1703, 1718, 2870, 2943, 3133 ; RMN ¹H (250 MHz, CDCl₃) : *δ* (ppm) 1.30 - 1.52 (m, 2H), 1.63 - 1.81 (m, 2H), 2.20 (q, 1H, *J* = 2.8 Hz), 2.71 - 3.03 (m, 4H), 3.33 - 3.50 (m, 2H), 3.87 (s, 3H), 4.71 - 4.83 (m, 1H), 7.51 (d, 1H, *J* = 3.5 Hz), 7.62 (d, 1H, *J* = 8.0 Hz), 7.70 - 7.81 (m, 2H), 7.93 (t, 1H, *J* = 8.0 Hz), 8.76 (s, 1H) ; RMN ¹³C (62.5 MHz, CDCl₃) : *δ* (ppm) 19.6 (CH₂), 25.4 (CH₂), 27.9 (CH), 46.5 (CH₂), 46.8 (CH₂), 51.8 (CH₂), 52.3 (CH₃), 58.3 (CH), 113.2 (d, CH, *J* = 38 Hz), 116.7 (d, C_{q}, *J* = 10 Hz), 119.5 (CH), 120.9 (d, CH, *J* = 6 Hz), 125.5 (CH), 125.9 (CH), 132.8 (CH), 134.1 (C_{q}), 140.4 (d, C_{q}, *J* = 15 Hz), 140.9 (d, C_{q}, *J* = 3 Hz), 142.2 (C_{q}), 162.3 (d, C_{q}, *J* = 258 Hz), 164.7 (d, C_{q}, *J* = 4 Hz) ; HRMS (EI-MS) : calculée pour C₂₁H₂₂N₄O₂FS m/z = 413.144202, trouvée m/z = 413.144364.

**Méthyl-2-chloro-4-[5-[1-[(3R)-quinuclidin-3-yl]-1H-1,2,3-triazol-4-yl]-2-thiènyl]benzoate (107).** Le produit est isolé sous la forme d'un solide blanc avec un rendement de 79% en suivant la procédure générale E2. R*_{f}* : 0,20 (CH₂Cl₂/MeOH/NH₄OH : 97/3/1) ; Mp : 200°C ; IR (ATR, Diamond) : *v* (cm⁻¹) : 792, 988, 1028, 1131, 1283, 1433, 1446, 1594, 1701, 2867, 2936, 3134 ; RMN ¹H (400 MHz, CDCl₃) : *δ* (ppm) 1.43 - 1.55 (m, 1H), 1.62 - 1.88 (m, 3H), 2.28 (q, 1H, *J* = 2.8 Hz), 2.87 - 3.00 (m, 3H), 3.08 - 3.20 (m, 1H), 3.48 (ddd, 1H, *J* = 14.4 Hz, 10.0 Hz et 2.8 Hz), 3.69 (dd, 1H, *J* = 14.4 Hz et 4.2 Hz), 3.92 (s, 3H), 4.64 - 4.67 (m, 1H), 7.36 (s, 2H), 7.51 (dd, 1H, *J* = 8.0 Hz et 1.2 Hz), 7.67 (s, 1H), 7.78 (s, 1H), 7.87 (d, 1H, *J* = 8.0 Hz) ; RMN ¹³C (100 MHz, CDCl₃) : *δ* (ppm) 19.9 (CH₂), 25.8 (CH₂), 28.1 (CH), 46.9 (CH₂), 47.2 (CH₂), 52.4 (CH₃), 52.5 (CH₂), 58.5 (CH), 118.8 (CH), 123.3 (CH), 125.1 (CH), 125.6 (CH), 127.6 (CH), 127.8 (C_{q}), 132.3 (CH), 134.4 (C_{q}), 134.7 (C_{q}), 138.4 (C_{q}), 140.5 (C_{q}), 142.1 (C_{q}), 165.5 (C_{q}) ; HRMS (EI-MS) : calculée pour C₂₁H₂₂N₄ClO₂S m/z = 429.114651, trouvée m/z = 429.114907.

**(R)-3-[4-[3-(6-chloro-3-pyridyl)phényl]-1H-1,2,3-triazol-1-yl]quinuclidine (108).** Le produit est isolé sous la forme d'un solide blanc avec un rendement de 20% en suivant la procédure générale E2. R*_{f}* : 0,17 (CH₂Cl₂/MeOH/NH₄OH : 98/2/1) ; Mp(decom) : 80°C ; IR (ATR, Diamond) : *v* (cm⁻¹) : 831, 987, 1039, 1233, 1343, 1403, 1455, 1581, 2865, 2938, 3053 ; RMN ¹H (400 MHz, CDCl₃) : *δ* (ppm) 1.43 - 1.56 (m, 1H), 1.66 - 1.70 (m, 1H), 1.77 - 1.90 (m, 2H), 2.31 (q, 1H, *J* = 3.0 Hz), 2.88 - 3.02 (m, 3H), 3.11 - 3.21 (m, 1H), 3.51 (ddd, 1H, *J* = 14.4 Hz, 9.8 Hz et 2.2 Hz), 3.72 (dd, 1H, *J* = 14.4 Hz et 3.6 Hz), 4.67 - 4.69 (m, 1H), 7.42 (d, 1H, *J* = 8.4 Hz), 7.52 - 7.56 (m, 2H), 7.82 (dt, 1H, *J* = 8.4 Hz et 1.2 Hz), 7.90 - 7.93 (m, 2H), 8.08 (s, 1H), 8.66 (d, 1H, *J* = 2.4 Hz) ; RMN ¹³C (100 MHz, CDCl₃) : *δ* (ppm) 20.0 (CH₂), 25.9 (CH₂), 28.1 (CH), 46.9 (CH₂), 47.2 (CH₂), 52.6 (CH₂), 58.5 (CH), 119.3 (CH), 124.2 (CH), 124.3 (CH), 125.6 (CH), 126.7 (CH), 129.7 (CH), 131.6 (C_{q}), 135.3 (C_{q}), 137.1 (Cq), 137.2 (CH), 146.9 (C_{q}), 147.9 (CH), 150.5 (C_{q}) ; HRMS (EI-MS) : calculée pour C₂₀H₂₁N₅Cl m/z = 366.1480, trouvée m/z = 366.148142.

**(R)-3-[4-[5-[3-[(4-méthylpiperazin-1-yl)méthyl]phényl]-2-thienyl]-1H-1,2,3-triazol-1-yl] quinuclidine (109).** Le produit est isolé sous la forme d'un solide blanc avec un rendement de 83% en suivant la procédure générale E2. R*_{f}* : 0,20 (CH₂Cl₂/MeOH/NH₄OH : 97/3/1) ; Mp : 155°C ; IR (ATR, Diamond) : *v* (cm⁻¹) : 788, 923, 1013, 1279, 1347, 1453, 1506, 2793, 2870, 2938, 3306 ; RMN ¹H (400 MHz, CDCl₃) : *δ* (ppm) 1.42 - 1.60 (m, 1H), 1.65 - 1.92 (m, 3H), 2.24 - 2.32 (m, 4H), 2.37 - 2.73 (m, 8H), 2.86 - 2.99 (m, 3H), 3.08 - 3.22 (m, 1H), 3.45 - 3.56 (m, 3H), 3.70 (dd, 1H, *J* = 14.5 Hz et 5.2 Hz), 4.60 - 4.70 (m, 1H), 7.26 - 7.38 (m, 4H), 7.54 (d, 1H, *J* = 7.5 Hz), 7.61 (m, 1H), 7.76 (s, 1H) ; RMN ¹³C (100 MHz, CDCl₃) : *δ* (ppm) 20.0 (CH₂), 26.0 (CH₂), 28.1 (CH), 46.0 (CH₃), 46.9 (CH₂), 47.2 (CH₂), 52.6 (CH₂), 53.1 (2CH₂), 55.1 (2CH), 58.5 (CH), 62.8 (CH₂), 118.3 (CH), 123.6 (CH), 124.4 (CH), 124.8 (CH), 126.3 (CH), 128.4 (CH), 128.8 (CH), 132.1 (C_{q}), 133.9 (C_{q}), 139.1 (C_{q}), 142.6 (C_{q}), 143.8 (C_{q}) ; HRMS (EI-MS) : calculée pour C₂₅H₃₃N₆S m/z = 449.248193, trouvée m/z = 449.247898

**(R)-3-[4-[5-(6-nitro-3-pyridyl)-2-thienyl]-1H-1,2,3-triazol-1-yl]quinuclidine (110).** Le produit est isolé sous la forme d'un solide blanc avec un rendement de 77% en suivant la procédure générale E2. R*_{f}* : 0,19 (CH₂Cl₂/MeOH/NH₄OH : 98/2/1) ; Mp : 160°C ; IR (ATR, Diamond) : *v* (cm⁻¹) : 788, 987, 1013, 1216, 1278, 1347, 1413, 1453, 1528, 2794, 2938, 3307 ; RMN ¹H (250 MHz, CDCl₃) : *δ* (ppm) 1.48 - 1.57(m, 1H), 1.65 - 1.69 (m, 1H), 1.73 - 1.90 (m, 2H), 2.30 (q, 1H, *J* = 2.8 Hz), 2.85 - 3.02 (m, 3H), 3.09 - 3.19 (m, 1H), 3.49 (ddd, 1H, *J* = 14.4 Hz, 10.0 Hz et 2.8 Hz), 3.70 (dd, 1H, *J* = 14.4 Hz et 4.4 Hz), 4.63 - 4.71 (m , 1H), 7.45 (d, 1H, *J* = 3.2 Hz), 7.54 (d, 1H, *J* = 3.2 Hz), 7.84 (s, 1H), 8.17 (d, 1H, *J* = 8.2 Hz), 8.31 (d, 1H, *J* = 8.2 Hz), 8.88 (s, 1H); RMN ¹³C (100 MHz, CDCl₃) : *δ* (ppm) 20.0 (CH₂), 26.0 (CH₂), 28.1 (CH), 46.9 (CH₂), 47.2 (CH₂), 52.6 (CH₂), 58.3 (CH), 118.5 (CH), 119.0 (CH), 125.4 (CH), 127.3 (CH), 135.2 (CH), 135.7 (C_{q}), 136.6 (C_{q}), 136.7 (C_{q}), 141.6 (C_{q}), 145.0 (CH), 155.0 (C_{q}) ; HRMS (EI-MS) : calculée pour C₁₈H₁₉N₆O₂S m/z = 383.128471, trouvée m/z = 383.128544.

**(R)-3-[4-[3-(6-nitro-3-pyridyl)phényl]-1H-1,2,3-triazol-1-yl]quinuclidine (111).** Le produit est isolé sous la forme d'un solide blanc avec un rendement de 80% en suivant la procédure générale E2. R*_{f}* : 0,23 (CH₂Cl₂/MeOH/NH₄OH : 98/2/1) ; Mp : 180°C ; IR (ATR, Diamond) : *v* (cm⁻¹) : 793, 984, 1016, 1159, 1346, 1455, 1526, 2795, 2870, 2938 ; RMN ¹H (250 MHz, CDCl₃) : *δ* (ppm) 1.45 - 1.64(m, 1H), 1.67 - 1.94 (m, 3H), 2.33 (q, 1H, *J* = 2.8 Hz), 2.88 - 3.02 (m, 3H), 3.12 - 3.22 (m, 1H), 3.55 (ddd, 1H, *J* = 14.5 Hz, 10.0 Hz et 2.8 Hz), 3.76 (dd, 1H, *J* = 14.5 Hz et 4.4 Hz), 4.65 - 4.77 (m , 1H), 7.63 (d, 2H, *J* = 4.5 Hz), 7.95 (s, 2H), 8.22 - 8.40 (m, 3H), 8.92 (s, 1H) ; RMN ¹³C (100 MHz, CDCl₃) : *δ* (ppm) 20.0 (CH₂), 26.0 (CH₂), 28.2 (CH), 46.9 (CH₂), 47.2 (CH₂), 52.6 (CH₂), 58.6 (CH), 118.2 (CH), 119.4 (CH), 124.6 (CH), 126.7 (CH), 127.0 (CH), 130.0 (CH), 132.0 (C_{q}), 136.0 (C_{q}), 137.9 (CH), 142.1 (C_{q}), 146.5 (C_{q}), 147.2 (CH), 155.8 (C_{q}) ; HRMS (EI-MS) : calculée pour C₂₀H₂₁N₆O₂ m/z = 377.172050, trouvée m/z = 377.172021.

### Fluoration des alcools 63 et 85

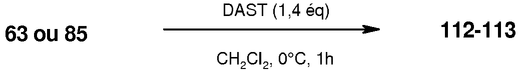

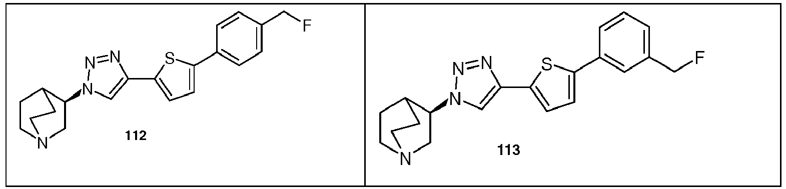

**Procédure générale H** : Le dérivé alcool **63** ou **85** (0,500 mmol) est solubilisé dans 10 mL de dichlorométhane puis à 0°C, du trifluorure de diethylaminosulfure (0,700 mmol) est ajouté goutte à goutte. La réaction est agitée à 0°C pendant une heure puis hydrolysée par l'ajout d'une solution saturée en NaHCO₃. La phase organique est séchée sur MgSO₄ anhydre, filtrée puis concentrée sous pression réduite. Le composé fluoré correspondant (**112** ou **113**) est purifié par chromatographie sur colonne de gel de silice en utilisant comme éluant un mélange CH₂Cl₂/MeOH/NH₄OH.

**(R)-3-[4-[5-[4-(fluorométhyl)phényl]-2-thiènyl]-1H-1,2,3-triazol-1-yl]quinuclidine (112).** Le produit est isolé sous la forme d'un solide blanc avec un rendement de 45% en suivant la procédure générale H. R_{f} : 0,25 (CH₂Cl₂/MeOH/NH₄OH : 98/2/1) ; Mp : 199°C ; IR (ATR, Diamond) : v (cm-1) : 962, 1042, 1060, 1219, 1376, 1414, 1454, 1502, 1601, 2163, 2321, 2937 ; RMN ¹H (400 MHz, CDCl₃) : δ (ppm) 1.45 - 1.54 (m, 1H), 1.66 - 1.70 (m, 1H), 1.77 - 1.90 (m, 2H), 2.30 (q, 1H, *J* = 3.2 Hz), 2.88 - 2.98 (m, 3H), 3.12 - 3.19 (m, 1H), 3.50 (ddd, 1H, *J* = 14.4 Hz, 9.6 Hz et 2.0 Hz), 3.71 (dd, 1H, *J* = 14.4 Hz et 3.6 Hz), 4.64 - 4.67 (m, 1H), 5.40 (d, 2H, *J* = 47.6 Hz), 7.31 (d, 1H, *J* = 3.6 Hz), 7.36 (d, 1H, *J* = 3.6 Hz), 7.40 (dd, 2H, *J* = 8.0 Hz et 1.2 Hz), 7.64 (d, 2H, *J* = 8.0 Hz), 7.75 (s, 1H); RMN ¹³C (100 MHz, CDCl₃) : δ (ppm) 20.0 (CH₂), 25.9 (CH₂), 28.1 (CH), 46.9 (CH₂), 47.2 (CH₂), 52.6 (CH₂), 58.5(CH), 84.2 (d, CH₂, *J* = 166 Hz), 118.4 (CH), 123.9 (CH), 124.9 (CH), 125.8 (2CH), 128.2 (d, 2CH, *J* = 6 Hz), 132.6 (C_{q}), 134.5 (d, C_{q}, *J* = 4 Hz), 135.4 (d, C_{q}, *J* = 17 Hz), 142.5 (C_{q}), 143.1 (C_{q}); HRMS (EI-MS) : calculée pour C₂₀H₂₂N₄FS m/z = 369.15437, trouvée m/z = 369.15433.

**(R)-3-[4-[5-[3-(fluoromethyl)phényl]-2-thièenyl]-1H-1,2,3-triazol-1-yl]quinuclidine (113).** Le produit est isolé sous la forme d'un solide blanc avec un rendement de 39% en suivant la procédure générale H. R_{f} : 0,25 (CH₂Cl₂/MeOH/NH₄OH : 98/2/1) ; Mp : 144°C ; IR (ATR, Diamond) : v (cm-1) : 792, 971, 1041, 1211, 1364, 1452, 1588, 1606, 2165, 2869, 2939 ; RMN ¹H (400 MHz, CDCl3) : δ (ppm) 1.46 - 1.54 (m, 1H), 1.65 - 1.80 (m, 3H), 2.27 - 2.31 (m, 1H), 2.88 - 2.98 (m, 3H), 3.11 - 3.18 (m, 1H), 3.50 (ddd, 1H, *J* = 14.4 Hz, 9.6 Hz et 2.0 Hz), 3.71 (dd, 1H, *J* = 14.4 Hz et 3.6 Hz), 4.62 - 4.66 (m, 1H), 5.43 (d, 2H, *J* = 47.6 Hz), 7.29 - 7.31 (m, 2H), 7.36 (d, 1H, *J* = 3.6 Hz), 7.42 (t, 1H, *J* = 7.6 Hz), 7.61 - 7.63 (m, 2H), 7.76 (s, 1H) ; RMN ¹³C (100 MHz, CDCl3) : δ (ppm) 20.0 (CH₂), 26.0 (CH₂), 28.1 (CH), 46.9 (CH₂), 47.2 (CH₂), 52.6 (CH₂), 58.5 (CH), 84.3 (d, CH₂, *J* = 166 Hz), 118.5 (CH), 123.9 (CH), 124.5 (d, CH, *J*= 6 Hz), 124.9 (CH), 125.9 (d, CH, *J* = 3 Hz), 126.4 (d, CH, *J* = 6 Hz), 129.2 (CH), 132.6 (C_{q}), 134.4 (C_{q}), 137.0 (d, C_{q}, *J* = 17 Hz), 142.5 (C_{q}), 143.1 (C_{q}) ; HRMS (EI-MS) : calculée pour C₂₀H₂₂N₄FS m/z = 369.154372, trouvée m/z = 369.154471.

### Réduction des aldéhydes 97 ou 101

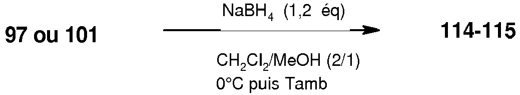

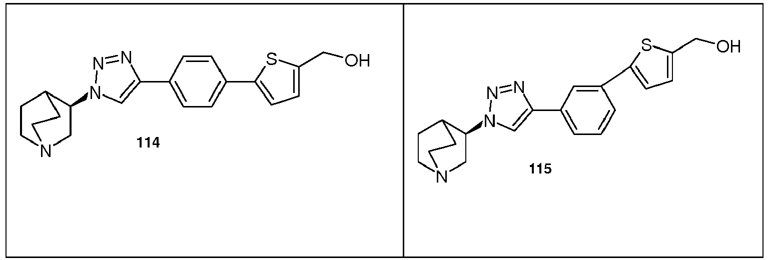

**Procédure générale I :** A une solution du dérivé aldéhyde (1,0 mmol) dans un mélange de 4 mL de dichlorométhane et 2 mL de méthanol est ajouté lentement du borohydrure de sodium (1,2 mmol) à 0°C. Le mélange réactionnel est ensuite agité à température ambiante pendant 15 minutes puis hydrolysée par l'ajout de 5 mL d'eau. 10 mL de dichlorométhane sont alors ajoutés au milieu et les deux phases sont séparées. La phase aqueuse est extraite par 2 fois 5 mL de dichlorométhane. Les phases organiques sont rassemblées, séchées sur MgSO₄ anhydre, filtrées et concentrées sous pression réduite. Le résidu ainsi obtenu est purifié par chromatographie sur colonne de gel de silice en utilisant comme éluant un mélange CH₂Cl₂/MeOH/NH₄OH.

**(R)-[5-[4-[1-[quinuclidin-3-yl]-1*H*-1,2,3-triazol-4-yl]phényl]-2-thiènyl]méthanol (114).** Le produit est isolé sous la forme d'un solide blanc avec un rendement de 85% en suivant la procédure générale I. R*_{f}*: 0,12 (CH₂Cl₂/MeOH/NH₄OH : 97/3/1) ; Mp : 234°C ; IR (ATR, Diamond) : *v*(cm⁻¹) : 791, 979, 1032, 1204, 1328, 1447, 1500, 1661, 2871, 2939, 3124 ; RMN ¹H (400 MHz, CDCl₃) : *δ*(ppm) 1.34 - 1.47 (m, 2H), 1.68 - 1.74 (m, 2H), 2.29 (q, 1H, *J* = 2.8 Hz), 2.71 - 2.78 (m, 3H), 2.93 - 3.02 (m, 1H), 3.38 (m, 2H), 4.62 (d, 2H, *J* = 5.2 Hz), 4.72 - 4.75 (m, 1H), 5.51 (t, 1H, *J* = 5.2 Hz), 6.95 (d, 1H, *J* = 3.6 Hz), 7.38 (d, 1H, *J* = 3.6 Hz), 7.69 (d, 2H, *J* = 8.4 Hz), 7.89 (d, 2H, *J* = 8.4 Hz), 8.75 (m, 1H) ; RMN ¹³C (100 MHz, CDCl₃) : *δ*(ppm) 20.0 (CH₂), 25.8 (CH₂), 28.1 (CH), 46.8 (CH₂), 47.0 (CH₂), 52.3 (CH₂), 57.9 (CH), 58.8 (CH₂), 121.3 (CH), 123.6 (CH), 125.7 (CH), 125.9 (2CH), 126.1 (2CH), 130.2 (C_{q}), 133.7 (C_{q}), 142.1 (C_{q}), 146.1 (C_{q}), 146.6 (C_{q}) ; HRMS (EI-MS) : calculée pour C₂₀H₂₃N₄SO m/z = 367.15871, trouvée m/z = 367.15900.

**(R)-[5-[3-[1-[quinuclidin-3-yl]-1*H*-1,2,3-triazol-4-yl]phényl]-2-thiènyl]méthanol (115).** Le produit est isolé sous la forme d'un solide blanc avec un rendement de 82% en suivant la procédure générale I. R*_{f}*: 0,16 (CH₂Cl₂/MeOH/NH₄OH : 97/3/1) ; Mp : 176°C ; IR (ATR, Diamond) : *v*(cm⁻¹) : 795, 981, 1029, 1206, 1373, 1416, 1450, 1608, 2877, 2942, 3070 ; RMN ¹H (400 MHz, CDCl₃) : *δ*(ppm) 1.32 - 1.51 (m, 2H), 1.65 - 1.82 (m, 2H), 2.29 (q, 1H, *J* = 2.8 Hz), 2.73 - 2.80 (m, 3H), 2.95 - 3.02 (m, 1H), 3.39 - 3.49 (m, 2H), 4.65 (s, 2H), 4.72 - 4.81 (m, 1H), 5.54 (bl, 1H), 6.98 (d, 1H, *J* = 3.6 Hz), 7.42 (d, 1H, *J* = 3.6 Hz), 7.47 (t, 1H, *J* = 7.8 Hz), 7.57 (d, 1H, *J* = 7.8 Hz), 7.80 (d, 1H, *J* = 7.8 Hz), 8.11 (s, 1H), 8.85 (s, 1H) ; RMN ¹³C (100 MHz, CDCl₃) : *δ*(ppm) 20.0 (CH₂), 25.7 (CH₂), 28.1 (CH), 46.8 (CH₂), 47.0 (CH₂), 52.3 (CH₂), 57.8 (CH), 58.8 (CH₂), 121.6 (CH), 122.0 (CH), 123.8 (CH), 124.4 (CH), 124.8 (CH), 125.6 (CH), 130.1 (CH), 132.0 (C_{q}), 135.0 (C_{q}), 142.1 (C_{q}), 146.1 (C_{q}), 146.8 (C_{q}) ; HRMS (EI-MS): calculée pour C₂₀H₂₃N₄SO m/z = 367.15871, trouvée m/z = 367.15892.

### Chloration des alcools 63 et 38

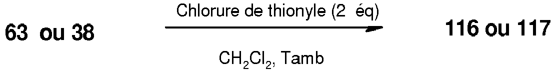

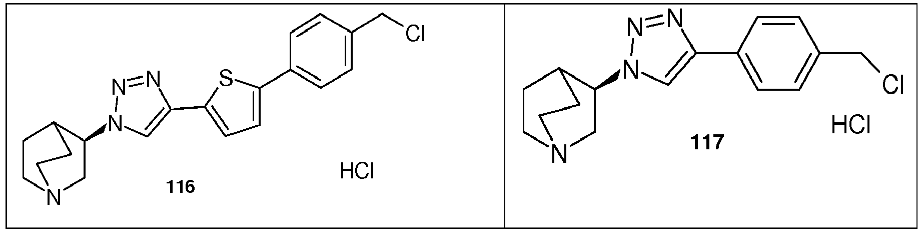

**Procédure générale J** : Le dérivé alcool **63** ou **38** (0,500 mmol) est solubilisé dans 10 mL de dichlorométhane puis à 0°C, du chlorure de thionyle (1,0 mmol) est ajouté goutte à goutte suivi d'une quantité catalytique de diméthylformamide. La réaction est ensuite agitée à température ambiante pendant une nuit. Après évaporation des solvants, le mélange réactionnel est lavé par l'éther diéthylique (5 x 10 mL). Le précipité ainsi formé est filtré puis séché sous vide.

**(R)-3-[4-[5-[4-(chlorométhyl)phenyl]-2-thiènyl]-1*H*-1,2,3-triazol-1-yl]quinuclidine, hydrochloride (116).** Le produit est isolé sous la forme d'un solide beige avec un rendement de 96% en suivant la procédure générale J. R*_{f}*: 0,3 (CH₂Cl₂/MeOH/NH₄OH : 98/2/1) ; Mp : 255°C ; IR (ATR, Diamond) : *v*(cm⁻¹) : 797, 973, 1042, 1229, 1319, 1455, 1600, 1694, 2880, 3392 ; RMN ¹H (400 MHz, , DMSO-*d₆*) : *δ*(ppm) 1.56 - 1.87 (m, 2H), 1.95 - 2.14 (m, 2H), 2.52 - 2.55 (m, 1H), 3.20 - 3.50 (m, 4H), 3.87 - 3.06 (m, 2H), 4.80 (s , 2H), 5.16 -5.32 (m, 1H), 7.42 - 7.60 (m, 4H), 7.67 - 7.78 (m, 2H), 8.87 (s, 1H), 11.20 (bl, 1H) ; RMN ¹³C (100 MHz, DMSO-*d₆*) : *δ*(ppm) 17.5 (CH₂), 21.6 (CH₂), 26.7 (CH), 45.6 (CH₂), 45.8 (CH₂), 46.3 (CH₂), 49.3 (CH₂), 55.1 (CH), 121.5 (CH), 125.3 (CH), 125.9 (2CH), 126.0 (CH), 130.2 (2CH), 132.8 (C_{q}), 133.8 (C_{q}), 137.5 (C_{q}), 142.2 (C_{q}), 142.3 (C_{q}) ; HRMS (EI-MS) : calculée pour C₂₀H₂₂N₄SCI m/z = 385.124822, trouvée m/z = 385.124944.

**(R)-3-[4-[4-(chlorométhyl)phényl]-1*H*-1,2,3-triazol-1-yl]quinuclidine, hydrocloride (117).** Le produit est isolé sous la forme d'un solide beige avec un rendement de 90% en suivant la procédure générale J. R*_{f}*: 0,3 (CH₂Cl₂/MeOH/NH₄OH : 98/2/1) ; Mp : 240°C ; IR (ATR, Diamond) : *v*(cm⁻¹) : 807, 973, 1040, 1210, 1319, 1455, 1613, 2560, 2956, 3398 ; RMN ¹H (400 MHz, DMSO-*d₆*) : *δ*(ppm) 1.56 - 1.88 (m, 2H), 1.99 - 2.11 (m, 2H), 2.54 - 2.55 (m, 1H), 3.28 - 3.46 (m, 4H), 3.87 - 3.05 (m, 2H), 4.81 (s , 2H), 5.19 - 5.30 (m, 1H), 7.54 (d, 2H, *J* = 8.2 Hz), 7.98 (d, 2H, *J* = 8.2 Hz), 8.94 (s, 1H), 11.24 (bl, 1H) ; RMN ¹³C (100 MHz, DMSO-*d₆*) : *δ*(ppm) 17.5 (CH₂), 21.6 (CH₂), 26.7 (CH), 45.6 (CH₂), 45.8 (CH₂), 46.4 (CH₂), 49.3 (CH₂), 54.9 (CH), 122.2 (CH), 125.8 (2CH), 129.9 (2CH), 130.9 (C_{q}), 137.8 (C_{q}), 146.6 (C_{q}) ; HRMS (EI-MS) : calculée pour C₁₆H₂₀N₄Cl m/z = 303.137101, trouvée m/z = 303.137321.

### Préparation du composé 79 de type quinuclidine (formule (II-2)

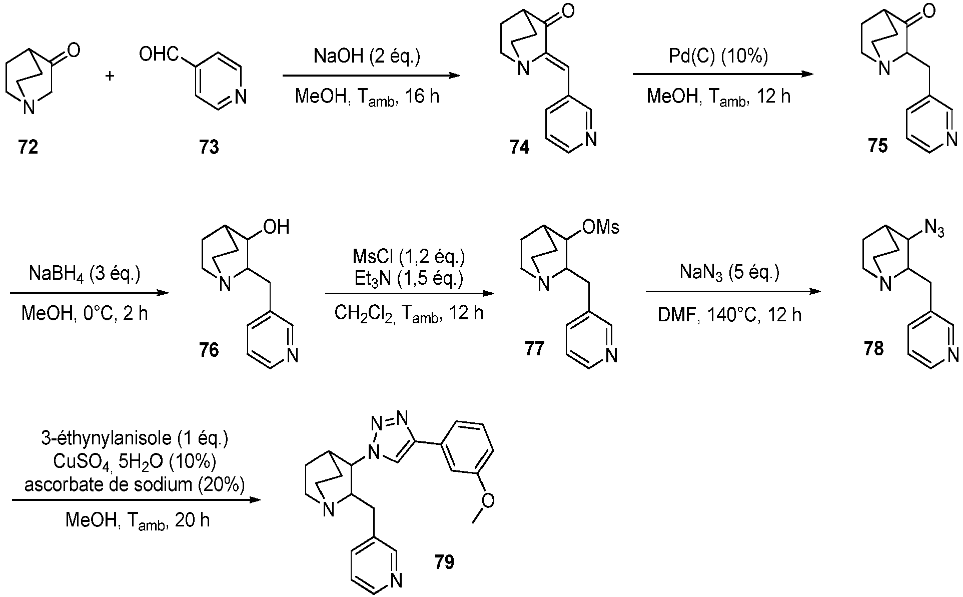

**2-(Pyridin-3-ylméthylène)quinuclidin-3-one (74).** A une solution de quinuclidone **72** (2,00 g, 12,4 mmol) dans 20 mL de méthanol est ajouté du NaOH (1,04 g, 26,0 mmol). Après 30 minutes d'agitation à température ambiante, la 3-pyridinecarboxaldehyde **73** (1,28 mL, 13,6 mmol) est ajoutée goutte à goutte puis la réaction est agitée pendant 16 heures. La réaction est hydrolysée par l'ajout d'un minimum d'eau jusqu'à dissolution totale des sels présents dans le milieu. Le milieu réactionnel est placé au réfrigérateur jusqu'à précipitation total puis la suspension est filtrée sous vide. La cétone **74** est isolée sous la forme d'un solide jaune avec un rendement de 79%. R*_{f}*: 0,72 (CH₂Cl₂/MeOH/NH₄OH : 90/10/0,1) ; Mp : 113°C ; IR (ATR, Diamond): *v*(cm⁻¹) : 972, 1031, 1095, 1168, 1186, 1218, 1244, 1332, 1409, 1451, 1627, 1704, 2869, 2937, 2957 ; RMN ¹H (250 MHz, CDCl₃) : *δ*(ppm) 1,99 (td, 4H, *J* = 7,9 Hz et 2,8 Hz), 2,60 (p, 1H, *J* = 3,0 Hz), 2,87 - 3,02 (m, 2H), 3,07 - 3,21 (m, 2H), 6,93 (s, 1H), 7,25 (dd, 1H, *J* = 8,0 Hz et 4,8 Hz), 8,45 (dt, 1H, *J* = 8,0 Hz et 1,8 Hz), 8,50 (dd, 1H, *J* = 4,8 Hz et 1,8Hz), 8,99 (d, 1H, *J* = 2,1 Hz) ; RMN ¹³C (100 MHz, CDCl₃) : *δ*(ppm) 25,8 (2CH₂), 40,2 (CH), 47,5 (2CH₂), 121,5 (CHₐᵣₒ), 123,4 (CHₐᵣₒ), 130,1 (C_{q}), 138,6 (CHₐᵣₒ), 146,6 (C_{q}), 150,0 (CHₐᵣₒ), 153,0 (CHₐᵣₒ), 205,7 (C=O) ; HRMS (EI-MS) : calculée pour C₁₃H₁₅N₂O m/z = 215,1184, trouvée m/z = 215,1192.

**2-(Pyridin-3-ylméthyl)quinuclidin-3-one (75).** Sous atmosphère d'hydrogène, la cétone **73** (500 mg, 2,33 mmol) dans 20 mL de méthanol est réduite par ajout d'une quantité catalytique de Pd/C 10% (124 mg, 0,117 mmol) et agitée à température ambiante pendant 12 heures. A la fin de la réaction, le milieu réactionnel est filtré sur célite puis le filtrat est évaporé sous pression réduite. Le produit est purifié par chromatographie sur colonne de gel de silice en utilisant comme éluant un mélange CH₂Cl₂/MeOH (9/1). Le composé **75** est isolé sous la forme d'un solide blanc avec un rendement de 63%. R*_{f}*: 0,23 (CH₂Cl₂/MeOH : 9/1) ; Mp : 95°C ; IR (ATR, Diamond) : *v*(cm⁻¹) : 984, 1028, 1053, 1071, 1096, 1185, 1328, 1422, 1459, 1477, 1576, 1715, 2869, 2962 ; RMN ¹H (250 MHz, CDCl₃) : *δ*(ppm) 1,93 - 2,07 (m, 4H), 2,47 (p, 1H, *J* = 3,0 Hz), 2,75 (dd, 1H, *J* = 14,9 Hz et 10,8 Hz), 2,82 - 2,93 (m, 2H), 3,03 - 3,24 (m, 3H), 3,31 (dd, 1H, *J* = 10,8 Hz et 4,0 Hz), 7,20 (dd, 1H, *J* = 7,8 Hz et 4,8 Hz), 7,59 (dt, 1H, *J* = 7,8 Hz et 1,7 Hz), 8,45 (d, 1H, *J* = 3,8 Hz), 8,51 (s, 1H) ; RMN ¹³C (100 MHz, CDCl₃) : *δ*(ppm) 25,1 (CH₂), 27,1 (CH₂), 30,8 (CH₂), 40,2 (CH), 41,2 (CH₂), 49,0 (CH₂), 71,0 (CH), 123,5 (CHₐᵣₒ), 134,7 (C_{q}), 136,4 (CHₐᵣₒ), 148,0 (CHₐᵣₒ), 150,4 (CHₐᵣₒ et C_{q}) ; HRMS (EI-MS) : calculée pour C₁₃H₁₇N₂O m/z = 217,1341, trouvée m/z = 217,1350.

**2-(Pyridin-3-ylméthyl)quinuclidin-3-ol (76).** A une solution de la cétone **75** (450 mg, 2,08 mmol) dans 40 mL de méthanol refroidie à 0°C à l'aide d'un bain de glace est ajouté par portion un excès de NaBH₄ (236 mg, 6,24 mmol). Après deux heures d'agitation, le méthanol est évaporé puis le résidu est repris dans le dichlorométhane. La phase organique est lavée avec de l'eau, séchée sur MgSO₄ anhydre, filtrée et évaporée sous pression réduite. L'alcool **76** est isolé sous la forme d'un solide blanc avec un rendement de 93% sous la forme de deux diastéréoisomères non séparables dans des proportions 1/1. R*_{f}*: 0,26 (CH₂Cl₂/MeOH/NH₄OH : 80/20/0,1) ; IR (ATR, Diamond) : *v*(cm⁻¹) : 981, 1025, 1042, 1066, 1093, 1130, 1168, 1193, 1311, 1344, 1422, 1454, 1480, 1575, 2364, 2871, 2940, 3143 ; RMN ¹H (250 MHz, CDCl₃) : *δ*(ppm) 1,26 - 1,40 (m, 2H), 1,42 - 1,56 (m, 2H), 1,58 - 1,72 (m, 2H), 1,77 - 2,01 (m, 4H), 2,58 - 2,92 (m, 10H), 2,95 - 3,25 (m, 6H), 3,47 - 3,53 (m, 1H), 3,83 - 3,91 (m, 1H), 7,14 - 7,23 (m, 2H), 7,57 - 7,65 (m, 2H), 8,30 - 8,55 (m, 4H) ; RMN ¹³C (100 MHz, CDCl₃) : *δ*(ppm) 18,7 (CH₂), 19,1 (CH₂), 24,7 (CH₂), 25,7 (CH₂), 29,5 (CH), 30,5 (CH), 31,0 (CH₂), 36,5 (CH₂), 41,0 (CH₂), 41,8 (CH₂), 48,9 (CH₂), 50,0 (CH₂), 62,2 (CH), 67,3 (CH), 68,4 (CH), 74,1 (CH), 123,5 (CHₐᵣₒ), 123,6 (CHₐᵣₒ), 135,2 (C_{q}), 136,5 (C_{q}), 137,0 (2CHₐᵣₒ), 147,2 (CHₐᵣₒ), 147,6 (CHₐᵣₒ), 150,3 (CHₐᵣₒ), 150,6 (CHₐᵣₒ) ; HRMS (EI-MS) : calculée pour C₁₃H₁₉N₂O m/z = 219,1497, trouvée m/z = 219,1491.

**2-(Pyridin-3-ylméthyl)quinuclidin-3-yl méthane sulfonate (77).** A une solution de l'alcool **76** (360 mg, 1,65 mmol) dans 15 mL de dichlorométhane sont ajoutés à température ambiante du chlorure de mésyle (150 µL, 1,98 mmol) et de la triéthylamine (350 µL, 2,48 mmol). Le milieu réactionnel est agité à température ambiante pendant 12 heures puis la réaction est hydrolysée par l'ajout d'une solution saturée en NaHCO₃. La phase organique est séparée, séchée sur MgSO₄ anhydre, filtrée et évaporée sous pression réduite. Le produit est purifié par chromatographie sur colonne de gel de silice en utilisant comme éluant un mélange CH₂Cl₂/MeOH/NH₄OH (90/10/0,1). Le composé mésylé **77** est isolé sous la forme d'un solide jaune avec un rendement de 70% sous la forme de deux diastéréoisomères non séparables dans des proportions 1/1. R*_{f}*: 0,35 (CH₂Cl₂/MeOH/NH₄OH : 90/10/0,1) ; IR (ATR, Diamond) : *v*(cm⁻¹) : 890, 937, 989, 1027, 1166, 1321, 1342, 1428, 1461, 1479, 2873, 2943 ; RMN ¹H (250 MHz, CDCl₃) : *δ*(ppm) 1,35 - 1,95 (m, 8H), 2,19 - 2,31 (m, 2H), 2,62 - 3,15 (m, 13H), 2,74 (s, 3H), 2,91 (s, 3H), 3,23 - 3,33 (m, 1H), 4,44 - 4,49 (m, 1H), 4,89 - 4,95 (m, 1H), 7,17 - 7,26 (m, 2H), 7,52 - 7,62 (m, 2H), 8,41 - 8,53 (m, 4H) ; RMN ¹³C (100 MHz, CDCl₃) : *δ*(ppm) 18,3 (CH₂), 19,2 (CH₂), 24,1 (CH₂), 25,1 (CH₂), 28,0 (CH), 28,5 (CH), 30,7 (CH₂), 35,7 (CH₂), 38,4 (CH₃), 38,5 (CH₃), 40,6 (CH₂), 41,3 (CH₂), 48,7 (CH₂), 49,8 (CH₂), 60,2 (CH), 64,3 (CH), 80,7 (CH), 84,1 (CH), 123,4 (CHₐᵣₒ), 123,6 (CHₐᵣₒ), 134,1 (C_{q}), 135,0 (C_{q}), 136,5 (CHₐᵣₒ), 136,8 (CHₐᵣₒ), 147,8 (CHₐᵣₒ), 148,1 (CHₐᵣₒ), 150,6 (CHₐᵣₒ), 150,7 (CHₐᵣₒ) ; HRMS (EI-MS) : calculée pour C₁₄H₂₁N₂O₃S m/z = 297,1273, trouvée m/z = 297,1281.

**3-(4-(3-Méthoxyphényl)-1H-1,2,3-triazol-1-yl)-2-(pyridin-3-ylméthyl)quinuclidine (79).** Le dérivé mésylé 77 (300 mg, 1,01 mmol) et l'azoture de sodium (330 mg, 5,05 mmol) sont chauffés à 140°C dans 10 mL de DMF pendant 12 heures. A l'issu de la réaction, le solvant est évaporé puis le résidu est repris dans du CH₂Cl₂. La phase organique est lavée deux fois à l'eau, séchée sur MgSO₄ anhydre et concentrée sous pression réduite. L'azoture **78** est purifié par chromatographie sur colonne de gel de silice en utilisant comme éluant un mélange CH₂Cl₂/MeOH/NH₄OH (90/10/0,1).
L'azoture **78** (110 mg, 0,452 mmol) est solubilisé dans 4 mL de méthanol puis sont ajoutés successivement du 3-ethynylanisole (60 µL, 0,452 mmol), du CuSO₄,5H₂O (11 mg, 0,045 mmol) et de l'ascorbate de sodium (18 mg, 0,090 mmol). Le milieu réactionnel est agité à température ambiante pendant 12 h. A l'issu du temps de réaction, le méthanol est évaporé sous pression réduite puis le résidu est chromatographié sur colonne de gel de silice en utilisant comme éluant un mélange CH₂Cl₂/MeOH/NH₄OH (90/10/0,1). Le produit **79** est isolé sous la forme d'une huile jaune avec un rendement de 48% sous la forme d'un seul diastéréoisomère. R*_{f}*: 0,24 (CH₂Cl₂/MeOH/NH₄OH : 90/10/0,1) ; IR (ATR, Diamond) : *v*(cm⁻¹) : 986, 1037, 1074, 1157, 1244, 1282, 1320, 1424, 1458, 1479, 1583, 1609, 2872, 2943 ; RMN ¹H (250 MHz, DMSO-*d₆*, 80°C) : *δ*(ppm) 1,36 - 1,51 (m, 1H), 1,62 - 1,96 (m, 3H), 2,21 (q, 1H, *J* = 2,8 Hz), 2,67 - 3,25 (m, 6H), 3,75 - 3,84 (m, 1H), 3,87 (s, 3H), 4,48 (d, 1H, *J* = 7,0 Hz), 6,89 - 7,01 (m, 1H), 7,22 (dd, 1H, *J* = 7,8 Hz et 4,8 Hz), 7,35 - 7,48 (m, 3H), 7,64 (dt, 1H, *J* = 7,8 Hz et 1,9 Hz), 8,34 (d, 1H, *J* = 3,4 Hz), 8,49 (s, 1H), 8,54 (s, 1H) ; RMN ¹³C (100 MHz, DMSO-*d₆*, 80°C) : *δ*(ppm) 19,1 (CH₂), 25,8 (CH₂), 29,2 (CH), 34,9 (CH₂), 40,0 (CH₂), 48,5 (CH₂), 54,8 (CH₃), 61,2 (CH), 63,7 (CH), 110,5 (CHₐᵣₒ), 113,1 (CHₐᵣₒ), 117,3 (CHₐᵣₒ), 120,4 (CHₐᵣₒ), 122,5 (CHₐᵣₒ), 129,4 (CHₐᵣₒ), 132,0 (C_{q}), 134,1 (C_{q}), 135,8 (CHₐᵣₒ), 145,6 (C_{q}), 146,7 (CHₐᵣₒ), 149,7 (CHₐᵣₒ), 159,4 (C_{q}) ; HRMS (EI-MS) : calculée pour C₂₂H₂₅N₅O m/z = 375,2059, trouvée m/z = 376,21334.

### ACTIVITE BIOCHIMIQUE DES COMPOSÉS DE L'INVENTION

### Détermination de l'affinité des composés de l'invention pour les récepteurs nicotiniques Ra7

Les composés de la présente invention sont testés pour leur affinité avec les Rα7 en compétition avec un ligand de référence, le [¹²⁵I]α-bungarotoxin, sur préparation membranaire cérébrale de rat, selon le protocole décrit par Davies et al. (1999) légèrement modifié.

### Préparation membranaire

Des rats mâles de souche Wistar (Centre d'élevage R. Janvier, Saint Berthevin) de 250g sont utilisés selon les règles d'expérimentation animale en vigueur. Après décapitation (2 animaux/expérience), les cerveaux sont rapidement prélevés, recueillis sur glace, et le cortex frontal est disséqué et pesé. Le tissu est broyé (Ultraturrax T25 Janke & Kunkel, 9500 tr/min.) dans 2 ml de tampon froid (tampon HEPES 15 mM contenant 120 mM NaCl, 5.4 mM KCl, 0.8 mM MgCl₂ et 1.8 mM CaCl₂, pH7.4) puis centrifugé (J2-21M/E, Beckman) à 45 000 x g à 4°C pendant 10 min. Le surnageant est éliminé, le culot est repris dans 2 ml du même tampon puis remis en suspension.

Le dosage des protéines est réalisé selon la méthode de Bradford (1976) sur le culot (Spectronic 20® Genesys™). Il est ensuite dilué dans le tampon de façon à avoir 0.25 g/ml de protéines.

### Etude de binding

Le ligand de référence utilisé est le [¹²⁵I]α-bungarotoxin (Perkin Elmer, activité spécifique 81.4TBq/mmol). Les tubes sont préparés en double.

Dans chaque tube (BD Vacutainer, AES Chemunex) contenant 0.4 ml de tampon Tris-HCl 50 mM contenant 120 mM NaCl, 5 mM KCl, 1 mM MgCl2, 2.5 mM CaCl2, pH 7.4, sont ajoutés 0.2 ml de la suspension protéique, 0.2 ml d'une solution de [¹²⁵I]α-bungarotoxin (soit une concentration de 2 nM) diluée dans le tampon Tris, et 0.2 ml d'une solution contenant le composé à tester à des concentrations variant entre 10⁻⁶ et 10⁻¹⁰ M. La fixation non-spécifique est déterminée en présence de 10⁻⁶ M de α-bungarotoxin (Tocris). Les tubes sont incubés dans une étuve à 22°C pendant 3 h.

Le contenu des tubes est ensuite dilué dans 3 ml de tampon Tris à 4°C additionné de 0.1% de BSA, filtré (Rampe de filtration Hoefer FH225V, Fisher Scientific) sur des filtres GF/C (Whatman), pré-trempés dans du tampon Tris froid additionné de polyéthylèneimine à 0.05%, puis rincés avec 2 fois 3 ml de tampon froid.

La radioactivité résiduelle des filtres est comptée (Cobra 5020, Beckman), l'IC₅₀ est déterminée graphiquement et le Ki calculé (Ki=IC₅₀/(1+[L*]/Kd) (Cheng et Prussof, 1973).

| Composé n° | Structure | Masse Moléculaire Formule brute Solubilité | Activité sur Rα7 |
|---|---|---|---|
| 16 | 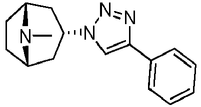 | M = 268,36 g/mol C₁₆H₂₀N₄ CHCl₃ - DMSO | 168±68 nM |
| 17 | 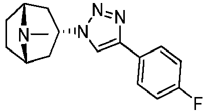 | M = 286,35 g/mol C₁₆H₁₉FN₄ CHCl₃ - DMSO | 210±31 nM |
| 19 | 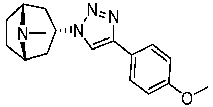 | M = 298,38 g/mol C₁₇H₂₂N₄O CHCl3 - DMSO | 283±76 nM |
| 22 | 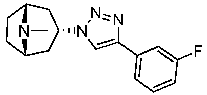 | M = 286,35 g/mol C₁₆H₁₉FN₄ CHCl₃ - DMSO | 16±9 nM |
| 24 | 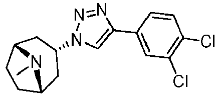 | M = 337,25 g/mol C₁₆H₁₈Cl₂N₄ CHCl₃ - DMSO | 110±36 nM |
| 25 | 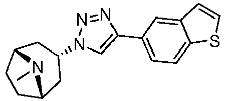 | M = 324,44 g/mol C₁₈H₂₀N₄S CHCl₃ - DMSO | 14±4 nM |
| 26 | 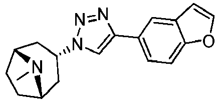 | M = 308,38 g/mol C₁₈H₂₀N₄O CHCl₃ - DMSO | 13±5 nM |
| 27 | 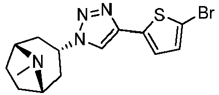 | M = 353,28 g/mol C₁₄H₁₇BrN₄S CHCl₃ - DMSO | 275±43 nM |
| 37 | 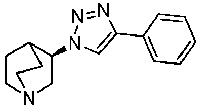 | M = 254,33 g/mol C₁₅H₁₈N₄ CHCl₃ - DMSO | 8±4 nM |
| 42 | 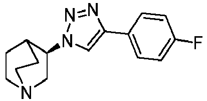 | M = 272,32 g/mol C₁₅H₁₇FN₄ DMSO | 15±4 nM |
| 46 | 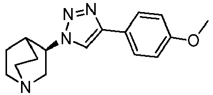 | M = 284,36 g/mol C₁₆H₂₀N₄O DMSO | 11±1 nM |
| 39 | 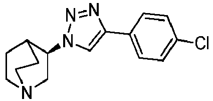 | M = 288,78 g/mol C₁₅H₁₇ClN₄ CHCl₃ - DMSO | 8±3 nM |
| 49 | 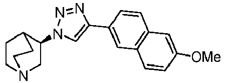 | M = 334,41 g/mol C₂₀H₂₂N₄O CHCl₃ - DMSO | 196±21 nM |
| 41 | 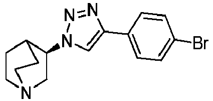 | M = 333,23 g/mol C₁₅H₁₇BrN₄ CHCl₃ - DMSO | 6±3 nM |
| 38 | 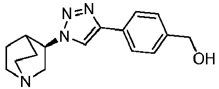 | M = 284,36 g/mol C₁₆H₂₀N₄O DMSO | 10±3 nM |
| 43 | 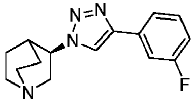 | M = 272,32 g/mol C₁₅H₁₇FN₄ CHCl₃ - DMSO | 10±1 nM |
| 50 | 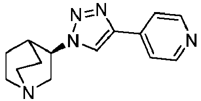 | M = 255,32 g/mol C₁₅H₁₇N₅ CHCl₃ - DMSO | 141±41 nM |
| 58 | 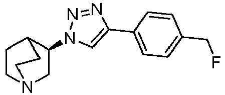 | M = 286,35 g/mol C₁₆H₁₉FN₅ CHCl₃ - DMSO | 21±7 nM |
| 40 | 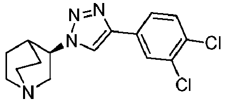 | M = 323,22 g/mol C₁₅H₁₆Cl₂N₄ CHCl₃ - DMSO | 19±6 nM |
| 55 | 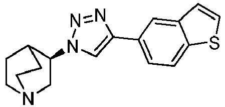 | M = 310,42 g/mol C₁₇H₁₈N₄S CHCl₃ - DMSO | 19±1 nM |
| 56 | 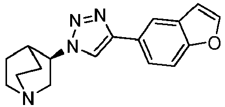 | M = 294,35 g/mol C₁₇H₁₈N₄O CHCl₃ - DMSO | 3±1 nM |
| 54 | 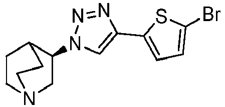 | M = 339,25 g/mol C₁₃H₁₅BrN₄S CHCl₃ - DMSO | 13±4 nM |
| 61 | 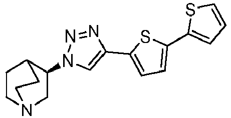 | M = 342,48 g/mol C₁₇H₁₈N₄S₂ CHCl₃ - DMSO | 20±2 nM |
| 62 | 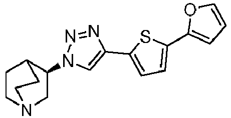 | M = 326,42 g/mol C₁₇H₁₈N₄OS CHCl₃ - DMSO | 10±1 nM |
| 44 | 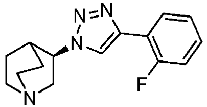 | M = 272,32 g/mol C₁₅H₁₇FN₄ CHCl₃ - DMSO | 13±3 nM |
| 48 | 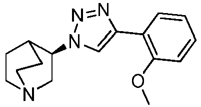 | M = 284,36 g/mol C₁₆H₂₀N₄O CDCl₃ - DMSO | 112±26 nM |
| 47 | 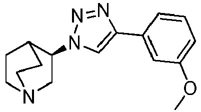 | M = 284,36 g/mol C₁₆H₂₀N₄O CDCl₃ - DMSO | 2,3±1 nM |
| 51 | 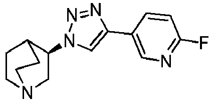 | M = 273,31 g/mol C₁₄H₁₆FN₅ CDCl₃ - DMSO | 469±6 nM |
| 52 | 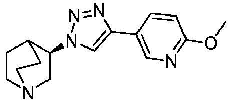 | M = 285,34 g/mol C₁₅H₁₉N₅O CDCl₃ - DMSO | 105±29 nM |
| 57 | 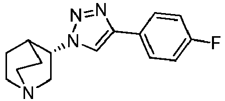 | M = 272,32 g/mol C₁₅H₁₇FN₄ CDCl₃ - DMSO | 114±38 nM |
| 45 | 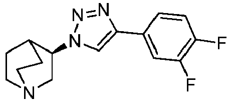 | M = 290,31 g/mol C₁₅H₁₆F₂N₄ CDCl₃ - DMSO | 10±4 nM |
| 53 | 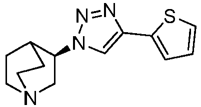 | M = 260,36 g/mol C₁₃H₁₆N₄S CDCl₃ - DMSO | 9,5±3 nM |
| 60 | 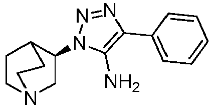 | M = 269,34 g/mol C₁₅H₁₉N₅ CDCl₃ - DMSO | 11±4 nM |
| 63 | 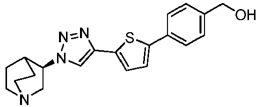 | M = 366,48 g/mol C₂₀H₂₂N₄OS CDCl₃ - DMSO | 0,6±0,2 nM |
| 79 | 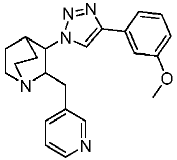 | M = 375,47 g/mol C₂₂H₂₅N₅O CHCl₃ - DMSO | ND |
| 83 | 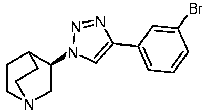 | M = 333,23 g/mol C15H17N4Br CH₂Cl₂/MeOH-DMSO | 11+/-2nM |
| 84 | 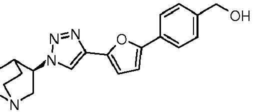 | M = 350,42 g/mol C₂₀H₂₂N₄O₂ DMSO | 6+/-3nM |
| 85 | 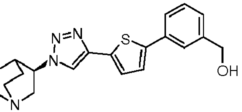 | M = 366,49 g/mol C₂₀H₂₂N₄OS DMSO | 0.7+/-0,3nM |
| 86 | 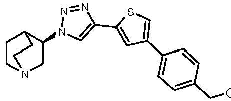 )H | M = 366,49 g/mol C₂₀H₂₂N₄OS DMSO | 1,5+/-0,6nM |
| 87 | 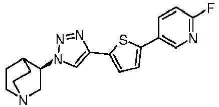 | M = 355,44 g/mol C₁₈H₁₈FN₅S CH₂Cl₂-CHCl₃ - DMSO | 13+/-2nM |
| 88 | 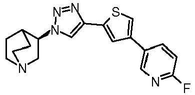 | M = 355,54 g/mol C18H18N5FS CH₂Cl₂/MeOH-DMSO | 31+/-4nM |
| 89 | 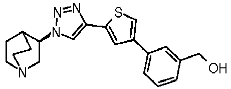 | M = 366,49 g/mol C₂₀H₂₂N₄OS CH₂Cl₂/MeOH-DMSO | 13+/-3nM |
| 90 | 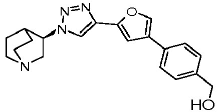 | M = 350,42 g/mol C20H22N4O2 CH₂Cl₂-CHCl₃ - DMSO | 9+/-2nM |
| 91 | 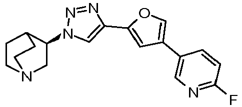 | M = 339,38 g/mol C18H18N5OF CH2Cl2-CHCl3 - DMSO | 117+/-26nM |
| 92 | 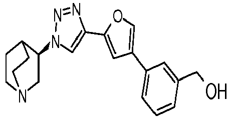 | M = 350,42 g/mol C20H22N4O2 CH2Cl2-CHCl3 - DMSO | 115+/-5nM |
| 93 | 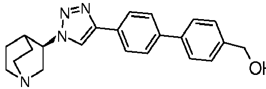 | M = 360,46 g/mol C22H24N4O CH₂Cl₂/MeOH-DMSO | 137 nM (n =2) |
| 95 | 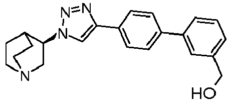 | M = 360,46 g/mol C22H24N4O CH₂Cl₂/MeOH-DMSO | 100+/-21 nM |
| 96 | 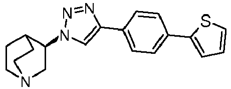 | M = 336,46 g/mol C19H20N4S CH₂Cl₂/MeOH-DMSO | 135 nM (n = 2) |
| 97 | 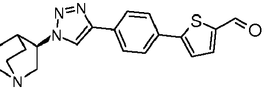 | M = 364,47 g/mol C20H20N4OS CH₂Cl₂/MeOH-DMSO | 90-120 nM |
| 98 | 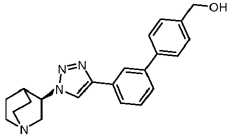 | M = 360,46 g/mol C22H24N4O CH₂Cl₂/MeOH-DMSO | 105 nM (n = 2) |
| 99 | 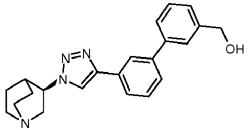 | M = 360,46 g/mol C22H24N4O CH₂Cl₂/MeOH-DMSO | 1,4+/-0,6nM |
| 100 | 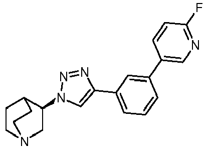 | M = 349,41 g/mol C20H20N5F CH2Cl2-CHCl3 - DMSO | 16+/-4 nM |
| 101 | 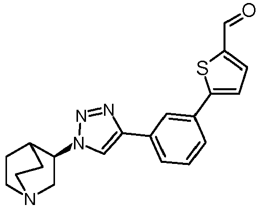 | M = 364,47 g/mol C20H20N4OS CH₂Cl₂/MeOH-DMSO | 7+/-2nM |
| 102 | 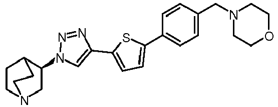 | M = 435 g/mol C24H29N5OS CH₂Cl₂/MeOH-DMSO | 0,9+/-0,2nM |
| 103 | 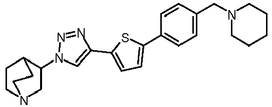 | M = 433 g/mol C25H31N5S CH₂Cl₂/MeOH-DMSO | 0,3+/-0,1nM |
| 104 | 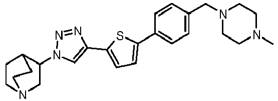 | M = 448 g/mol C25H32N6S CH₂Cl₂/MeOH-DMSO | 0,3+/-0,1nM |
| 105 | 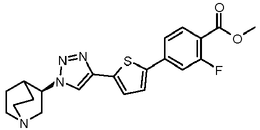 | M = 412 g/mol C21H21FN4O2S CH₂Cl₂/MeOH-DMSO | 85 nM (n = 2) |
| 112 | 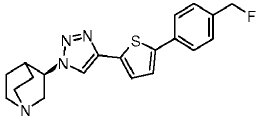 | M = 368,48 g/mol C₂₀H₂₁FN₄S CH₂Cl₂-CHCl₃ - DMSO | 5+/-2nM |
| 114 | 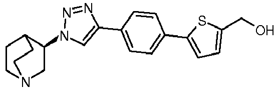 | M = 366,49 g/mol C₂₀H₂₂N₄OS CH₂Cl₂/MeOH-DMSO | 160 nM (n=2) |
| 115 | 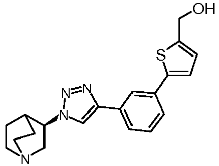 | M = 366,49 g/mol C₂₀H₂₂N₄OS CH₂Cl₂/MeOH-DMSO | 0,5+/-0,2nM |

### Détermination de la spécificité des composés de l'invention pour les récepteurs nicotiniques Ra7

Les composés fluorés présentant une bonne affinité pour les Rα7 (Ki vis-à-vis de [¹²⁵I]α-bungarotoxin ≤ 20 nM) sont évalués *in vitro* pour leur affinité vis-à-vis des récepteurs nicotiniques α4β2 (traceur de référence [³H]cytisine, Pabreza et al. 1991), muscariniques (traceur de référence [³H]QNB, Richards 1990) et sérotoninergiques 5-HT3 (traceur de référence [³H]BRL-43694, Hope et al. 1996).

### Références

Bradford et al. (1976) Anal Biochem 72: 248-254.
Cheng et al. (1973) Biochem Pharmacol 22: 3099-3108.
Davies et al. (1999) Neuropharmacology 38: 679-690.
Hope et al. (1996) BrJ Pharmacol 118: 1237-1245.
Pabreza et al. (1991) Mol Pharmacol 39: 9-12.
Richards et al. (1990) Br J Pharmacol 99: 753-761.

## Revendications

1. Composé de formule générale (II) suivante : 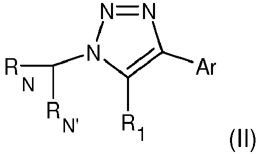 dans laquelle :
- le groupe Ar est choisi parmi les groupes phényle, pyridyle, thiophényle, furanyle, benzothiophényle, benzofuranyle et naphtalènyle, éventuellement substitués par un ou plusieurs groupes choisis parmi :
- les atomes d'halogène,
- le groupe -OH,
- les groupes alkyles et alcoxyles linéaires ou ramifiés comprenant de 1 à 10 atomes de carbone, éventuellement substitués, et
- les groupes aryles comprenant de 6 à 30 atomes de carbone et les groupes hétéroaryles comprenant de 1 à 30 atomes de carbone, éventuellement substitués,
- les groupes R_{N} et R_{N'} forment ensemble, avec l'atome de carbone auxquels ils sont liés, un groupe azacycloalcane choisi parmi les groupes tropane, quinuclidine et octahydroquinolizine de formules suivantes : 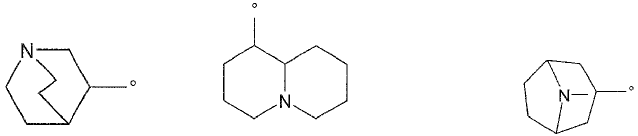 ledit groupe azacycloalcane étant éventuellement substitué par un ou plusieurs groupes choisis parmi :
- les atomes d'halogène,
- le groupe -OH, et
- les groupes alkyles et alcoxyles linéaires ou ramifiés comprenant de 1 à 10 atomes de carbone, éventuellement substitués,
- R₁ est un groupe choisi parmi : H ou NH₂ ;
ainsi que ses sels pharmaceutiquement acceptables, ses hydrates ou ses structures cristallines polymorphiques, ses racémates, diastéréoisomères ou énantiomères, les radicaux "alkyle", "aryle", et "hétéroaryle" éventuellement substitués susmentionnés pouvant être substitués par un ou plusieurs substituants choisis dans le groupe constitué de : CHO, amino, hydroxy, halogène, halogénoalkyle, carboxyle, alkyle, (hétéro)aryle, cycloalkyle, hétérocycloalkyle, alkaryle, alkoxy, alkylthio, alkylcarbonyle, aminocarbonyle, alkylcarboxyle, alkylamino, aryloxy, arylalkoxy, cyano, alkylsulfonyle, nitro et carboxyalkyle.

2. Composé selon la revendication 1, de formule générale (II-1) suivante : 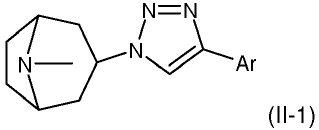 dans laquelle Ar est tel que défini dans la revendication 1.

3. Composé selon la revendication 1, de formule générale (II-2) suivante : 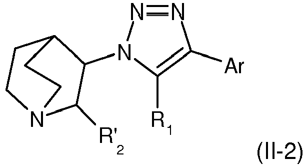 dans laquelle :
- le groupe R'₂ représente un groupe alkyle linéaire ou ramifié comprenant de 1 à 10 atomes de carbone, éventuellement substitué par un groupe aryle comprenant de 6 à 30 atomes de carbone ou par un groupe hétéroaryle comprenant de 1 à 30 atomes de carbone, et
- les groupes R₁ et Ar sont tels que définis dans la revendication 1.

4. Composé selon la revendication 1, de formule générale (II-3) suivante : 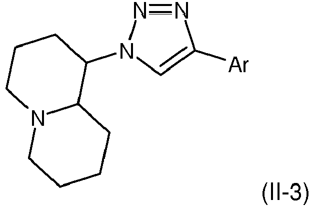 dans laquelle Ar est tel que défini dans la revendication 1.

5. Composé selon l'une quelconque des revendications 1 à 4, comportant au moins un isotope radioactif choisi parmi D, ¹⁸F, ¹¹C ou ¹²³I.

6. Composé selon l'une quelconque des revendications 1 à 5, pour son utilisation comme médicament.

7. Composé selon l'une quelconque des revendications 1 à 5, pour son utilisation dans le traitement ou la prévention de maladies du système nerveux central.

8. Composé selon la revendication 5, pour son utilisation comme marqueur radiopharmaceutique.

9. Composé selon la revendication 5, pour son utilisation comme marqueur radiopharmaceutique dans le diagnostic précoce et le suivi de maladies du système nerveux central.

## Patentansprüche

1. Verbindung der folgenden allgemeinen Formel (II): 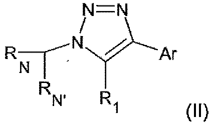 worin:
- die Gruppe Ar ausgewählt ist aus den Gruppen Phenyl, Pyridyl, Thiophenyl, Furanyl, Benzothiophenyl, Benzofuranyl und Naphthalenyl, gegebenenfalls substituiert mit einer oder mehreren Gruppen ausgewählt aus:
- Halogenatomen,
- der Gruppe -OH,
- linearen oder verzweigten Alkyl- und Alkoxylgruppen mit 1 bis 10 Kohlenstoffatomen, die gegebenenfalls substituiert sind, und
- Arylgruppen mit 6 bis 30 Kohlenstoffatomen und Heteroarylgruppen mit 1 bis 30 Kohlenstoffatomen, die gegebenenfalls substituiert sind,
- die Gruppen R_{N} und R_{N'} mit dem Kohlenstoffatom, an das sie gebunden sind, eine Azacycloalkangruppe zusammen bilden, die ausgewählt ist aus den Tropan-, Chinuclidin- und Octahydrochinolizingruppen der folgenden Formel: 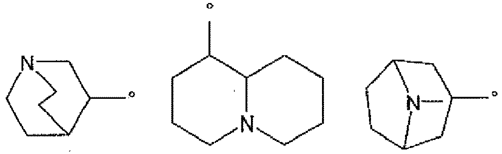 wobei die Azacycloalkangruppe gegebenenfalls substituiert mit einer oder mehreren Gruppen ist, die ausgewählt sind aus:
- Halogenatomen,
- der Gruppe -OH, und
- linearen oder verzweigten Alkyl- und Alkoxylgruppen mit 1 bis 10 Kohlenstoffatomen, die gegebenenfalls substituiert sind,
- R₁ eine Gruppe ist ausgewählt aus: H oder NH₂;
sowie ihre pharmazeutisch akzeptablen Salze, ihre Hydrate oder ihre kristallinen polymorphen Strukturen, ihre Racemate, Diastereoisomeren oder Enantiomeren, wobei die obengenannten, gegebenenfalls substituierten "Alkyl-''', "Aryl-" und "Heteroaryl"-Reste substituiert mit einem oder mehreren Substituenten sein können, die ausgewählt aus der Gruppe bestehend aus: CHO, Amino, Hydroxy, Halogen, Halogenoalkyl, Carboxyl, Alkyl, (Hetero)Aryl, Cycloalkyl, Heterocycloalkyl, Alkaryl, Alkoxy, Alkylthio, Alkylcarbonyl, Aminocarbonyl, Alkylcarboxyl, Alkylamino, Aryloxy, Arylalkoxy, Cyano, Alkylsulfonyl, Nitro und Carboxyalkyl.

2. Verbindung nach Anspruch 1, der folgenden allgemeinen Formel (II-1): 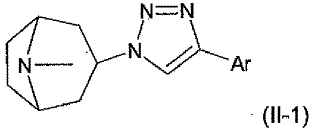 worin Ar wie in Anspruch 1 definiert ist.

3. Verbindung nach Anspruch 1, der folgenden allgemeinen Formel (II-2): 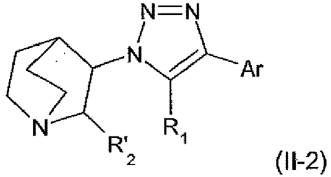 worin:
- die Gruppe R'₂ eine lineare oder verzweigte Alkylgruppe mit 1 bis 10 Kohlenstoffatomen darstellt, die gegebenenfalls mit einer Arylgruppe mit 6 bis 30 Kohlenstoffatomen oder mit einer Heteroarylgruppe mit 1 bis 30 Kohlenstoffatomen substituiert ist, und
- die Gruppen R₁ und Ar wie in Anspruch 1 definiert sind.

4. Verbindung nach Anspruch 1, der folgenden allgemeinen Formel (II-3): 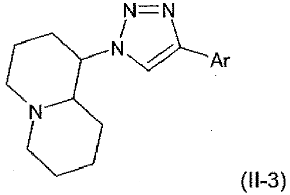 worin Ar wie in Anspruch 1 definiert ist.

5. Verbindung nach einem der Ansprüche 1 bis 4, aufweisend mindestens ein radioaktives Isotop ausgewählt aus D, ¹⁸F, ¹¹C oder ¹²³I.

6. Verbindung nach einem der Ansprüche 1 bis 5 zur Verwendung als Medikament.

7. Verbindung nach einem der Ansprüche 1 bis 5 zur Verwendung zur Behandlung oder Vorbeugung von Erkrankungen des zentralen Nervensystems.

8. Verbindung nach Anspruch 5 zur Verwendung als radiopharmazeutischer Marker.

9. Verbindung nach Anspruch 5 zur Verwendung als radiopharmazeutischer Marker bei der frühen Diagnose und Überwachung von Erkrankungen des zentralen Nervensystems.

## Claims

1. A compound, having the general formula (II) as follows: 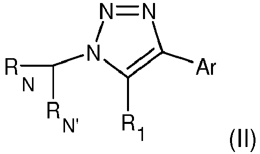 wherein:
- the Ar group is selected from among the phenyl, pyridyl, thiophenyl, furanyl, benzothiophenyl, benzofuranyl and naphthalenyl groups, possibly substituted by one or more substituents selected from among :
- the halogen atoms,
- the -OH group,
- the linear or branched alkyl and alkoxy groups comprising from 1 to 10 carbon atoms, possibly substituted, and
- the aryl groups comprising from 6 to 30 carbon atoms and the heteroaryl groups comprising from 1 to 30 carbon atoms, possibly substituted ;
- the R_{N} and R_{N'} groups, together with the carbon atoms to which they are bound, form a group selected from among the tropane, quinuclidine and octahydro-quinolizine groups having the following formulae: 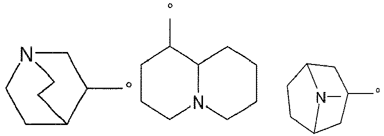 said azacycloalkane group being possibly substituted by one or more groups selected from among:
- the halogen atoms,
- the -OH group, and
- the linear or branched alkyl and alkoxy groups comprising from 1 to 10 carbon atoms, possibly substituted,
- R₁ is a group selected from: H or NH₂;
as well as the pharmaceutically acceptable salts thereof, the hydrates or polymorphic crystalline structures, racemates, diastereoisomers or enantiomers thereof,
wherein the possibly substituted "alkyl", "aryl" and "heteroaryl" radicals as mentioned above may be substituted by one or more substituents chosen from the group consisting of: CHO, amino, hydroxy, halogen, haloalkyl, carboxyl, alkyl, (hetero)aryl, cycloalkyl, heterocycloalkyl, alkaryl, alkoxy, alkylthio, alkylcarbonyl, aminocarbonyl, alkylcarboxyl, alkylamino, aryloxy, arylalkoxy, cyano, alkylsulfonyl, nitro, and carboxyalkyl.

2. A compound according to claim 1, having the general formula (II-1) as follows: 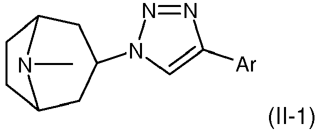 wherein Ar is as defined in claim 1.

3. A compound according to claim 1, having the general formula (II-2) as follows: 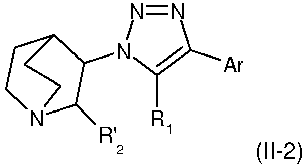 wherein
- the R_{'2} group represents a linear or branched alkyl group comprising from 1 to 10 carbon atoms, possibly substituted by an aryl group comprising from 6 to 30 carbon atoms or by a heteroaryl group comprising from 1 to 30 carbon atoms, and
- the R₁ and Ar groups are as defined in claim 1.

4. A compound according to claim 1, having the general formula (II-3) as follows: 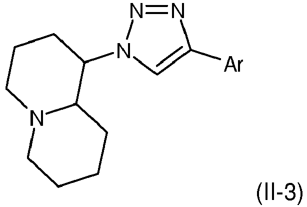 wherein Ar is as defined in claim 1.

5. A compound according to any one of claims 1 to 4, comprising at least one radioactive isotope selected from among D, ¹⁸F, ¹¹C or ¹²³I.

6. A compound according to any one of claims 1 to 5, for use as a medicament.

7. A compound according to any one of claims 1 to 5, for its use for the treatment or prevention of diseases of the central nervous system.

8. A compound according to claim 5, for use as a radiopharmaceutical marker.

9. A compound according to claim 5, for use as a radiopharmaceutical marker in the early diagnosis and monitoring of diseases of the central nervous system.
